(19) Europäisches Patentamt
European Patent Office
Office européen des brevets

(11) EP 4 653 426 A1

(12) # EUROPEAN PATENT APPLICATION
published in accordance with Art. 153(4) EPC

(43) Date of publication:
**26.11.2025 Bulletin 2025/48**

(21) Application number: **24744386.4**

(22) Date of filing: **19.01.2024**

(51) International Patent Classification (IPC):
***C07D 311/22*** $^{(2006.01)}$ ***C07D 311/04*** $^{(2006.01)}$
***C07D 405/14*** $^{(2006.01)}$ ***C07D 209/02*** $^{(2006.01)}$
***A61K 31/403*** $^{(2006.01)}$ ***A61K 31/352*** $^{(2006.01)}$
***A61P 35/00*** $^{(2006.01)}$

(52) Cooperative Patent Classification (CPC):
**A61K 31/352; A61K 31/403; A61P 35/00; C07D 209/02; C07D 311/04; C07D 311/22; C07D 405/14**

(86) International application number:
**PCT/CN2024/073173**

(87) International publication number:
**WO 2024/153216 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.01.2023 CN 202310095725**
**12.01.2024 CN 202410052474**

(71) Applicant: **Beijing Tide Pharmaceutical Co., Ltd.**
**Beijing 100176 (CN)**

(72) Inventors:
- **LI, Wenming**
  **Beijing 100176 (CN)**
- **LI, Xiaobo**
  **Beijing 100176 (CN)**
- **LU, Peng**
  **Beijing 100176 (CN)**
- **LIU, Junrong**
  **Beijing 100176 (CN)**
- **WANG, Ning**
  **Beijing 100176 (CN)**
- **KANG, Jialong**
  **Beijing 100176 (CN)**
- **HU, Huijuan**
  **Beijing 100176 (CN)**
- **ZHOU, Rong**
  **Beijing 100176 (CN)**
- **YU, Xiaolei**
  **Beijing 100176 (CN)**
- **PENG, Lijiao**
  **Beijing 100176 (CN)**
- **ZHOU, Liying**
  **Beijing 100176 (CN)**
- **GAN, Leling**
  **Beijing 100176 (CN)**
- **ZHANG, Yu**
  **Beijing 100176 (CN)**

(74) Representative: **Novitas Patent AB**
**P.O. Box 55557**
**102 04 Stockholm (SE)**

(54) **PROTEOLYSIS TARGETING CHIMERA COMPOUND**

(57) The present invention provides a targeted degradation chimera compound targeting protein tyrosine phosphatase, in particular a compound represented by formula (I), or a pharmaceutically acceptable salt, an isotope variant, a tautomer, a stereoisomer, a prodrug, a polymorph, a hydrate or a solvate thereof. The present invention also provides a preparation method for the compound, a pharmaceutical composition comprising the compound, and an effect of the compound in prevention and treatment of related diseases, such as hematological tumor or solid tumor diseases.

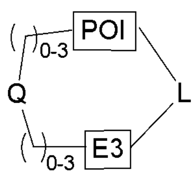

(I)

EP 4 653 426 A1

## Description

The present disclosure claims the priority of Chinese application 202310095725.2 filed on January 20, 2023, and Chinese application 202410052474.4 filed on January 12, 2024, which are incorporated herein by reference in their entirety.

## TECHNICAL FIELD

The present disclosure relates to a chimeric compound for targeted protein degradation, in particular to a compound represented by formula (I), or a pharmaceutically acceptable salt, an isotope variant, a tautomer, a stereoisomer, a prodrug, a polymorph, a hydrate or a solvate thereof.

## BACKGROUND

The protein tyrosine phosphatase (PTP) superfamily contains about 112 proteins, whose active regions are highly conserved, and their active site sequences are (H/V)C(X)5R(S/T). According to the differences in structure and function, the PTP superfamily can be divided into four subfamilies, which include classical tyrosine-specific phosphatases (classical pTyr specific PTPs), dual-specificity phosphatases (DSPs), Cdc25 phosphatases, and low molecular weight PTPS (LMW-PTPs). The tyrosine-specific PTPs can be further divided into two classes based on their location in the cell: one is the receptor-like PTPs represented by CD45 and PTP-$\alpha$ and the like, which contains a transmembrane region, several domains on one side of the cytoplasm, and a domain that can bind to a ligand outside the cell; the other is the intracellular PTPs represented by PTP1B, SHP1 and SHP2, which contains a single catalytic domain. The N- or C-terminal of this type of PTP has a variety of structures, for example, SH-PTP is a PTP with several tandem SH domains, and these SH structures play an important role in locating and regulating.

Protein tyrosine phosphatases containing Src homology domains (also known as Src homology domain PTP, SH-PTP) are non-receptor tyrosine phosphatases encoded by a series of PTPN genes, including PTP1B, SHP1, SHP2, TCPTP and MEG-PTP, etc. SH-PTP is generally expressed in various tissues and cell types, in which PTP1B contains only one tyrosine phosphatase activity domain and is one of the smallest protein tyrosine phosphatases. Both SHP1 and SHP2 comprise a conserved tyrosine phosphatase domain, two tandem SH2 domains connected in different ways, and a C-terminal tail structure. The structure and arrangement of the two tandem SH2 domains determine the different subcellular localization and regulatory functions of SHP1 and SHP2. In their non-activated state, the two SH2 domains will bind to the PTP domain, cover the active region, and inactivate it. When the SH2 domains bind to a specific tyrosine residue on a receptor or an adaptor protein, the PTP domain will be exposed. For example, the exposure of the catalytic site caused by stimulation of cytokines and growth factors leads to the activation of such enzymes. They play an important role in various signaling pathways that regulate cellular biological processes and are involved in the signaling pathways of various growth factors and cytokines. Within a single signaling pathway, PTP family members can simultaneously play a positive (signal enhancement) role and a negative (signal attenuation) role during intracellular signaling; it acts by attenuating local signaling flow through dephosphorylating its related signaling molecules. For example, SHP2 is a positive regulator of the Ras-Raf pathway and the ERK/MAPK signaling pathway, which plays a key role in regulating cell proliferation and survival. (For an overview of SHP2 phosphatases, see (e.g.) K. S. Grossman et al., Adv. Cancer Res .2010, 106, 53-89; and references cited therein).

In its basal state, SHP2 is typically automatically inhibited due to the intramolecular interactions between its N-terminal SH2 (N-SH2) domain and its catalytic (PTP) domain, thereby blocking access to the catalytic site. The activation of proteins that interact with the SH2 domains induces a conformational change that reverses this inhibition and allows substrate access to the catalytic site. Mutations in the PTPN11 gene may affect mutations of the N-SH2 or PTP domain residues that are involved in the basic inhibition of SHP2 to produce a more easily activated form of SHP2 protein, which can lead to unregulated or increased SHP2 activity. SHP2 is widely expressed and participates in multiple cellular signaling processes, such as the Ras-Erk, PI3K-Akt, Jak-Stat, Met, FGFR, and EGFR, as well as the insulin receptor and NF-kB pathway, which plays a crucial role in cell proliferation, differentiation, cell cycle, and migration. Hyperactivation of SHP2 caused by germline or somatic mutations has been found in Noonan Syndrome, Leopard Syndrome, Juvenile myelomonocytic leukemia, myelodysplastic syndrome, B cell acute lymphoblastic leukemia, and acute myeloid leukemia. In addition, the activating mutations of PTPN11 have also been found in solid tumors, such as lung cancer, colon cancer, ovarian cancer, cervical cancer, prostate cancer, melanoma, neuroblastoma, and liver cancer. Furthermore, phosphatases such as TCPTP and PTP-MEG 2 have also been reported to be highly correlated with the occurrence and development of blood and solid tumors. Therefore, the activated or upregulated phosphatases in human tumors or other diseases become a new therapeutic target, and there is an urgent need to develop formulations for intracellular protein phosphatases including SHP2.

## SUMMARY

In one aspect, the present disclosure provides a compound represented by formula (I), or a pharmaceutically acceptable salt, a stereoisomer, a tautomer, a cis-trans isomer, a solvate, a hydrate, a polymorph, a prodrug or an isotope variant thereof, and a mixture thereof:

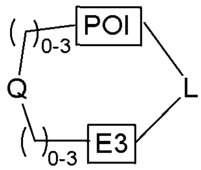
(I)

wherein,

the POI is

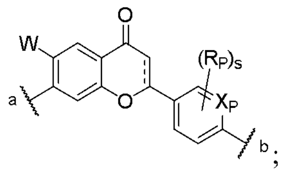

- - - represents a single bond or a double bond, alternatively a double bond;
each $R_P$ is independently selected from H, D, halogen, $C_{1-6}$ alkyl, $C_{1-6}$ haloalkyl, $C_{2-6}$ alkenyl and $C_{2-6}$ alkynyl;
s is selected from 1, 2 and 3;
$X_P$ is selected from N and $CR_P$;
W is selected from H, D, halogen, $C_{1-6}$ alkyl, $C_{1-6}$ haloalkyl, $C_{2-6}$ alkenyl, $C_{2-6}$ alkynyl, $C_{6-10}$ aryl and 5- to 10-membered heteroaryl;
said W is optionally substituted with 1, 2 or 3 $R_W$;
Rw is selected from H, D, $-C(O)R_a$, $-C(O)NR_bR_c$, $-C(O)OR_a$ or $-OC(O)R_a$, $-NR_bC(O)R_a$, $C_{1-6}$ alkyl, $C_{1-6}$ haloalkyl, $C_{2-6}$ alkenyl and $C_{2-6}$ alkynyl;
the a-terminal of the POI is connected to Q via $C_{0-3}$ alkylene, and the b-terminal is connected to L;
L is

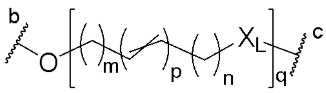
;

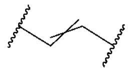

represents a single bond, a cis- or trans-double bond, alternatively a cis-double bond;
each $X_L$ is independently selected from a chemical bond, -O-, $C_{1-6}$ alkylene, $C_{2-6}$ alkenylene, $C_{2-6}$ alkynylene, $C_{3-10}$ cycloalkylene, 5- to 10-membered heterocyclylene, $C_{6-10}$ arylene and 5- to 10-membered heteroarylene; the $X_L$ is optionally deuterated, up to completely deuterated;
each m is independently selected from 0, 1, 2, 3, 4, 5, 6, 7, 8, 9 and 10;
each n is independently selected from 0, 1, 2, 3, 4, 5, 6, 7, 8, 9 and 10;
each p is independently selected from 0, 1, 2, 3, 4 and 5;
each q is independently selected from 1, 2, 3, 4 and 5;
the b-terminal of L is connected to POI, and the c-terminal is connected to E3, or the c-terminal of L is connected to POI, and the b-terminal is connected to E3;
E3 is

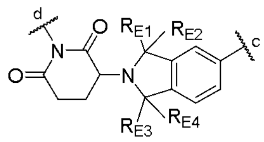

;

$R_{E1}$, $R_{E2}$, $R_{E3}$ or $R_{E4}$ are independently selected from H and D, and at least one pair of $R_{E1}$ and $R_{E2}$, $R_{E3}$ and $R_{E4}$ represents =O, =S or =N-OH;

the c-terminal of E3 is connected to L, and the d-terminal is connected to Q via $C_{0-3}$ alkylene;

Q is

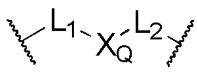

;

$L_1$ and $L_2$ are independently selected from NH and O;

$X_Q$ is selected from -P(O)($Y_Q$-$R_Q$)-, -S(O)- and -C(O)-$X_{Q1}$-C(O)-;

$Y_Q$ is selected from NH and O;

$X_{Q1}$ is selected from a chemical bond, $C_{1-6}$ alkylene, $C_{2-6}$ alkenylene, $C_{2-6}$ alkynylene, -$C_{1-6}$ alkylene-O-$C_{1-6}$ alkylene, -$C_{1-6}$ alkylene-N$R_b$-$C_{1-6}$ alkylene, $C_{3-10}$ cycloalkylene, 5- to 10-membered heterocyclylene, $C_{6-10}$ arylene and 5- to 10-membered heteroarylene, and the $X_{Q1}$ is optionally substituted with 1, 2, 3, 4 or 5 independently selected R#;

$R_Q$ is selected from H, $C_{1-6}$ alkyl, $C_{1-6}$ haloalkyl, $C_{2-6}$ alkenyl, $C_{2-6}$ alkynyl, $C_{3-10}$ cycloalkyl, 5- to 10-membered heterocyclyl, $C_{6-10}$ aryl and 5- to 10-membered heteroaryl;

$R_a$, $R_b$ and $R_c$ are independently selected from H, $C_{1-6}$ alkyl and $C_{1-6}$ haloalkyl;

R# is selected from H, halogen, OH, $NH_2$, $C_{1-6}$ alkyl, $C_{1-6}$ haloalkyl, $C_{2-6}$ alkenyl and $C_{2-6}$ alkynyl.

In another aspect, the present disclosure provides a pharmaceutical composition comprising a compound of the present disclosure, and optionally a pharmaceutically acceptable excipient, such as a carrier, adjuvant or vehicle.

In another aspect, the present disclosure provides a pharmaceutical composition comprising a compound of the present disclosure and a pharmaceutically acceptable excipient, which further comprises an additional therapeutic agent.

In another aspect, the present disclosure provides use of a compound of the present disclosure in the manufacture of a medicament for the treatment and/or prevention of a tumor disease.

In another aspect, the present disclosure provides use of a compound of the present disclosure in the manufacture of a medicament for the treatment and/or prevention of a hematological tumor or a solid tumor disease.

In another aspect, the present disclosure provides a method for treating and/or preventing a tumor disease in a subject, comprising administering to the subject a compound of the present disclosure or a pharmaceutical composition of the present disclosure.

In another aspect, the present disclosure provides a method for treating and/or preventing a hematological tumor or a solid tumor disease in a subject, comprising administering to the subject a compound or a pharmaceutical composition of the present disclosure.

In another aspect, the present disclosure provides a compound of the present disclosure or a pharmaceutical composition of the present disclosure for use in the treatment and/or prevention of a tumor disease.

In another aspect, the present disclosure provides a compound of the present disclosure or a pharmaceutical composition of the present disclosure for use in the treatment and/or prevention of a hematological tumor or a solid tumor disease.

In a specific embodiment, the present disclosure is used in the treatment and/or prevention of solid tumors such as breast cancer, lung cancer, neuroblastoma, oral squamous cell carcinoma, colorectal tumor, ovarian cancer, cervical cancer, prostate cancer and pancreatic cancer, hematological tumor diseases such as myeloid leukemia and lymphoid leukemia, and other relapsed and refractory advanced solid and hematological tumors.

## Definition

### Chemical definitions

Definitions of specific functional groups and chemical terms are described in more detail below.

When a range of values is listed, it is intended to encompass each value and sub-range within the range. For example, "$C_{1-6}$ alkyl" is intended to include $C_1$, $C_2$, $C_3$, $C_4$, Cs, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$ and $C_{5-6}$ alkyl.

"$C_{1-6}$ alkyl" refers to a radical of a straight or branched, saturated hydrocarbon group having 1 to 6 carbon atoms. In some embodiments, $C_{1-4}$ alkyl and $C_{1-2}$ alkyl are alternative. Examples of $C_{1-6}$ alkyl include methyl ($C_1$), ethyl ($C_2$), n-propyl ($C_3$), iso-propyl ($C_3$), n-butyl ($C_4$), tert-butyl ($C_4$), sec-butyl ($C_4$), iso-butyl ($C_4$), n-pentyl ($C_5$), 3-pentyl ($C_5$), pentyl ($C_5$), neopentyl (Cs), 3-methyl-2-butyl ($C_5$), tert-pentyl ($C_5$) and n-hexyl ($C_6$). The term "$C_{1-6}$ alkyl" also includes heteroalkyl, wherein one or more (e.g., 1, 2, 3 or 4) carbon atoms are subsituted with heteroatoms (e.g., oxygen, sulfur, nitrogen, boron, silicon, phosphorus). Alkyl groups can be optionally substituted with one or more substituents, for example, with 1 to 5 substituents, 1 to 3 substituents or 1 substituent. Conventional abbreviations of alkyl include Me ($-CH_3$), Et ($-CH_2CH_3$), iPr ($-CH(CH_3)_2$), nPr ($-CH_2CH_2CH_3$), n-Bu ($-CH_2CH_2CH_2CH_3$) or i-Bu ($-CH_2CH(CH_3)_2$).

"$C_{1-6}$ alkylene" refers to a divalent group formed by removing another hydrogen from the $C_{1-6}$ alkyl, and can be substituted or unsubstituted. In some embodiments, $C_{1-4}$ alkylene, $C_{2-4}$ alkylene and $C_{1-3}$ alkylene are alternative. The unsubstituted alkylene groups include, but are not limited to, methylene ($-CH_2-$), ethylene ($-CH_2CH_2-$), propylene ($-CH_2CH_2CH_2-$), butylene ($-CH_2CH_2CH_2CH_2-$), pentylene ($-CH_2CH_2CH_2CH_2CH_2-$), hexylene ($-CH_2CH_2CH_2CH_2CH_2CH_2-$), etc. Examples of substituted alkylene groups, such as those substituted with one or more alkyl (methyl) groups, include, but are not limited to, substituted methylene ($-CH(CH_3)-$, $-C(CH_3)_2-$), substituted ethylene ($-CH(CH_3)CH_2-$, $-CH_2CH(CH_3)-$, $-C(CH_3)_2CH_2-$, $-CH_2C(CH_3)_2-$), substituted propylene ($-CH(CH_3)CH_2CH_2-$, $-CH_2CH(CH_3)CH_2-$, $-CH_2CH_2CH(CH_3)-$, $-C(CH_3)_2CH_2CH_2-$, $-CH_2C(CH_3)_2CH_2-$, $-CH_2CH_2C(CH_3)_2-$), etc.

"$C_{2-6}$ alkenyl" refers to a radical of a straight or branched hydrocarbon group having 2 to 6 carbon atoms and at least one carbon-carbon double bond. In some embodiments, $C_{2-4}$ alkenyl is alternative. Examples of $C_{2-6}$ alkenyl include vinyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl ($C_5$), hexenyl ($C_6$), etc. The term "$C_{2-6}$ alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). The alkenyl groups can be optionally substituted with one or more substituents, for example, with 1 to 5 substituents, 1 to 3 substituents or 1 substituent.

"$C_{2-6}$ alkenylene" refers to a $C_{2-6}$ alkenyl group wherein another hydrogen is removed to provide a divalent radical of alkenylene, and which may be substituted or unsubstituted. In some embodiments, $C_{2-4}$ alkenylene is alternative.

"$C_{2-6}$ alkynyl" refers to a radical of a straight or branched hydrocarbon group having 2 to 6 carbon atoms, at least one carbon-carbon triple bond and optionally one or more carbon-carbon double bonds. In some embodiments, $C_{2-4}$ alkynyl is alternative. Examples of $C_{2-6}$ alkynyl include, but are not limited to, ethynyl ($C_2$), 1-propynyl ($C_3$), 2-propynyl ($C_3$), 1-butynyl ($C_4$), 2-butynyl ($C_4$), pentynyl ($C_5$), hexynyl ($C_6$), etc. The term "$C_{2-6}$ alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). The alkynyl groups can be substituted with one or more substituents, for example, with 1 to 5 substituents, 1 to 3 substituents or 1 substituent.

"$C_{2-6}$ alkynylene" refers to a $C_{2-6}$ alkynyl group wherein another hydrogen is removed to provide a divalent radical of alkynylene, and which may be substituted or unsubstituted. In some embodiments, $C_{2-4}$ alkynylene is alternative.

"Halo" or "halogen" refers to fluorine (F), chlorine (Cl), bromine (Br) and iodine (I).

Thus, "$C_{1-6}$ haloalkyl" refers to the above "$C_{1-6}$ alkyl", which is substituted with one or more halogen. In some embodiments, $C_{1-4}$ haloalkyl is yet alternative, and still alternatively $C_{1-2}$ haloalkyl. Exemplary haloalkyl groups include, but are not limited to, $-CF_3$, $-CH_2F$, $-CHF_2$, $-CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, and the like. The haloalkyl can be substituted at any available point of attachment, for example, with 1 to 5 substituents, 1 to 3 substituents or 1 substituent.

"$C_{3-10}$ cycloalkyl" refers to a radical of a non-aromatic cyclic hydrocarbon group having from 3 to 10 ring carbon atoms and zero heteroatoms. In some embodiments, $C_{3-8}$ cycloalkyl, $C_{3-7}$ cycloalkyl and $C_{3-5}$ cycloalkyl are yet alternative, and still alternatively $C_{5-6}$ cycloalkyl. The cycloalkyl also includes a ring system in which the cycloalkyl described herein is fused with one or more aryl or heteroaryl groups, wherein the point of attachment is on the cycloalkyl ring, and in such case, the number of carbon atoms continues to represent the number of carbon atoms in the cycloalkyl system. Exemplary cycloalkyl groups include, but are not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), etc. The cycloalkyl groups can be substituted with one or more substituents, for example, with 1 to 5 substituents, 1 to 3 substituents or 1 substituent.

"$C_{3-10}$ cycloalkylene" refers to a divalent group formed by removing another hydrogen from the $C_{3-10}$ cycloalkyl, and can be substituted or unsubstituted. In some embodiments, $C_{3-7}$ cycloalkylene, $C_{3-5}$ cycloalkylene and $C_{5-6}$ cycloalkylene are alternative.

"5- to 10-membered heterocyclyl" refers to a saturated or unsaturated radical of 5- to 10-membered non-aromatic ring system having ring carbon atoms and 1 to 4 ring heteroatoms, wherein each of the heteroatoms is independently selected from nitrogen, oxygen, sulfur, boron, phosphorus and silicon. In the heterocyclyl containing one or more nitrogen atoms, the point of attachment can be a carbon or nitrogen atom as long as the valence permits. In some embodiments, 4- to 7-membered heterocyclyl is alternative, which is a radical of 4- to 7-membered non-aromatic ring system having ring carbon atoms and 1 to 3 ring heteroatoms; 5- to 6-membered heterocyclyl is yet alternative, which is a radical of 5- to 6-membered non-aromatic ring system having ring carbon atoms and 1 to 3 ring heteroatoms. The heterocyclyl also includes a ring system wherein the heterocyclyl described above is fused with one or more cycloalkyl groups, wherein the point of attachment is on the cycloalkyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. Exemplary 3-membered heterocyclyl groups containing one heteroatom include, but are not limited to, aziridinyl, oxiranyl and thiorenyl. Exemplary 5-membered heterocyclyl groups containing two heteroatoms include, but are not limited to, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocycly groups containing one heteroatom include, but are not limited to, azepanyl, oxepanyl and thiepanyl. Exemplary 5-membered heterocyclyl groups fused with a $C_6$ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a $C_6$ aryl (also referred as 6,6-bicyclic heterocyclyl herein) include, but are not limited to, tetrahydroquinolinyl, tetrahydroisoquinolinyl, etc. The heterocyclyl also includes a bridged ring or a spiral ring formed by the above heterocyclyl and a cycloalkyl, heterocyclyl, aryl or heteroaryl group sharing one or two atoms, and the shared atom can be a carbon or nitrogen atom as long as the valence permits. The heterocyclyl also includes the above heterocyclyl and heterocyclyl groups which can be optionally substituted with one or more substituents, for example, with 1 to 5 substituents, 1 to 3 substituents or 1 substituent.

"5- to 10-membered heterocyclylene" refers to a divalent group formed by removing another hydrogen from the 5- to 10-membered heterocyclyl, and can be substituted or unsubstituted. In some embodiments, 5- to 6-membered heterocyclylene is alternative.

"$C_{6-10}$ aryl" refers to a radical of monocyclic or polycyclic (e.g., bicyclic) 4n+2 aromatic ring system having 6 to 10 ring carbon atoms and zero heteroatoms (e.g., having 6 or 10 shared $\pi$ electrons in a cyclic array). In some embodiments, the aryl group has six ring carbon atoms ("$C_6$ aryl"; for example, phenyl). In some embodiments, the aryl group has ten ring carbon atoms ("$C_{10}$ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. The aryl can be substituted with one or more substituents, for example, with 1 to 5 substituents, 1 to 3 substituents or 1 substituent.

"$C_{6-10}$ arylene" refers to a divalent group formed by removing another hydrogen from the $C_{6-10}$ aryl, and can be substituted or unsubstituted. In some embodiments, phenylene is alternative.

"5- to 10-membered heteroaryl" refers to a radical of 5- to 10-membered monocyclic or bicyclic 4n+2 aromatic ring system (e.g., having 6 or 10 shared $\pi$ electrons in a cyclic array) having ring carbon atoms and 1 to 4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more cycloalkyl or heterocyclyl groups, and the point of attachment is on the heteroaryl ring. In such case, the number the carbon atoms continues to represent the number of carbon atoms in the heteroaryl ring system. In some embodiments, 5- to 6-membered heteroaryl groups are alternative, which are radicals of 5- to 6-membered monocyclic or bicyclic 4n+2 aromatic ring systems having ring carbon atoms and 1 to 4 ring heteroatoms. Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,4-oxadiazoly), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl. The heteroaryl can be substituted with one or more substituents, for example, with 1 to 5 substituents, 1 to 3 substituents or 1 substituent.

"5- to 10-membered heteroarylene" refers to a divalent group formed by removing another hydrogen from the 5- to 10-membered heteroaryl, and can be substituted or unsubstituted. In some embodiments, 5- to 6-membered heteroarylene is alternative.

Ring-forming groups such as cycloalkyl, cycloalkylene, heterocyclyl, heterocyclylene, aryl, arylene, heteroaryl and heteroarylene defined above are collectively referred to as "cyclyl groups".

Alkyl, alkylene, alkenyl, alkenylene, alkynyl, alkynylene, cycloalkyl, cycloalkylene, heterocyclyl, heterocyclylene, aryl, arylene, heteroaryl and heteroarylene as defined herein are optionally substituted groups.

Exemplary substituents on carbon atoms include, but are not limited to, halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3{}^+X^-$, $-N(OR^{cc})P^{bb}$, -SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, $-C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, $-C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, $-OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, $-C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, $-OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, $-P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, cycloalkyl, heterocyclyl, aryl and heteroaryl, wherein each of the alkyl, alkenyl, alkynyl, cycloalkyl, heterocyclyl, aryl and heteroaryl is independently substituted with 0, 1, 2, 3, 4 or 5 $R^{dd}$ groups;

or two geminal hydrogen on a carbon atom are replaced with =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$ or $=NOR^{cc}$ groups;

each of the $R^{aa}$ is independently selected from alkyl, haloalkyl, alkenyl, alkynyl, cycloalkyl, heterocyclyl, aryl and heteroaryl, or two of the $R^{aa}$ groups are combined to form a heterocyclyl or heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{bb}$ is independently selected from hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, cycloalkyl, heterocyclyl, aryl and heteroaryl, or two $R^{bb}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{cc}$ is independently selected from hydrogen, alkyl, haloalkyl, alkenyl, alkynyl, cycloalkyl, heterocyclyl, aryl and heteroaryl, or two $R^{cc}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{dd}$ is independently selected from halogen, -CN, $-NO_2$, $-N_3$, $-SO_2H$, $-SO_3H$, -OH, $-OR^{ee}$, $-ON(R^{ff})_2$, $-N(R^{ff})_2$, $-N(R^{ff})_3{}^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, $-OCO_2R^{ee}$, $-C(=O)N(R^{ff})_2$, $-OC(=O)N(R^{ff})_2$, $-NR^{ff}C(=O)R^{ee}$, $-NR^{ff}CO_2R^{ee}$, $-NR^{ff}C(=O)N(R^{ff})_2$, $-C(=NR^{ff})OR^{ee}$, $-OC(=NR^{ff})R^{ee}$, $-OC(=NR^{ff})OR^{ee}$, $-C(=NR^{ff})N(R^{ff})_2$, $-OC(=NR^{ff})N(R^{ff})_2$, $-NR^{ff}C(=NR^{ff})N(R^{ff})_2$, $-NR^{ff}SO_2R^{ee}$, $-SO_2N(R^{ff})_2$, $-SO_2R^{ee}$, $-SO_2OR^{ee}$, $-OSO_2R^{ee}$, $-S(=O)R^{ee}$, $-Si(R^{ee})_3$, $-OSi(R^{ee})_3$, $-C(=S)N(R^{ff})_2$, $-C(=O)SR^{ee}$, $-C(=S)SR^{ee}$, $-SC(=S)SR^{ee}$, $-P(=O)_2R^{ee}$, $-P(=O)(R^{ee})_2$, $-OP(=O)(R^{ee})_2$, $-OP(=O)(OR^{ee})_2$, alkyl, haloalkyl, alkenyl, alkynyl, cycloalkyl, heterocyclyl, aryl, and heteroaryl, wherein, each of the alkyl, alkenyl, alkynyl, cycloalkyl, heterocyclyl, aryl and heteroaryl is independently substituted with 0, 1, 2, 3, 4 or 5 $R^{gg}$ groups, or two geminal $R^{dd}$ substituents can be combined to form=O or =S;

each of the $R^{ee}$ is independently selected from alkyl, haloalkyl, alkenyl, alkynyl, cycloalkyl, aryl, heterocyclyl, and heteroaryl, wherein, each of the alkyl, alkenyl, alkynyl, cycloalkyl, heterocyclyl, aryl and heteroaryl is independently substituted with 0, 1, 2, 3, 4 or 5 $R^{gg}$ groups;

each of the $R^{ff}$ is independently selected from hydrogen, alkyl, haloalkyl, alkenyl, alkynyl, cycloalkyl, heterocyclyl, aryl and heteroaryl, or two $R^{ff}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{gg}$ groups;

each of the $R^{gg}$ is independently halogen, -CN, $-NO_2$, $-N_3$, $-SO_2H$, $-SO_3H$, -OH, $-OC_{1-6}$ alkyl, $-ON(C_{1-6}$ alkyl$)_2$, $-N(C_{1-6}$ alkyl$)_2$, $-N(C_{1-6}$ alkyl$)_3{}^+X^-$, $-NH(C_{1-6}$ alkyl$)_2{}^+X^-$, $-NH_2(C_{1-6}$ alkyl$)^+X^-$, $-NH_3{}^+X^-$, $-N(OC_{1-6}$ alkyl$)(C_{1-6}$ alkyl), $-N(OH)(C_{1-6}$ alkyl), -NH(OH), -SH, $-SC_{1-6}$ alkyl, $-SS(C_{1-6}$ alkyl), $-C(=O)(C_{1-6}$ alkyl), $-CO_2H$, $-CO_2(C_{1-6}$ alkyl), $-OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl$)C(=O)(C_{1-6}$ alkyl), $-NHCO_2(C_{1-6}$ alkyl), $-NHC(=O)N(C_{1-6}$ alkyl$)_2$, $-NHC(=O)NH(C_{1-6}$ alkyl), $-NHC(=O)NH_2$, $-C(=NH)O(C_{1-6}$ alkyl), $-OC(=NH)(C_{1-6}$ alkyl), $-OC(=NH)OC_{1-6}$ alkyl, $-C(=NH)N(C_{1-6}$ alkyl$)_2$, $-C(=NH)NH(C_{1-6}$ alkyl), $-C(=NH)NH_2$, $-OC(=NH)N(C_{1-6}$ alkyl$)_2$, $-OC(NH)NH(C_{1-6}$ alkyl), $-OC(NH)NH_2$, $-NHC(NH)N(C_{1-6}$ alkyl$)_2$, $-NHC(=NH)NH_2$,

-$NHSO_2(C_{1-6}$ alkyl), -$SO_2N(C_{1-6}$ alkyl$)_2$, -$SO_2NH(C_{1-6}$ alkyl), -$SO_2NH_2$, -$SO_2C_{1-6}$ alkyl, -$SO_2OC_{1-6}$ alkyl, -$OSO_2C_{1-6}$ alkyl, -$SOC_{1-6}$ alkyl, -$Si(C_{1-6}$ alkyl$)_3$, -$OSi(C_{1-6}$ alkyl$)_3$, -C(=S)N($C_{1-6}$ alkyl$)_2$, C(=S)NH($C_{1-6}$ alkyl), C(=S)$NH_2$, -C(=O)S($C_{1-6}$ alkyl), -C(=S)$SC_{1-6}$ alkyl, -SC(=S)$SC_{1-6}$ alkyl, -P(=O$)_2$($C_{1-6}$ alkyl), -P(=O)($C_{1-6}$ alkyl$)_2$, -OP(=O)($C_{1-6}$ alkyl$)_2$, -OP(=O)($OC_{1-6}$ alkyl$)_2$, $C_{1-6}$ alkyl, $C_{1-6}$ haloalkyl, $C_2$-$C_6$ alkenyl, $C_2$-$C_6$ alkynyl, $C_3$-$C_7$ cycloalkyl, $C_6$-$C_{10}$ aryl, $C_3$-$C_7$ heterocyclyl, or $C_5$-$C_{10}$ heteroaryl; or two geminal $R^{gg}$ substituents may combine to form =O or =S; wherein, $X^-$ is a counter-ion.

Exemplary substituents on nitrogen atoms include, but are not limited to, hydrogen, -OH, -$OR^{aa}$, -$N(R^{cc})_2$, -CN, -C(=O)$R^{aa}$, -C(=O)N($R^{cc})_2$, -$CO_2R^{aa}$, -$SO_2R^{aa}$, -C(=$NR^{bb}$)$R^{aa}$, -C(=$NR^{cc}$)$OR^{aa}$, -C(=$NR^{cc}$)N($R^{cc})_2$, -$SO_2N(R^{cc})_2$, -$SO_2R^{cc}$, -$SO_2OR^{cc}$, -$SOR^{aa}$, -C(=S)N($R^{cc})_2$, -C(=O)$SR^{cc}$, -C(=S)$SR^{cc}$, -P(=O$)_2R^{aa}$, -P(=O)($R^{aa})_2$, -P(=O$)_2$N($R^{cc})_2$, -P(=O)($NR^{cc})_2$, alkyl, haloalkyl, alkenyl, alkynyl, cycloalkyl, heterocyclyl, aryl and heteroaryl, or two $R^{cc}$ groups attached to a nitrogen atom combine to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as described herein.

As used herein, unless otherwise specified, the divalent structure may be attached to the remainder of the compound in any direction from left to right or from right to left (or, from top to bottom or from bottom to top). In one embodiment, alternatively, the divalent structure is attached to the remainder of the compound in a direction from left to right. For example, in General Formula (I),

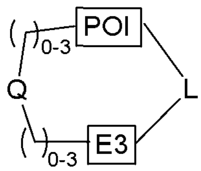
(I)

when Q is defined as

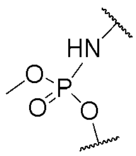
,

it represents the following two connection modes:

(1) 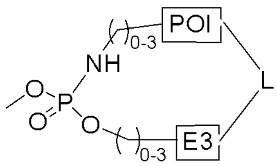 ; (2) 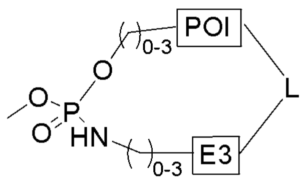 .

Other divalent structures should be understood similarly.

Other definitions

The term "cancer" includes, but is not limited to, the following cancers: pancreatic cancer, lung cancer, colorectal cancer, bile duct cancer, multiple myeloma, melanoma, uterine cancer, endometrial cancer, thyroid cancer, acute myeloid leukemia, bladder cancer, urothelial carcinoma, gastric cancer, cervical cancer, ovarian cancer, head and neck squamous cell carcinoma, diffuse large B-cell lymphoma, esophageal cancer, chronic lymphocytic leukemia, hepatocellular carcinoma, breast cancer, ovarian cancer, prostate cancer, glioblastoma, renal cancer, and sarcoma.

The term "treating" as used herein relates to reversing, alleviating or inhibiting the progression or prevention of the disorders or conditions to which the term applies, or of one or more symptoms of such disorders or conditions. The noun "treatment" as used herein relates to the action of treating, which is a verb, and the latter is as just defined.

The term "pharmaceutically acceptable salt" as used herein refers to those carboxylate and amino acid addition salts of the compounds of the present disclosure, which are suitable for the contact with patients' tissues within a reliable

medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

The pharmaceutically acceptable base addition salts are formed with metals or amines, such as alkali metal and alkaline earth metal hydroxides or organic amines. Examples of the metals used as cations include sodium, potassium, magnesium, calcium, etc. Examples of suitable amines are N,N'-dibenzylethylenediamine, chloroprocaine, choline, diethanolamine, ethylenediamine, N-methylglucamine and procaine.

The base addition salt of the acidic compound can be prepared by contacting the free acid form with a sufficient amount of the required base to form a salt in a conventional manner. The free acid can be regenerated by contacting the salt form with an acid in a conventional manner and then isolating the free acid. The free acid forms are somewhat different from their respective salt forms in their physical properties, such as solubility in polar solvents. But for the purposes of the present disclosure, the salts are still equivalent to their respective free acids.

The salts can be prepared from the inorganic acids, which include sulfates, pyrosulfates, bisulfates, sulfites, bisulfites, nitrates, phosphates, monohydrogen phosphates, dihydrogen phosphates, metaphosphates, pyrophosphates, chlorides, bromides and iodides. Examples of the acids include hydrochloric acid, nitric acid, sulfuric acid, hydrobromic acid, hydroiodic acid, phosphoric acid, etc. The representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthalate, methanesulfonate, glucoheptanate, lactobionate, lauryl sulfonate, isethionate, etc. The salts can also be prepared from the organic acids, which include aliphatic monocarboxylic and dicarboxylic acids, phenyl-substituted alkanoic acids, hydroxyalkanoic acids, alkanedioic acid, aromatic acids, aliphatic and aromatic sulfonic acids, etc. The representative salts include acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, besylate, tosylate, phenylacetate, citrate, lactate, maleate, tartrate, methanesulfonate, etc. The pharmaceutically acceptable salts can include cations based on alkali metals and alkaline earth metals, such as sodium, lithium, potassium, calcium, magnesium, etc., as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc. Salts of amino acids are also included, such as arginine salts, gluconates, galacturonates, etc. (for example, see Berge S. M. et al., "Pharmaceutical Salts," J. Pharm. Sci., 1977; 66: 1- 19 for reference).

"Subjects" to which administration is contemplated include, but are not limited to, humans (e.g., males or females of any age group, e.g., paediatric subjects (e.g., infants, children, adolescents) or adult subjects (e.g., young adults, middle-aged adults or older adults)) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "human", "patient" and "subject" can be used interchangeably herein.

"Disease," "disorder," and "condition" can be used interchangeably herein.

Unless indicated, otherwise the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment").

Generally, the "effective amount" of a compound refers to an amount sufficient to elicit a target biological response. As understood by those skilled in the art, the effective amount of the compound of the disclosure can vary depending on the following factors, such as the desired biological endpoint, the pharmacokinetics of the compound, the diseases being treated, the mode of administration, and the age, health status and symptoms of the subjects. The effective amount includes therapeutically effective amount and prophylactically effective amount.

Unless indicated, otherwise the "therapeutically effective amount" of the compound as used herein is an amount sufficient to provide therapeutic benefits in the course of treating a disease, disorder or condition, or to delay or minimize one or more symptoms associated with the disease, disorder or condition. The therapeutically effective amount of a compound refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless indicated, otherwise the "prophylactically effective amount" of the compound as used herein is an amount sufficient to prevent a disease, disorder or condition, or an amount sufficient to prevent one or more symptoms associated with a disease, disorder or condition, or an amount sufficient to prevent the recurrence of a disease, disorder or condition. The prophylactically effective amount of a compound refers to the amount of a therapeutic agent that, when used alone or in combination with other agents, provides a prophylactic benefit in the prevention of a disease, disorder or condition. The term "prophylactically effective amount" can include an amount that improves the overall prevention, or an amount that

enhances the prophylactic effect of other preventive agents.

"Combination" and related terms refer to the simultaneous or sequential administration of the compounds of the present disclosure and other therapeutic agents. For example, the compounds of the present disclosure can be administered simultaneously or sequentially in separate unit dosage with other therapeutic agents, or simultaneously in a single unit dosage with other therapeutic agents.

## DETAILED DESCRIPTION

The "compounds of the present disclosure" herein refers to the following compounds of formula (I), or a pharmaceutically acceptable salt, a stereoisomer, a tautomer, a cis-trans isomer, a solvate, a hydrate, a polymorph, a prodrug or an isotope variant thereof, and a mixture thereof.

In one embodiment, the present disclosure relates to a compound represented by formula (I), or a pharmaceutically acceptable salt, a stereoisomer, a tautomer, a cis-trans isomer, a solvate, a hydrate, a polymorph, a prodrug or an isotope variant thereof, and a mixture thereof:

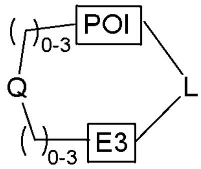

(I)

wherein,

the POI is

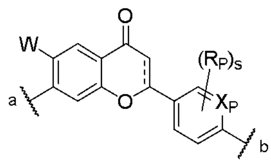

;

- - - represents a single bond or a double bond, alternatively a double bond;
each $R_P$ is independently selected from H, D, halogen, $C_{1-6}$ alkyl, $C_{1-6}$ haloalkyl, $C_{2-6}$ alkenyl and $C_{2-6}$ alkynyl;
s is selected from 1, 2 and 3;
$X_P$ is selected from N and $CR_P$;
W is selected from H, D, halogen, $C_{1-6}$ alkyl, $C_{1-6}$ haloalkyl, $C_{2-6}$ alkenyl, $C_{2-6}$ alkynyl, $C_{6-10}$ aryl and 5- to 10-membered heteroaryl;
said W is optionally substituted with 1, 2 or 3 $R_W$;
Rw is selected from H, D, $-C(O)R_a$, $-C(O)NR_bR_c$, $-C(O)OR_a$ or $-OC(O)R_a$, $-NR_bC(O)R_a$, $C_{1-6}$ alkyl, $C_{1-6}$ haloalkyl, $C_{2-6}$ alkenyl and $C_{2-6}$ alkynyl;
the a-terminal of the POI is connected to Q via $C_{0-3}$ alkylene, and the b-terminal is connected to L;
L is

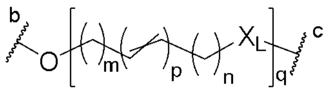

;

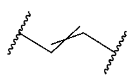

represents a single bond, a cis- or trans-double bond, alternatively a cis-double bond;
each $X_L$ is independently selected from a chemical bond, -O-, $C_{1-6}$ alkylene, $C_{2-6}$ alkenylene, $C_{2-6}$ alkynylene, $C_{3-10}$ cycloalkylene, 5- to 10-membered heterocyclylene, $C_{6-10}$ arylene and 5- to 10-membered heteroarylene; the $X_L$ is optionally deuterated, up to completely deuterated;
each m is independently selected from 0, 1, 2, 3, 4, 5, 6, 7, 8, 9 and 10;

each n is independently selected from 0, 1, 2, 3, 4, 5, 6, 7, 8, 9 and 10;
each p is independently selected from 0, 1, 2, 3, 4 and 5;
each q is independently selected from 1, 2, 3, 4 and 5;
the b-terminal of L is connected to POI, and the c-terminal is connected to E3, or the c-terminal of L is connected to POI, and the b-terminal is connected to E3;
E3 is

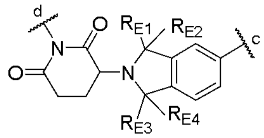

;

$R_{E1}$, $R_{E2}$, $R_{E3}$ or $R_{E4}$ are independently selected from H and D, and at least one pair of $R_{E1}$ and $R_{E2}$, $R_{E3}$ and $R_{E4}$ represents =O, =S or =N-OH;
the c-terminal of E3 is connected to L, and the d-terminal is connected to Q via $C_{0-3}$ alkylene;
Q is

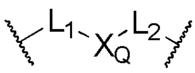

;

$L_1$ and $L_2$ are independently selected from NH and O;
$X_Q$ is selected from -P(O)($Y_Q$-$R_Q$)-, -S(O)- and -C(O)-$X_{Q1}$-C(O)-;
$Y_Q$ is selected from NH and O;
$X_{Q1}$ is selected from a chemical bond, $C_{1-6}$ alkylene, $C_{2-6}$ alkenylene, $C_{2-6}$ alkynylene, -$C_{1-6}$ alkylene-O-$C_{1-6}$ alkylene, -$C_{1-6}$ alkylene-$NR_b$-$C_{1-6}$ alkylene, $C_{3-10}$ cycloalkylene, 5- to 10-membered heterocyclylene, $C_{6-10}$ arylene and 5- to 10-membered heteroarylene, and the $X_{Q1}$ is optionally substituted with 1, 2, 3, 4 or 5 independently selected R#;
$R_Q$ is selected from H, $C_{1-6}$ alkyl, $C_{1-6}$ haloalkyl, $C_{2-6}$ alkenyl, $C_{2-6}$ alkynyl, $C_{3-10}$ cycloalkyl, 5- to 10-membered heterocyclyl, $C_{6-10}$ aryl and 5- to 10-membered heteroaryl;
$R_a$, $R_b$ and $R_c$ are independently selected from H, $C_{1-6}$ alkyl and $C_{1-6}$ haloalkyl;
R# is selected from H, halogen, OH, $NH_2$, $C_{1-6}$ alkyl, $C_{1-6}$ haloalkyl, $C_{2-6}$ alkenyl and $C_{2-6}$ alkynyl.

**POI**

In a specific embodiment, the POI is

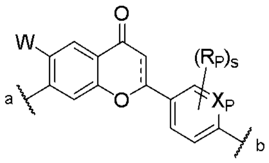

;

in another specific embodiment, the POI is

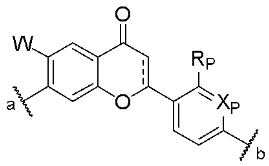

.

In a specific embodiment, the POI is

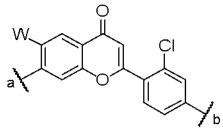

in another specific embodiment, the POI is

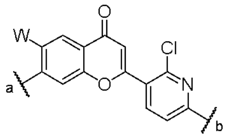

in another specific embodiment, the POI is

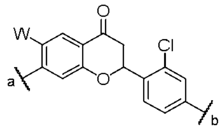

in another specific embodiment, the POI is

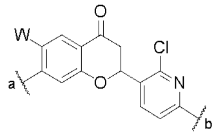

the a-terminal of the POI is connected to Q via $C_{0-3}$ alkylene, and the b-terminal is connected to L.

=

- - - represents a single bond or a double bond, alternatively a double bond;

W

In a specific embodiment, W is H; in another specific embodiment, W is D; in another specific embodiment, W is halogen; in another specific embodiment, W is $C_{1-6}$ alkyl; in another specific embodiment, W is $C_{1-4}$ alkyl; in another specific embodiment, W is $C_{1-6}$ haloalkyl; in another specific embodiment, W is $C_{1-4}$ haloalkyl; in another specific embodiment, W is $C_{2-6}$ alkenyl; in another specific embodiment, W is $C_{2-6}$ alkynyl; in another specific embodiment, W is $C_{6-10}$ aryl; in another specific embodiment, W is 5- to 10-membered heteroaryl.

In a specific embodiment, W is

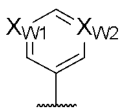

;

In a specific embodiment, W is H; in another specific embodiment, W is halogen, such as I; in another specific embodiment, W is

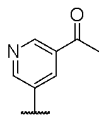

;

in another specific embodiment, W is

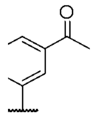

;

in another specific embodiment, W is

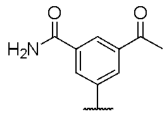

.

In a specific embodiment, W is unsubstituted; in another specific embodiment, W is substituted with 1 $R_W$; in another specific embodiment, W is substituted with 2 independently selected $R_W$; in another specific embodiment, W is substituted with 3 independently selected $R_W$.

$R_W$

In a specific embodiment, $R_W$ is H; in another specific embodiment, $R_W$ is D; in another specific embodiment, Rw is -C(O)$R_a$; in another specific embodiment, Rw is -C(O)N$R_b$$R_c$; in another specific embodiment, Rw is -C(O)O$R_a$; in another specific embodiment, Rw is -OC(O)$R_a$; in another specific embodiment, Rw is -N$R_b$C(O)$R_a$; in another specific embodiment, Rw is $C_{1-6}$ alkyl; in another specific embodiment, $R_W$ is $C_{1-6}$ haloalkyl; in another specific embodiment, $R_W$ is $C_{2-6}$ alkenyl; in another specific embodiment, $R_W$ is $C_{2-6}$ alkynyl.

$R_a$, $R_b$ and $R_c$ are independently selected from H, $C_{1-6}$ alkyl and $C_{1-6}$ haloalkyl.

$X_P$

In a specific embodiment, $X_P$ is N; in another specific embodiment, $X_P$ is $CR_P$, such as CH.

$X_{W1}$ and $X_{W2}$

In a specific embodiment, $X_{W1}$ is N; in another specific embodiment, $X_{W1}$ is $CR_W$.

In a specific embodiment, $X_{W2}$ is N; in another specific embodiment, $X_{W2}$ is $CR_W$.

$R_P$

In a specific embodiment, $R_P$ is H; in another specific embodiment, $R_P$ is D; in another specific embodiment, $R_P$ is halogen; in another specific embodiment, $R_P$ is $C_{1-6}$ alkyl; in another specific embodiment, $R_P$ is $C_{1-4}$ alkyl; in another specific embodiment, $R_P$ is $C_{1-6}$ haloalkyl; in another specific embodiment, $R_P$ is $C_{1-4}$ haloalkyl; in another specific embodiment, $R_P$ is $C_{2-6}$ alkenyl; in another specific embodiment, $R_P$ is $C_{2-6}$ alkynyl.

s

In a specific embodiment, s is 1; in another specific embodiment, s is 2; in another specific embodiment, s is 3.

L

In a specific embodiment, L is

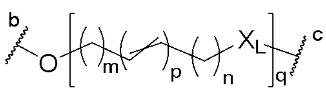

;

in another more specific embodiment, L is selected from:

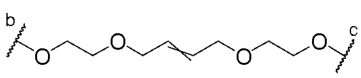

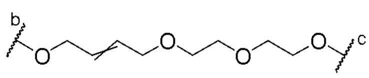

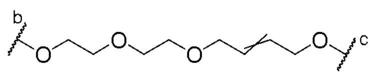

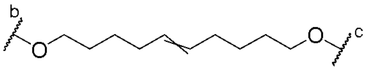 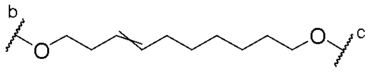 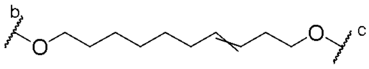

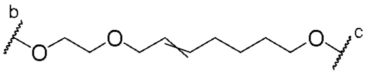 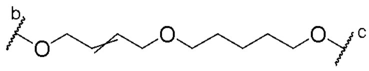 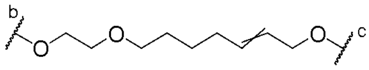

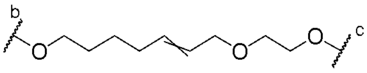 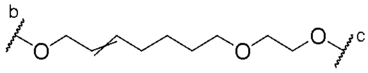 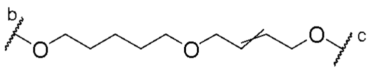

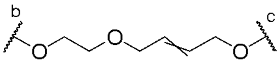 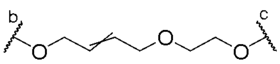 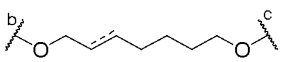 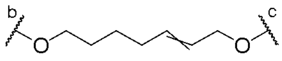

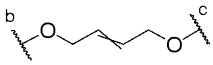 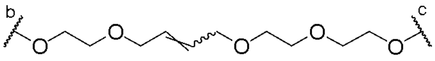 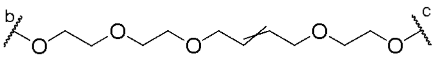

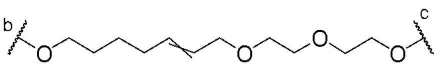 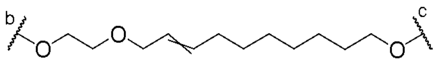

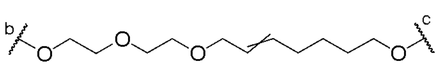 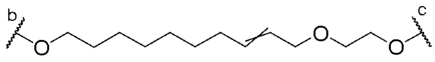

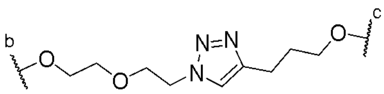 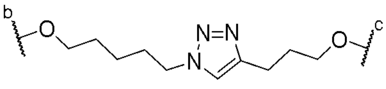 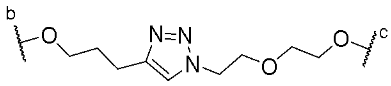

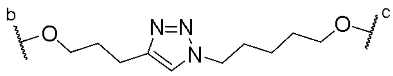 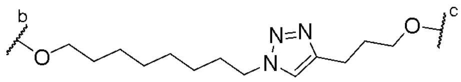

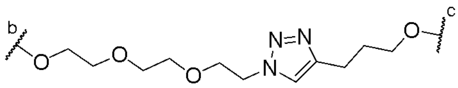 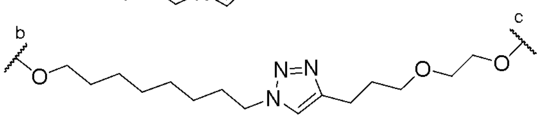

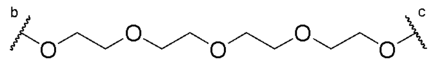.

The b-terminal of the L is connected to the POI, and the c-terminal is connected to E3, or the c-terminal of the L is connected to the POI, and the b-terminal is connected to E3.

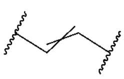

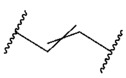

represents a single bond, a cis- or trans-double bond, alternatively a cis-double bond.

$X_L$

In a specific embodiment, $X_L$ is a chemical bond; in another specific embodiment, $X_L$ is -O-; in another specific embodiment, $X_L$ is $C_{1-6}$ alkylene, such as a $C_{1-4}$alkylene; in another specific embodiment, $X_L$ is $C_{2-6}$ alkenylene; in another specific embodiment, $X_L$ is $C_{2-6}$ alkynylene; in another specific embodiment, $X_L$ is $C_{3-10}$ cycloalkylene; in another specific embodiment, $X_L$ is 5- to 10-membered heterocyclylene; in another specific embodiment, $X_L$ is $C_{6-10}$ arylene; in another

specific embodiment, $X_L$ is 5- to 10-membered heteroarylene.

In a more specific embodiment, $X_L$ is independently selected from a chemical bond; in another more specific embodiment, $X_L$ is -O-; in another more specific embodiment, $X_L$ is $-CH_2-$; in another more specific embodiment, $X_L$ is

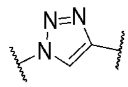
;

in another more specific embodiment, $X_L$ is

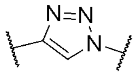
.

The $X_L$ is optionally deuterated, up to completely deuterated.

m

In a specific embodiment, m is independently selected from 0, 1, 2, 3, 4, 5, 6, 7, 8, 9 and 10.

n

In a specific embodiment, n is independently selected from 0, 1, 2, 3, 4, 5, 6, 7, 8, 9 and 10.

p

In a specific embodiment, p is independently selected from 0, 1, 2, 3, 4 and 5.

q

In a specific embodiment, q is independently selected from 1, 2, 3, 4 and 5.

E3

In a specific embodiment, E3 is

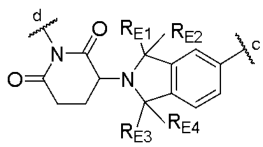
.

In a more specific embodiment, E3 is

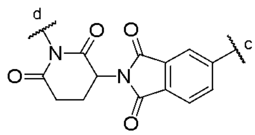
;

in another more specific embodiment, E3 is

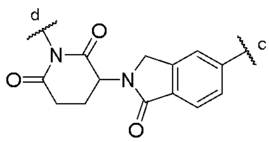
;

in another more specific embodiment, E3 is

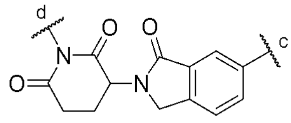.

the c-terminal of E3 is connected to L, and the d-terminal is connected to Q via $C_{0-3}$ alkylene.

$R_{E1}$, $R_{E2}$, $R_{E3}$ or $R_{E4}$

In a specific embodiment, $R_{E1}$ is H; in another specific embodiment, $R_{E1}$ is D.
In a specific embodiment, $R_{E2}$ is H; in another specific embodiment, $R_{E2}$ is D.
In a specific embodiment, $R_{E3}$ is H; in another specific embodiment, $R_{E3}$ is D.
In a specific embodiment, $R_{E4}$ is H; in another specific embodiment, $R_{E4}$ is D.
In a specific embodiment, $R_{E1}$ and $R_{E2}$ represent =O, =S or =N-OH; in another specific embodiment, $R_{E3}$ and $R_{E4}$ represent =O, =S or =N-OH; in another specific embodiment, at least one pair of $R_{E1}$ and $R_{E2}$, $R_{E3}$ and $R_{E4}$ represents =O, =S or =N-OH.

Q

In a specific embodiment, Q is

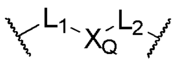.

In a more specific embodiment, Q is

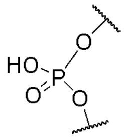;

in another more specific embodiment, Q is

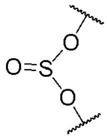;

in another more specific embodiment, Q is

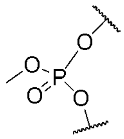;

in another more specific embodiment, Q is

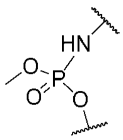;

in another more specific embodiment, Q is

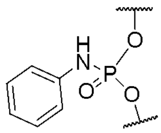

;

in another more specific embodiment, Q is

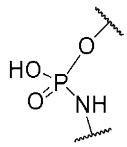

;

in another more specific embodiment, Q is

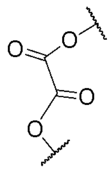

;

in another more specific embodiment, Q is

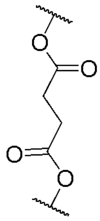

;

in another more specific embodiment, Q is

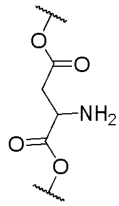

;

in another more specific embodiment, Q is

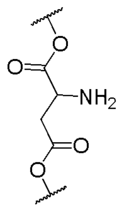

;

in another more specific embodiment, Q is

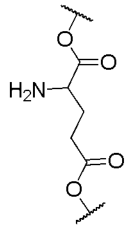
;

in another more specific embodiment, Q is

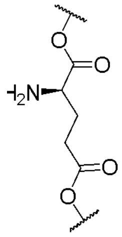
;

in another more specific embodiment, Q is

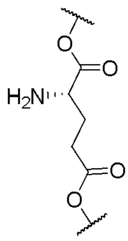
;

in another more specific embodiment, Q is

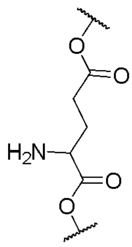
;

in another more specific embodiment, Q is

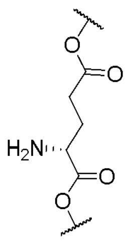
;

in another more specific embodiment, Q is

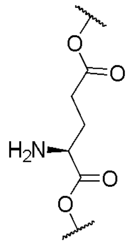

;

in another more specific embodiment, Q is

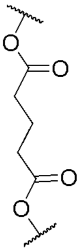

;

in another more specific embodiment, Q is

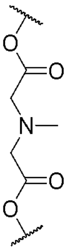

;

in another more specific embodiment, Q is

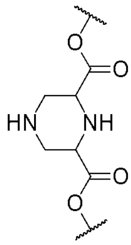

;

in another more specific embodiment, Q is

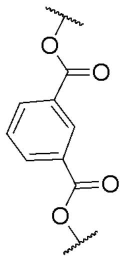

;

in another more specific embodiment, Q is

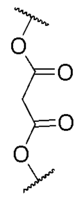

.

$L_1$ and $L_2$

In a specific embodiment, $L_1$ is NH; in another specific embodiment, $L_1$ is O.
In a specific embodiment, $L_2$ is NH; in another specific embodiment, $L_2$ is O.

$X_Q$

In a specific embodiment, $X_Q$ is -P(O)($Y_Q$-$R_Q$)-; in another specific embodiment, $X_Q$ is -S(O)-; in another specific embodiment, $X_Q$ is -C(O)-$X_{Q1}$-C(O)-.

$Y_Q$

In a specific embodiment, $Y_Q$ is NH; in another specific embodiment, $Y_Q$ is O.

$X_{Q1}$

In a specific embodiment, $X_{Q1}$ is a chemical bond; in another specific embodiment, $X_{Q1}$ is $C_{1-6}$ alkylene; in another specific embodiment, $X_{Q1}$ is $C_{1-4}$ alkylene; in another specific embodiment, $X_{Q1}$ is $C_{2-6}$ alkenylene; in another specific embodiment, $X_{Q1}$ is $C_{2-6}$ alkynylene; in another specific embodiment, $X_{Q1}$ is -$C_{1-6}$ alkylene -O-$C_{1-6}$ alkylene, such as -$C_{1-4}$ alkylene-O-$C_{1-4}$ alkylene; in another specific embodiment, $X_{Q1}$ is -$C_{1-6}$ alkylene-$NR_b$-$C_{1-6}$ alkylene, such as -$C_{1-4}$ alkylene-$NR_b$-$C_{1-4}$ alkylene; in another specific embodiment, $X_{Q1}$ is $C_{3-10}$ cycloalkylene; in another specific embodiment, $X_{Q1}$ is 5- to 10-membered heterocyclylene; in another specific embodiment, $X_{Q1}$ is 5- to 10-membered heteroarylene; in another specific embodiment, $X_{Q1}$ is 5- to 6-membered heteroarylene, such as

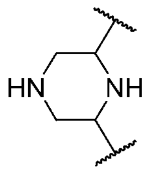

;

in another specific embodiment, $X_{Q1}$ is $C_{6-10}$ arylene; in another specific embodiment, $X_{Q1}$ is phenylene.
In a specific embodiment, $X_{Q1}$ is unsubstituted; in another specific embodiment, $X_{Q1}$ is substituted with 1 R#; in another specific embodiment, $X_{Q1}$ is substituted with 2 independently selected R#; in another specific embodiment, $X_{Q1}$ is substituted with 3 independently selected R#; in another specific embodiment, $X_{Q1}$ is substituted with 4 independently selected R#; in another specific embodiment, $X_{Q1}$ is substituted with 5 independently selected R#.

$R_Q$

In a specific embodiment, $R_Q$ is H; in another specific embodiment, $R_Q$ is $C_{1-6}$ alkyl; in another specific embodiment, $R_Q$ is $C_{1-4}$ alkyl; in another specific embodiment, $R_Q$ is $C_{2-6}$ alkenyl; in another specific embodiment, $R_Q$ is $C_{2-6}$ alkynyl; in another specific embodiment, $R_Q$ is $C_{6-10}$ aryl, such as phenyl.

**R#**

In a specific embodiment, R# is H; in another specific embodiment, R# is halogen; in another specific embodiment, R# is OH; in another specific embodiment, R# is $NH_2$; in another specific embodiment, R# is $C_{1-6}$ alkyl, such as $C_{1-4}$ alkyl; in another specific embodiment, R# is $C_{1-6}$ haloalkyl, such as $C_{1-4}$ haloalkyl; in another specific embodiment, R# is $C_{2-6}$ alkenyl; in another specific embodiment, R# is $C_{2-6}$ alkynyl.
Any technical solution in any one of the above specific embodiments, or any combination thereof, may be

combined with any technical solution in other specific embodiments or any combination thereof. For example, any technical solution of POI or any combination thereof may be combined with any technical solution of L, E3, and Q, etc., or any combination thereof. The present disclosure is intended to include all combination of such technical solutions, which are not exhaustively listed here to save space.

In a more specific embodiment, the present disclosure provides a compound of formula (I) above, or a pharmaceutically acceptable salt, an isotope variant, a tautomer, a stereoisomer, a prodrug, a polymorph, a hydrate or a solvate thereof, wherein:

the POI is

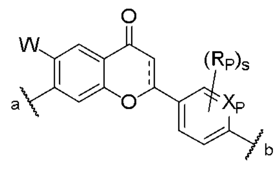;

$\overline{- - -}$ represents a single bond or a double bond, alternatively a double bond;
each $R_P$ is independently selected from H, D, halogen, $C_{1-4}$ alkyl and $C_{1-4}$ haloalkyl;
s is selected from 1, 2 and 3;
$X_P$ is selected from N and $CR_P$;
W is selected from H, D, halogen, $C_{1-4}$ alkyl, $C_{1-4}$ haloalkyl and

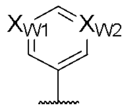;

$X_{W1}$ and $X_{W2}$ are independently selected from N and $CR_W$;
Rw is selected from H, D, $-C(O)R_a$, $-C(O)NR_bR_c$, $-C(O)OR_a$, $-OC(O)R_a$ and $-NR_bC(O)R_a$;
$R_a$, $R_b$ and $R_c$ are independently selected from H, $C_{1-4}$ alkyl and $C_{1-4}$ haloalkyl;
the a-terminal of the POI is connected to Q via $C_{0-3}$ alkylene, and the b-terminal is connected to L;
alternatively,
the POI is

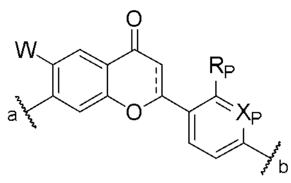;

$\overline{- - -}$ represents a single bond or a double bond, alternatively a double bond;
$R_P$ is halogen;
$X_P$ is selected from N and CH;
W is selected from H, halogen and

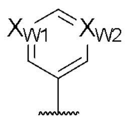;

$X_{W1}$ and $X_{W2}$ are independently selected from N and $CR_W$;
Rw is selected from H, $-C(O)R_a$ and $-C(O)NR_bR_c$;
$R_a$, $R_b$ and $R_c$ are independently selected from H, $C_{1-4}$ alkyl and $C_{1-4}$ haloalkyl;
the a-terminal of the POI is connected to Q via $C_{0-3}$ alkylene, and the b-terminal is connected to L;
still alternatively,
the POI is selected from

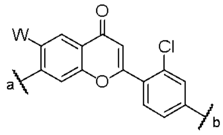, 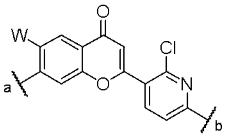, 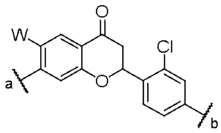

and

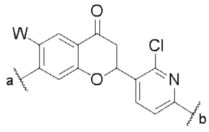;

W is selected from H, halogen,

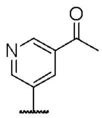, 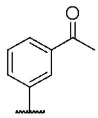 and 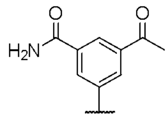,

alternatively W is selected from H, I and

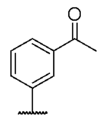;

the a-terminal of the POI is connected to Q via $C_{0-3}$ alkylene, and the b-terminal is connected to L.

In a more specific embodiment, the present disclosure provides a compound of formula (I) above, or a pharmaceutically acceptable salt, an isotope variant, a tautomer, a stereoisomer, a prodrug, a polymorph, a hydrate or a solvate thereof, wherein:

L is

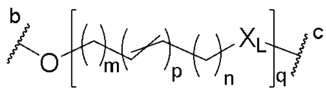;

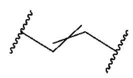

represents a single bond, a cis- or trans-double bond, alternatively a cis-double bond;
each $X_L$ is independently selected from a chemical bond, -O-, $C_{1-4}$ alkylene, phenylene and 5- to 6-membered heteroarylene; the $X_L$ is optionally deuterated, up to completely deuterated;
each m is independently selected from 0, 1, 2, 3, 4, 5, 6, 7, 8, 9 and 10;
each n is independently selected from 0, 1, 2, 3, 4, 5, 6, 7, 8, 9 and 10;
each p is independently selected from 0, 1, 2, 3, 4 and 5;
each q is independently selected from 1, 2, 3, 4 and 5;
the b-terminal of L is connected to POI, and the c-terminal is connected to E3, or the c-terminal of L is connected to POI, and the b-terminal is connected to E3;
alternatively,
L is

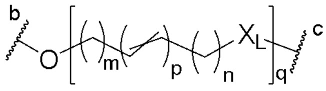;

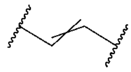

represents a single bond, a cis- or trans-double bond, alternatively a cis-double bond;

each $X_L$ is independently selected from a chemical bond, -O-, $-CH_2-$,

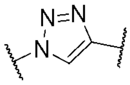 and 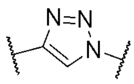,

alternatively each $X_L$ is independently selected from -O- and -CH2-;

each m is independently selected from 0, 1, 2, 3, 4, 5, 6, 7 and 8, alternatively each m is independently selected from 0, 1, 2, 3, 4, still alternatively each m is 1;

each n is independently selected from 0, 1, 2, 3, 4, 5, 6, 7 and 8, alternatively each n is independently selected from 0, 1, 2, 3, 4, still alternatively each n is independently selected from 1 and 2;

each p is independently selected from 0, 1, 2 and 3, alternatively each p is independently selected from 0 and 1;

each q is independently selected from 1, 2, 3, 4 and 5, alternatively each q is 3;

the b-terminal of L is connected to POI, and the c-terminal is connected to E3, or the c-terminal of L is connected to POI, and the b-terminal is connected to E3;

still alternatively,

L is selected from:

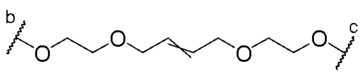 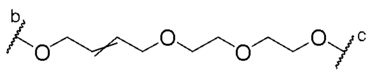 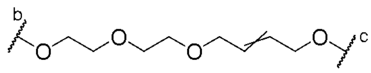

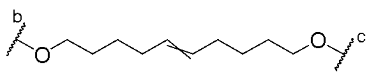 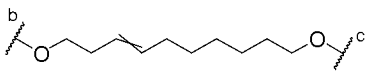 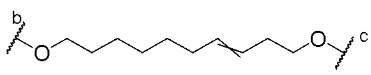

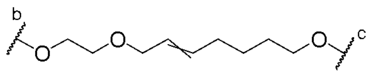 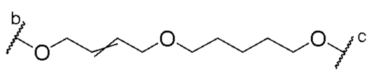 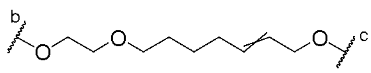

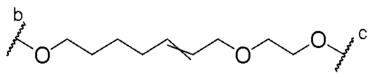 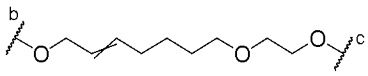 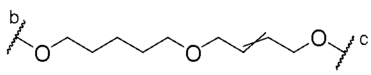

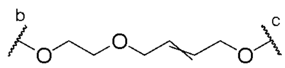 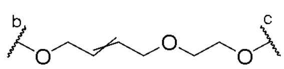 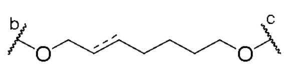 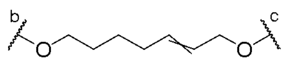

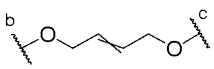 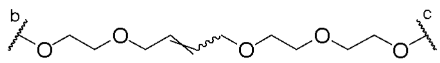 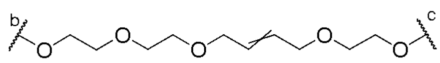

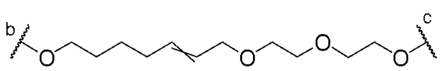 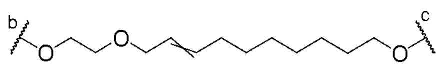

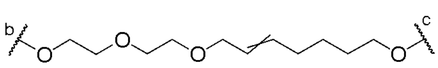 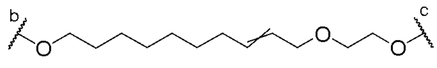

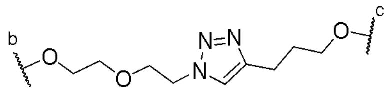 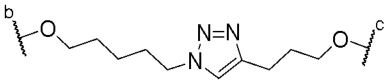 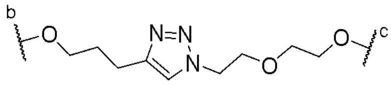

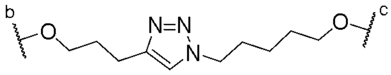 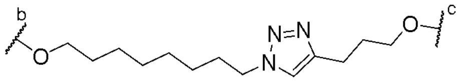

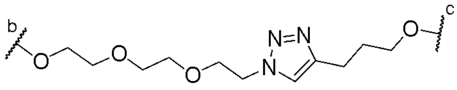 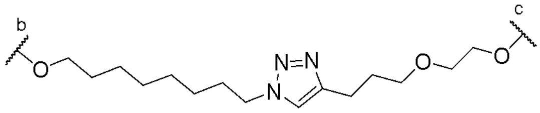

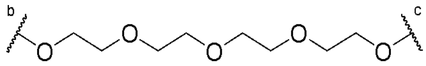;

alternatively, L is

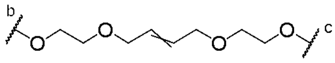,

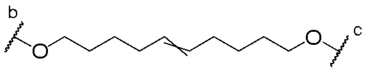, 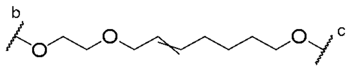, 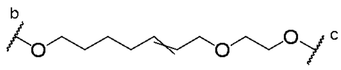

or

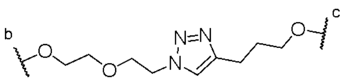;

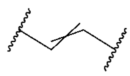

represents a single bond, a cis- or trans-double bond, alternatively a cis-double bond; the b-terminal of the L is connected to the POI, and the c-terminal is connected to E3, or the c-terminal of the L is connected to the POI, and the b-terminal is connected to E3.

In a more specific embodiment, the present disclosure provides a compound of formula (I) above, or a pharmaceutically acceptable salt, an isotope variant, a tautomer, a stereoisomer, a prodrug, a polymorph, a hydrate or a solvate thereof, wherein:

E3 is

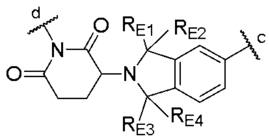;

$R_{E1}$, $R_{E2}$, $R_{E3}$ or $R_{E4}$ are independently selected from H and D, and at least one pair of $R_{E1}$ and $R_{E2}$, $R_{E3}$ and $R_{E4}$ represents =O;

the c-terminal of E3 is connected to L, and the d-terminal is connected to Q via $C_{0-3}$ alkylene;

alternatively,

E3 is selected from

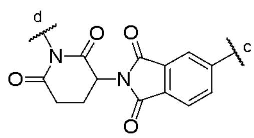 , 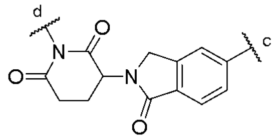

and

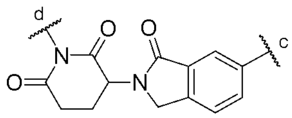 ;

the c-terminal of E3 is connected to L, and the d-terminal is connected to Q via $C_{0-3}$ alkylene.

In a more specific embodiment, the present disclosure provides a compound of formula (I) above, or a pharmaceutically acceptable salt, an isotope variant, a tautomer, a stereoisomer, a prodrug, a polymorph, a hydrate or a solvate thereof, wherein:

Q is

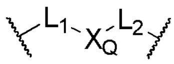 ;

$L_1$ and $L_2$ are independently selected from NH and O;
$X_Q$ is selected from -P(O)($Y_Q$-$R_Q$)-, -S(O)- and -C(O)-$X_{Q1}$-C(O)-;
$Y_Q$ is selected from NH and O;
$X_{Q1}$ is selected from a chemical bond, $C_{1-6}$ alkylene, $C_{2-6}$ alkenylene, $C_{2-6}$ alkynylene, -$C_{1-6}$ alkylene-$NR_b$-$C_{1-6}$ alkylene, 5- to 10-membered heterocyclylene and $C_{6-10}$ arylene, and the $X_{Q1}$ is optionally substituted with 1, 2, 3, 4 or 5 independently selected R#;
$R_b$ is selected from H, $C_{1-4}$ alkyl and $C_{1-4}$ haloalkyl;
$R_Q$ is selected from H, $C_{1-6}$ alkyl, $C_{2-6}$ alkenyl, $C_{2-6}$ alkynyl and $C_{6-10}$ aryl;
R# is selected from H, halogen, OH, $NH_2$, $C_{1-4}$ alkyl, $C_{2-4}$ alkenyl and $C_{2-4}$ alkynyl;
alternatively,
Q is

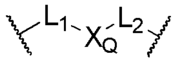 ;

$L_1$ and $L_2$ are independently selected from NH and O;
$X_Q$ is selected from -P(O)($Y_Q$-$R_Q$)-, -S(O)- and -C(O)-$X_{Q1}$-C(O)-;
$Y_Q$ is selected from NH and O;
$X_{Q1}$ is selected from a chemical bond, $C_{1-4}$ alkylene, -$C_{1-4}$ alkylene-$NR_b$-$C_{1-4}$ alkylene, 5- to 6-membered heterocyclylene (e.g.,

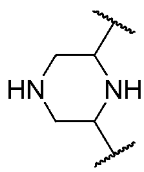

) and phenylene, wherein the $C_{1-4}$ alkylene is optionally substituted with 1, 2 or 3 independently selected R#;
$R_b$ is selected from H, $C_{1-4}$ alkyl and $C_{1-4}$ haloalkyl;
$R_Q$ is selected from H, $C_{1-4}$ alkyl and phenyl;
R# is selected from H and $NH_2$;
still alternatively,
Q is selected from

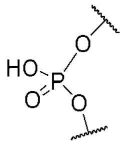, 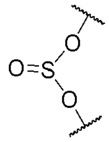, 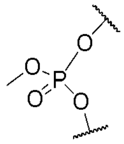, 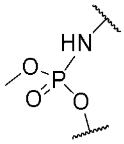, 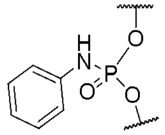, 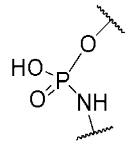,

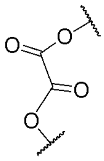, 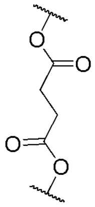, 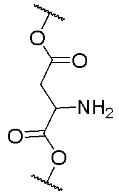, 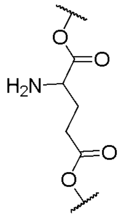, 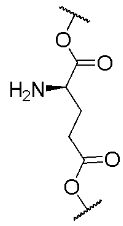, 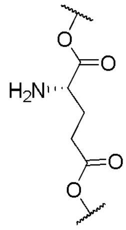, 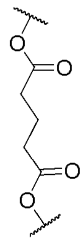, 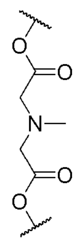, 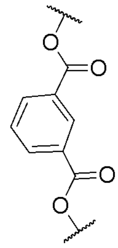,

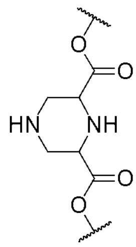 and 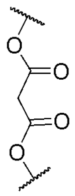 ;

alternatively, Q is selected from

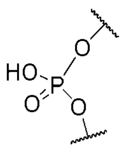, 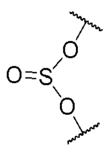, 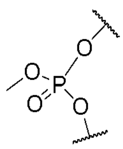, 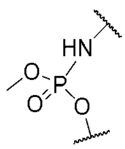,

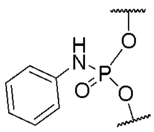, 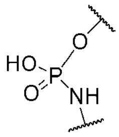, 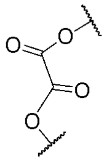, 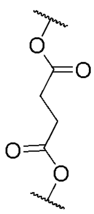, 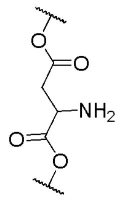, 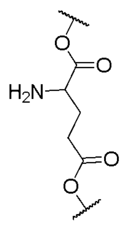, 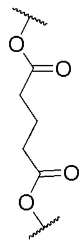, 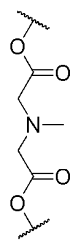, 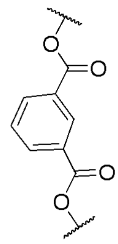,

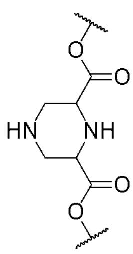 and 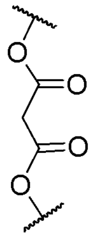 ;

and still alternatively, Q is selected from

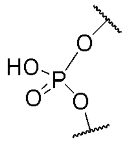, 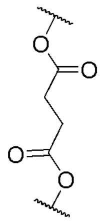, 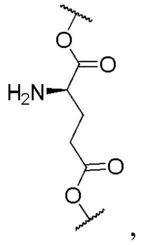,

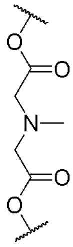, 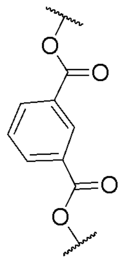 and 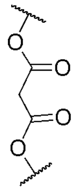.

In a more specific embodiment, the present disclosure provides a compound of formula (I) above, or a pharmaceutically acceptable salt, an isotope variant, a tautomer, a stereoisomer, a prodrug, a polymorph, a hydrate or a solvate thereof, wherein:

the POI is

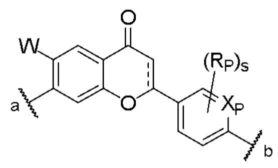;

- - - represents a single bond or a double bond, alternatively a double bond;
each $R_P$ is independently selected from H, D, halogen, $C_{1-4}$ alkyl and $C_{1-4}$ haloalkyl;
s is selected from 1, 2 and 3;
$X_P$ is selected from N and $CR_P$;
W is selected from H, D, halogen, $C_{1-4}$ alkyl, $C_{1-4}$ haloalkyl and

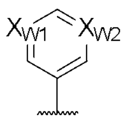;

$X_{W1}$ and $X_{W2}$ are independently selected from N and $CR_W$;
Rw is selected from H, D, $-C(O)R_a$, $-C(O)NR_bR_c$, $-C(O)OR_a$, $-OC(O)R_a$ and $-NR_bC(O)R_a$;
the a-terminal of the POI is connected to Q via $C_{0-3}$ alkylene, and the b-terminal is connected to L;
L is

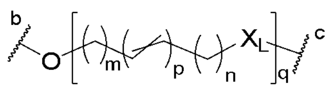;

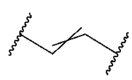

represents a single bond, a cis- or trans-double bond, alternatively a cis-double bond;
each $X_L$ is independently selected from a chemical bond, -O-, $C_{1-4}$ alkylene, phenylene and 5- to 6-membered heteroarylene; the $X_L$ is optionally deuterated, up to completely deuterated;

each m is independently selected from 0, 1, 2, 3, 4, 5, 6, 7, 8, 9 and 10;
each n is independently selected from 0, 1, 2, 3, 4, 5, 6, 7, 8, 9 and 10;
each p is independently selected from 0, 1, 2, 3, 4 and 5;
each q is independently selected from 1, 2, 3, 4 and 5;
the b-terminal of L is connected to POI, and the c-terminal is connected to E3, or the c-terminal of L is connected to POI, and the b-terminal is connected to E3;
E3 is

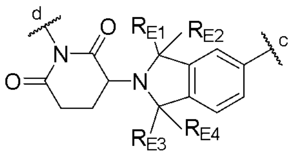
;

$R_{E1}$, $R_{E2}$, $R_{E3}$ or $R_{E4}$ are independently selected from H and D, and at least one pair of $R_{E1}$ and $R_{E2}$, $R_{E3}$ and $R_{E4}$ represents =O;
the c-terminal of E3 is connected to L, and the d-terminal is connected to Q via $C_{0-3}$ alkylene;
Q is

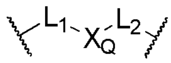
;

$L_1$ and $L_2$ are independently selected from NH and O;
$X_Q$ is selected from -P(O)($Y_Q$-$R_Q$)-, -S(O)- and -C(O)-$X_{Q1}$-C(O)-;
$Y_Q$ is selected from NH and O;
$X_{Q1}$ is selected from a chemical bond, $C_{1-6}$ alkylene, $C_{2-6}$ alkenylene, $C_{2-6}$ alkynylene, -$C_{1-6}$ alkylene-$NR_b$-$C_{1-6}$ alkylene, 5- to 10-membered heterocyclylene and $C_{6-10}$ arylene, and the $X_{Q1}$ is optionally substituted with 1, 2, 3, 4 or 5 independently selected R#;
$R_Q$ is selected from H, $C_{1-6}$ alkyl, $C_{2-6}$ alkenyl, $C_{2-6}$ alkynyl and $C_{6-10}$ aryl;
$R_a$, $R_b$ and $R_c$ are independently selected from H, $C_{1-4}$ alkyl and $C_{1-4}$ haloalkyl;
R# is selected from H, halogen, OH, $NH_2$, $C_{1-4}$ alkyl, $C_{2-4}$ alkenyl and $C_{2-4}$ alkynyl.

In a more specific embodiment, the present disclosure provides a compound of formula (I) above, or a pharmaceutically acceptable salt, an isotope variant, a tautomer, a stereoisomer, a prodrug, a polymorph, a hydrate or a solvate thereof, wherein:

the POI is

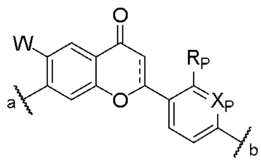
;

- - - represents a single bond or a double bond, alternatively a double bond;
$R_P$ is halogen;
$X_P$ is selected from N and CH;
W is selected from H, halogen and

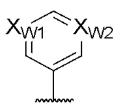
;

$X_{W1}$ and $X_{W2}$ are independently selected from N and $CR_W$;
Rw is selected from H, -C(O)$R_a$ and -C(O)$NR_bR_c$;
the a-terminal of the POI is connected to Q via $C_{0-3}$ alkylene, and the b-terminal is connected to L;

L is

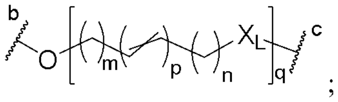

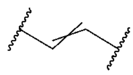

represents a single bond, a cis- or trans-double bond, alternatively a cis-double bond;
each $X_L$ is independently selected from a chemical bond, -O-, $-CH_2-$,

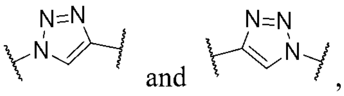

alternatively each $X_L$ is independently selected from -O- and $-CH_2-$;
each m is independently selected from 0, 1, 2, 3, 4, 5, 6, 7 and 8, alternatively independently selected from 0, 1, 2, 3, 4, and still alternatively independently is 1;
each n is independently selected from 0, 1, 2, 3, 4, 5, 6, 7 and 8, alternatively independently selected from 0, 1, 2, 3, 4, and still alternatively independently selected from 1 and 2;
each p is independently selected from 0, 1, 2 and 3, alternatively independently selected from 0 and 1;
each q is independently selected from 1, 2, 3, 4 and 5, alternatively independently is 3;
the b-terminal of L is connected to POI, and the c-terminal is connected to E3, or the c-terminal of L is connected to POI, and the b-terminal is connected to E3;
E3 is

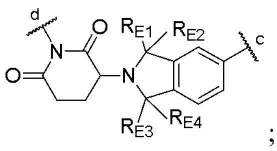

$R_{E1}$, $R_{E2}$, $R_{E3}$ or $R_{E4}$ are independently selected from H and D, and at least one pair of $R_{E1}$ and $R_{E2}$, $R_{E3}$ and $R_{E4}$ represents =O;
the c-terminal of E3 is connected to L, and the d-terminal is connected to Q via $C_{0-3}$ alkylene;
Q is

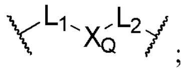

$L_1$ and $L_2$ are independently selected from NH and O;
$X_Q$ is selected from $-P(O)(Y_Q-R_Q)-$, -S(O)- and $-C(O)-X_{Q1}-C(O)-$;
$Y_Q$ is selected from NH and O;
$X_{Q1}$ is selected from a chemical bond, $C_{1-4}$ alkylene, $-C_{1-4}$ alkylene-$NR_b$-$C_{1-4}$ alkylene, 5- to 6-membered heterocyclylene (e.g.,

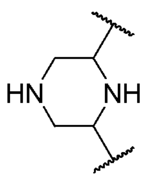

) and phenylene, wherein the $C_{1-4}$ alkylene is optionally substituted with 1, 2 or 3 independently selected R#;
$R_Q$ is selected from H, $C_{1-4}$ alkyl and phenyl;
$R_a$, $R_b$ and $R_c$ are independently selected from H, $C_{1-4}$ alkyl and $C_{1-4}$ haloalkyl;

R# is selected from H and $NH_2$.

In a more specific embodiment, the present disclosure provides a compound of formula (I) above, or a pharmaceutically acceptable salt, an isotope variant, a tautomer, a stereoisomer, a prodrug, a polymorph, a hydrate or a solvate thereof, wherein:

the POI is selected from

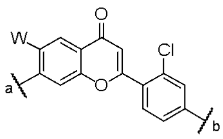,
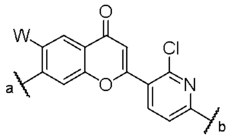,
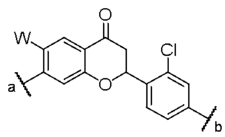

and

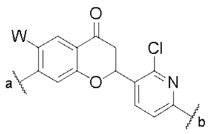,

alternatively the POI is selected from

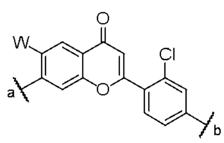 and 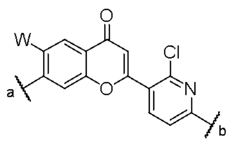;

W is selected from H, halogen,

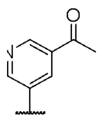, 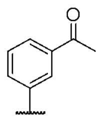 and 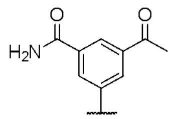,

alternatively W is selected from H, I and

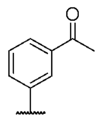;

the a-terminal of the POI is connected to Q via $C_{0-3}$ alkylene, and the b-terminal is connected to L;
L is selected from:

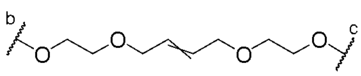
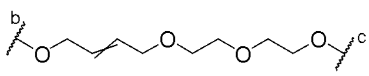
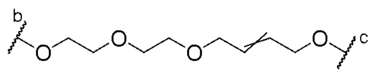
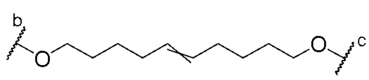
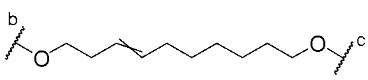
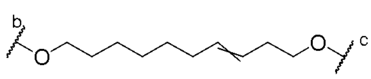
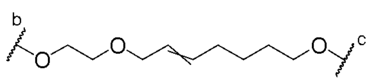
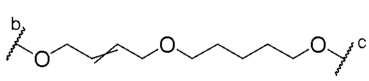
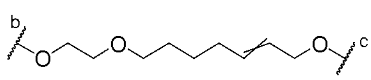

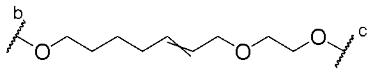 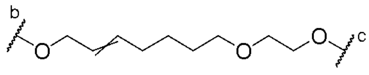 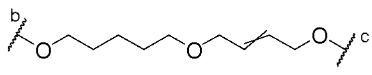

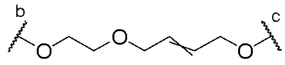 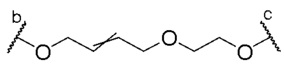 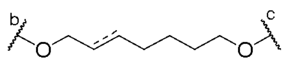 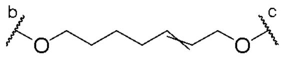

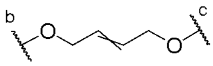 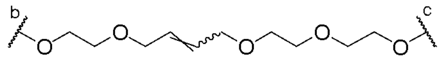 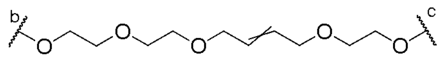

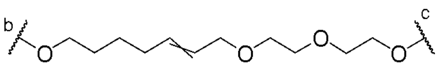 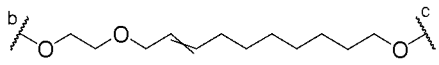

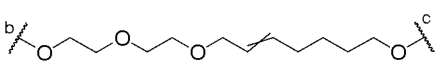 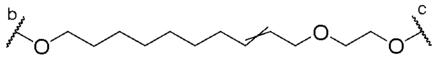

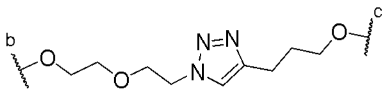 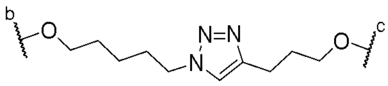 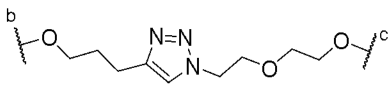

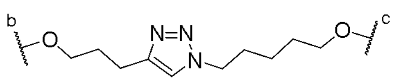 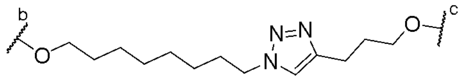

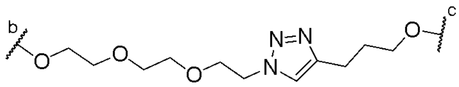 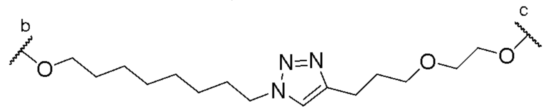

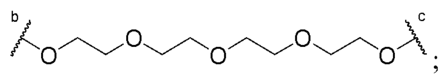;

alternatively, L is

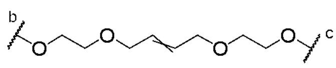, 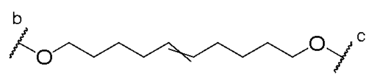,

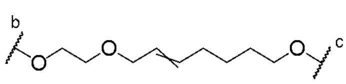, 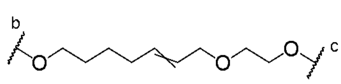 or 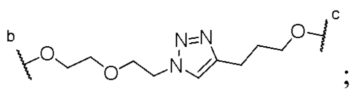;

the b-terminal of L is connected to POI, and the c-terminal is connected to E3, or the c-terminal of L is connected to POI, and the b-terminal is connected to E3;

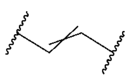

represents a single bond, a cis- or trans-double bond, alternatively a cis-double bond;
E3 is selected from

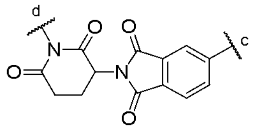, 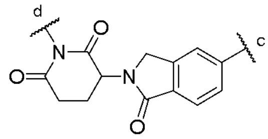

and

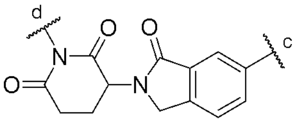;

the c-terminal of E3 is connected to L, and the d-terminal is connected to Q via $C_{0-3}$ alkylene; Q is selected from

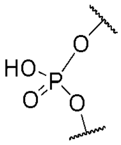, 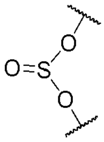, 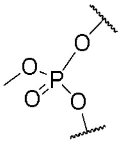, 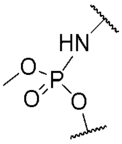, 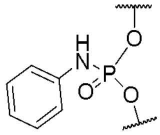, 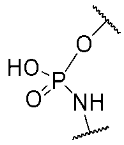,

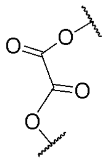, 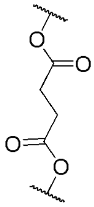, 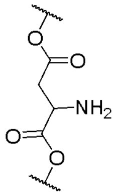, 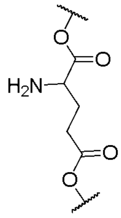, 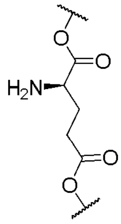, 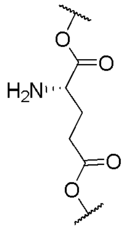, 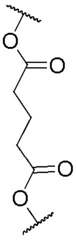, 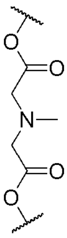, 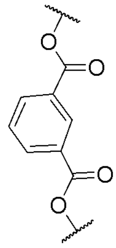,

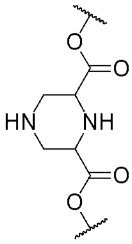 and 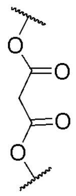;

alternatively, Q is selected from

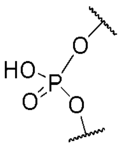, 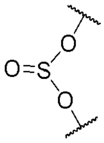, 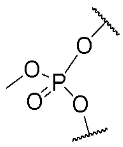, 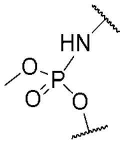,

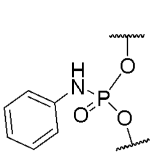, 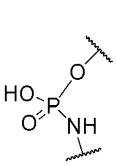, 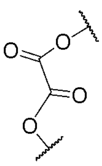, 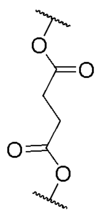, 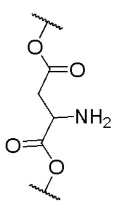, 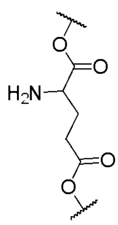, 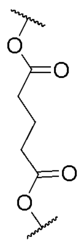, 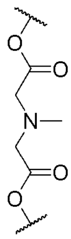, 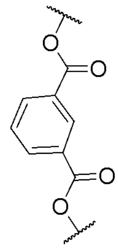,

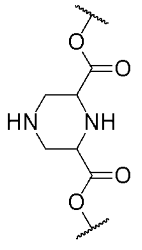 and 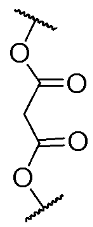 ;

and still alternatively, Q is

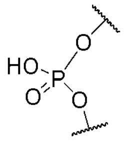 , 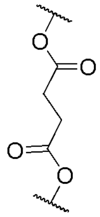 , 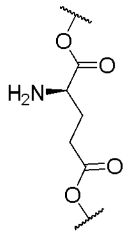 , 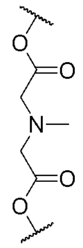 , 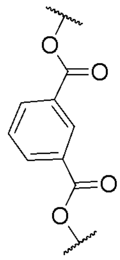

or

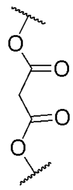 .

In a more specific embodiment, the present disclosure provides a compound of formula (I) above, or a pharmaceutically acceptable salt, an isotope variant, a tautomer, a stereoisomer, a prodrug, a polymorph, a hydrate or a solvate thereof, wherein the compound is selected from:

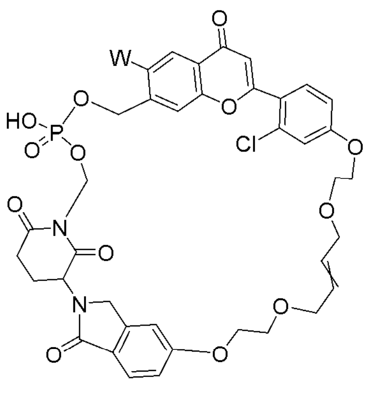 1, 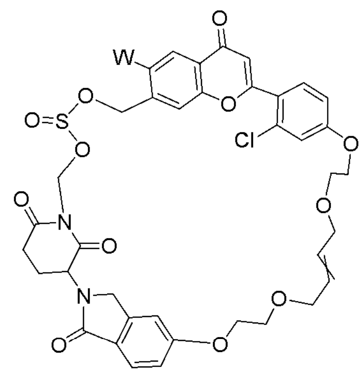 2, 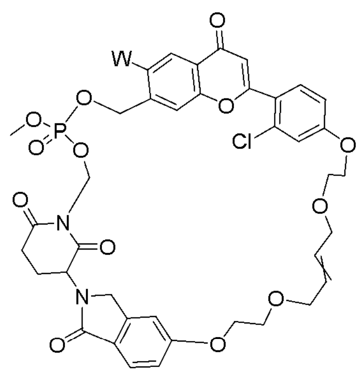 3,

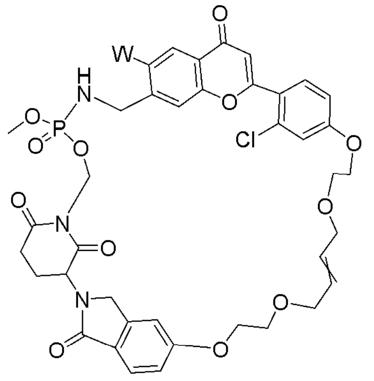

,

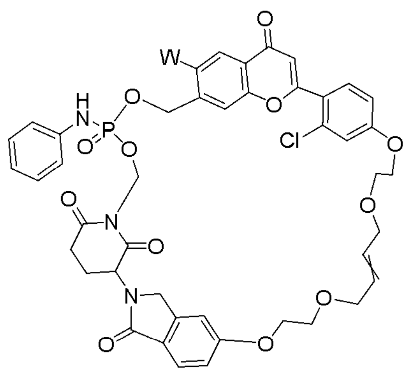

,

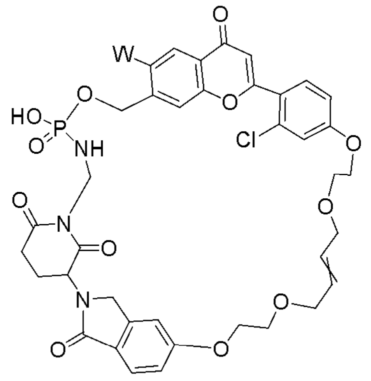

,

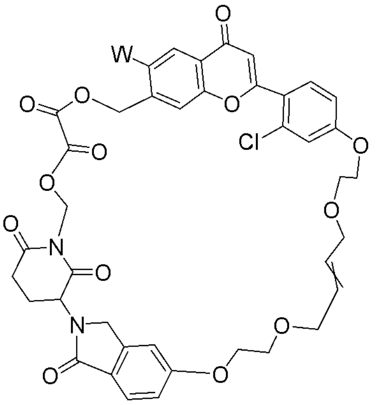

4,

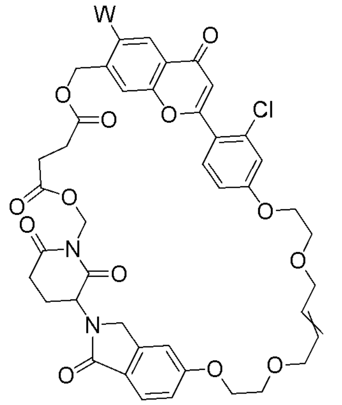

5,

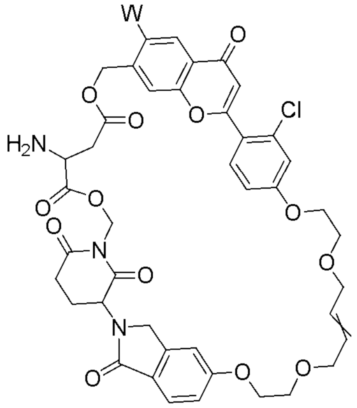

6,

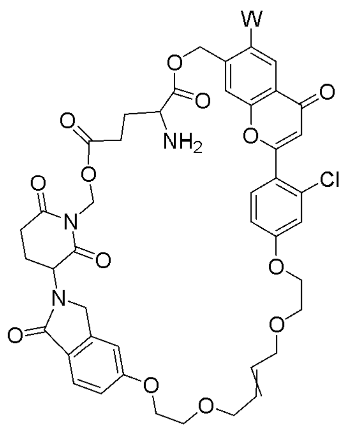

7,

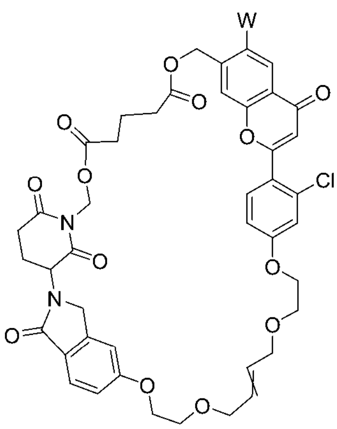

8,

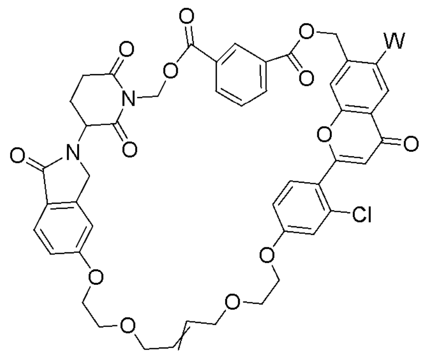

9,

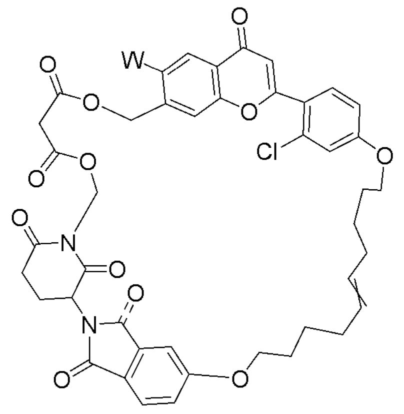 10, 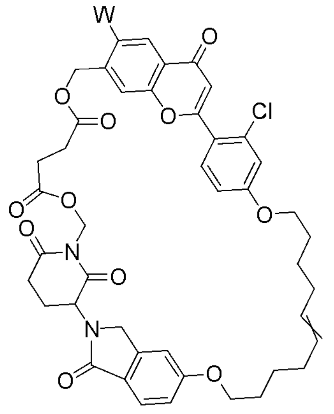 13, 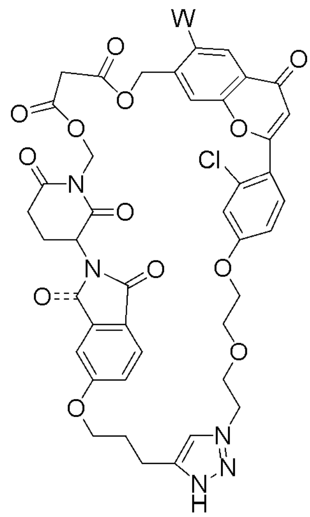 11,

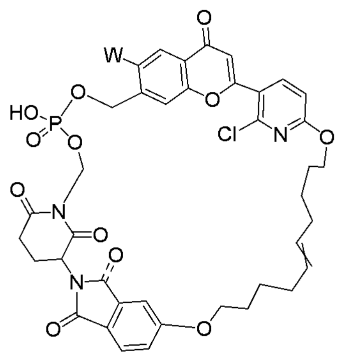 12 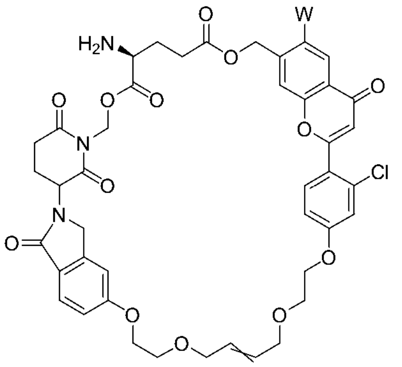 or 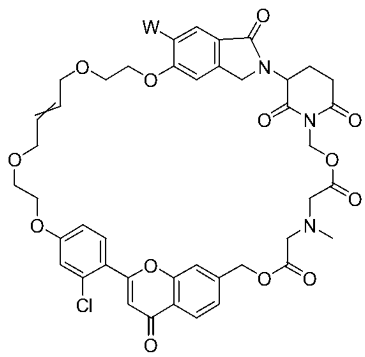 ,

wherein,

- - - represents a single bond or a double bond;

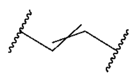

represents a single bond, a cis- or trans-double bond, alternatively a cis-double bond;

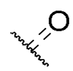

represents absent or

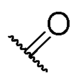 ;

W is selected from H, I,

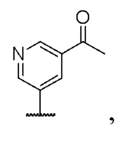 , 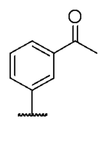 and 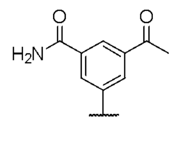 ,

alternatively W is selected from H, I and

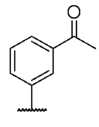
;

alternatively, the compound is selected from:

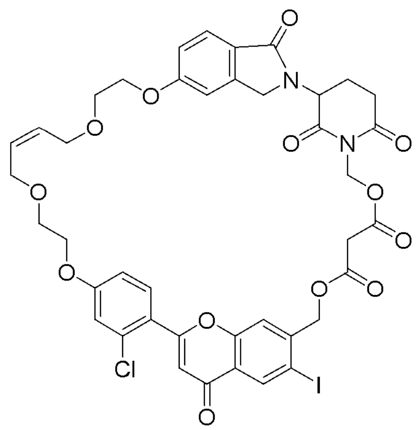
,
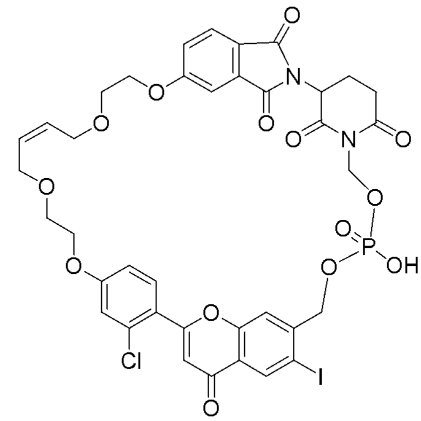
,
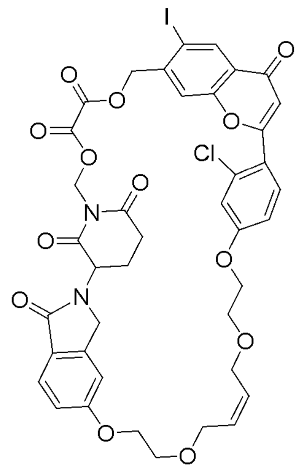
,
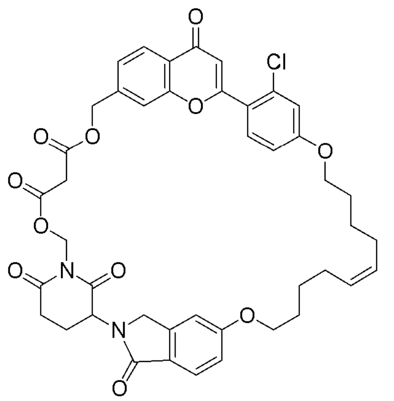
,
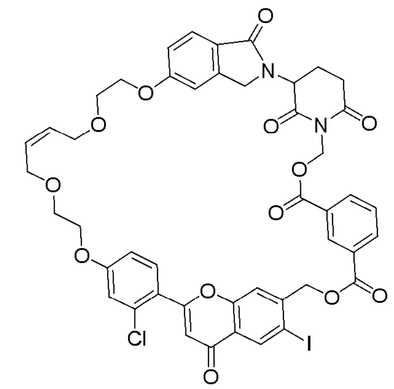
,
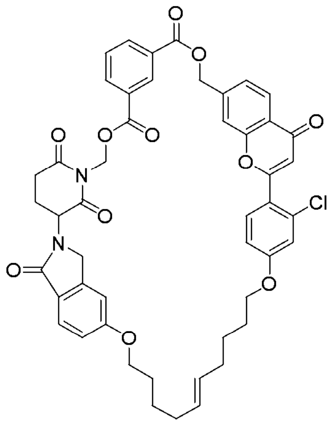
,
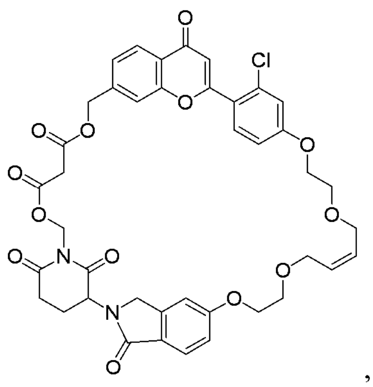
,
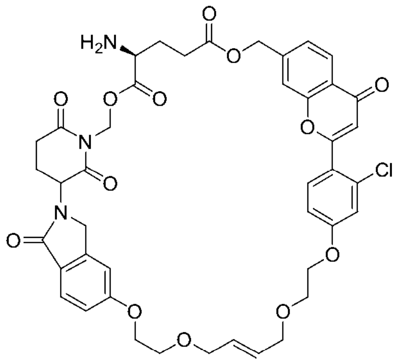
,
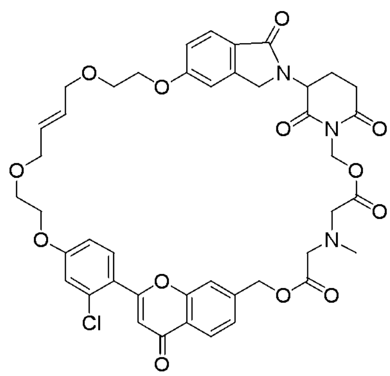

and

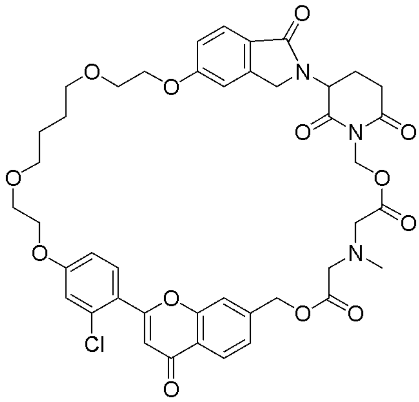

.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

The compounds of the present disclosure may also exist as tautomers. In compounds existing in different tautomeric forms, a compound is not limited to any particular tautomer, but is intended to cover all tautomeric forms. Tautomers are functional group isomers produced by the rapid movement of an atom in a molecule between two positions. The tautomers are special functional group isomers. A pair of tautomers can be converted into each other, but usually a relatively stable isomer is the main form of existence. The most important examples are the enol and keto tautomers.

It will be understood by those skilled in the art that the organic compounds can form complexes with solvents in which they are reacted or from which they are precipitated or crystallized. These complexes are known as "solvates." When the solvent is water, the complex is known as "hydrate." The present disclosure encompasses all solvates of the compounds of the present disclosure.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula $R \cdot x\ H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates ($R \cdot 0.5\ H_2O$)) and polyhydrates (x is a number greater than 1, for example, dihydrates ($R \cdot 2\ H_2O$) and hexahydrates ($R \cdot 6\ H_2O$)).

Compounds of the present disclosure may be in an amorphous or a crystalline form (polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes (isotope variants), which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and

chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., $^{3}H$ and $^{14}C$), can be used for the measurement of the distribution of drug and/or substrate in tissue. Tritium, which is $^{3}H$ and carbon-14, which is $^{14}C$ isotope, are yet alternative, because they are easy to prepare and detect. Furthermore, replaced by heavier isotopes, such as deuterium, which is $^{2}H$, may provide therapeutic benefits due to the higher metabolic stability, such as prolonging the half-life in vivo or decreasing the dosage requirements, and thus may be alternative in some cases. Isotope-labeled compounds of formula (A) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects in vivo by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which is incorporated herein by reference.

Pharmaceutical compositions and kits

In another aspect, the present disclosure provides a pharmaceutical composition comprising a compound of the present disclosure (also referred to as the "active ingredient") and a pharmaceutically acceptable excipient. In certain embodiments, the pharmaceutical composition comprises an effective amount of the compound of the present disclosure. In certain embodiments, the pharmaceutical composition comprises a therapeutically effective amount of the compound of the present disclosure. In certain embodiments, the pharmaceutical composition comprises a prophylactically effective amount of the compound of the present disclosure.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other suitable containers) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

Administration

The pharmaceutical composition provided by the present disclosure can be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a

subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, for example, over 3 months, 6 months, 1 year, 2 years, 3 years, 5 years, etc, or may be continued indefinitely, for example, for the rest of the subject's life. In certain embodiments, the chronic administration is intended to provide a constant level of the compound in the blood, e.g., within the therapeutic window over the extended period of time.

The pharmaceutical compostions of the present disclosure may be further delivered using a variety of dosing methods. For example, in certain embodiments, the pharmaceutical composition may be given as a bolus, e.g., in order to raise the concentration of the compound in the blood to an effective level. The placement of the bolus dose depends on the systemic levels of the active ingredient desired throughout the body, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with alternative doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and still alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as a ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are

merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

The compounds of the present disclosure can also be administered in sustained release forms or from sustained release drug delivery systems. A description of representative sustained release materials can be found in Remington's Pharmaceutical Sciences.

The present disclosure also relates to the pharmaceutically acceptable formulations of a compound of the present disclosure. In one embodiment, the formulation comprises water. In another embodiment, the formulation comprises a cyclodextrin derivative. The most common cyclodextrins are α-, β- and γ-cyclodextrins consisting of 6, 7 and 8 α-1,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, for example, sulfobutyl ether β-cyclodextrin, also known as Captisol. See, e.g., U.S. 5,376,645. In certain embodiments, the formulation comprises hexapropyl-β-cyclodextrin (e.g., 10-50% in water).

## Examples

The following examples are intended to illustrate the present disclosure but are not intended to limit the scope of the present disclosure.

Abbreviation

| | |
|---|---|
| ACN | Acetonitrile |
| eq | Equivalent |
| DCM | Dichloromethane |
| DCE | Ethylene dichloride |
| DBU | 1,8-Diazabicyclo[5.4.0]undec-7-ene |
| DEAD | Diethyl azodicarboxylate |
| DIAD | Diisopropyl azodicarboxylate |
| DMF | N,N-Dimethylformamide |
| EA | Ethyl acetate |
| FA | Formic acid |
| MsCl | Methylsufonyl chloride |
| SEMCl | 2-(Trimethylsilyl)ethoxymethyl chloride |
| TFA | Trifluoroacetic acid |
| THF | Tetrahydrofuran |
| TEA | Triethylamine |

Example 1

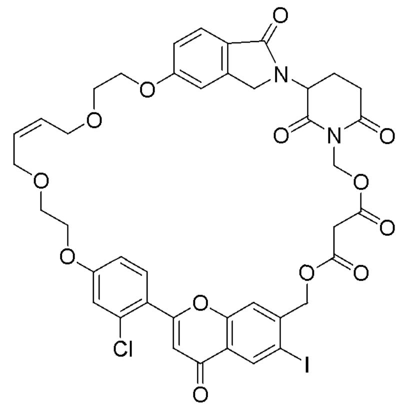

Synthetic route

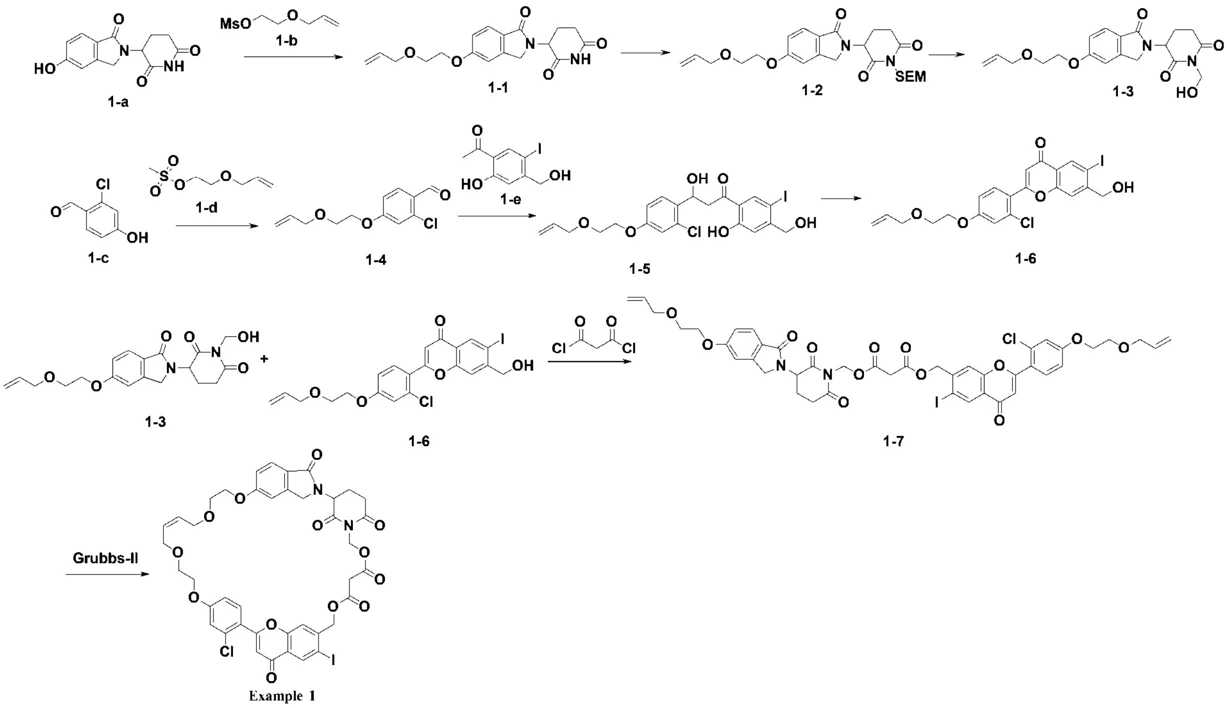


Example 1

Synthesis of 1-1

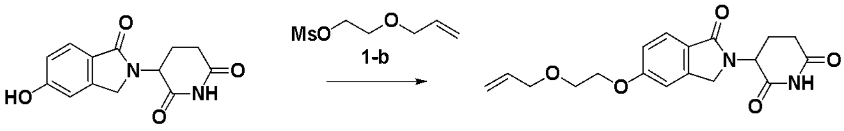


1-a 1-1

1-a (10 g, 1.2 eq) was added to 100 mL DMF, and then 1-b (6.87 g, 1 eq), KI (7.5 g, 1.2 eq) and $NaHCO_3$ (6.5 g, 2 eq) were added sequentially under nitrogen protection. The mixture was stirred at room temperature for 30 min, heated to 70 °C, and stirred overnight. LCMS monitoring showed that no raw material remained. The reaction was quenched with saturated sodium chloride aqueous solution. The mixture was washed with saturated brine, extracted with ethyl acetate, dried over anhydrous sodium sulfate, and concentrated. The residue was purified by reverse column (0.1% FA in

water/ACN =50%) to obtain 3.2 g of product 1-1. MS: =344.4.

Synthesis of 1-2

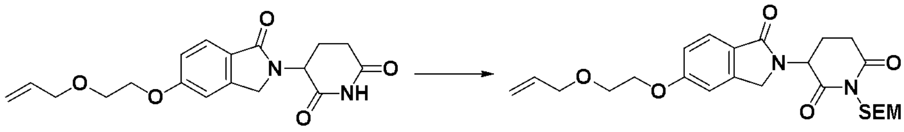

1-1 1-2

1-1 (3.0 g, 1.0 eq) was added to 30 mL DCM. SEMCl (2.1 g, 1.1 eq) was added, and then DBU (2.2 g, 1.2eq) was added at 0 °C. The mixture was stirred at room temperature for 16 h. LCMS monitoring showed that no raw material remained. The reaction was quenched with saturated sodium chloride aqueous solution. The mixture was extracted with ethyl acetate, dried over anhydrous sodium sulfate, and concentrated. The residue was purified by reverse column (0.1% FA in water/ACN =50 %) to obtain 3.4 g of product 1-2, MS: =474.63

Synthesis of 1-3

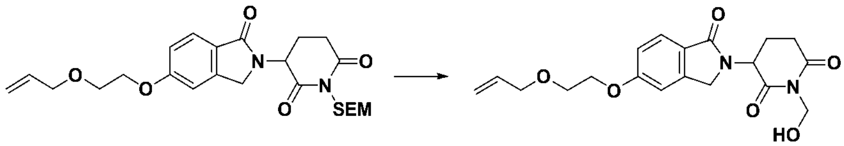

1-2 1-3

1-2 (3.0 g, 1.0 eq) was added to 30 mL DCM, and then TFA (6 mL) was added. The mixture was stirred at room temperature for 3 h. LCMS monitoring showed that no raw material remained. The reaction was concentrated directly. The residue was purified by reverse column (0.1% FA in water/ACN =50 %) to obtain 2 g of product 1-3, MS: =374.4.

Synthesis of 1-4

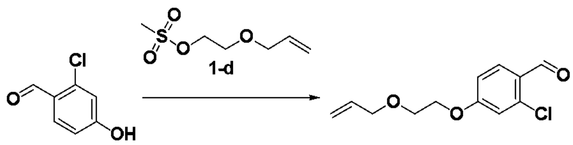


1-c 1-4

1-c (20 g, 1.0 eq) was added to 30 mL ACN, and then 1-d (27 g, 1.2 eq) and K2CO3 (21.2 g, 1.2 eq) were added. The mixture was stirred at 70 °C for 6 h. LCMS monitoring showed that no raw material remained. The reaction was quenched with saturated sodium chloride aqueous solution. The mixture was extracted with ethyl acetate, dried over anhydrous sodium sulfate, and concentrated. The residue was purified by normal column to obtain 20 g of product 1-4, MS: =240.7.

Synthesis of 1-5

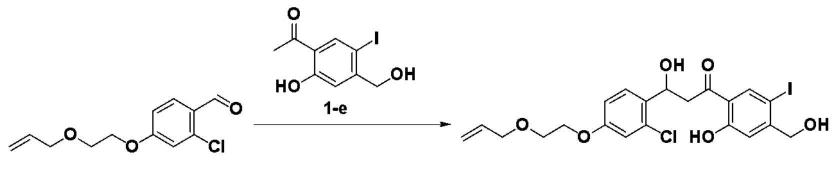


1-4 1-5

1-4 (3 g, 2.0 eq) and 1-e (1.1 g, 1 eq) were added to 30 mL THF, and potassium tert-butoxide (1.35 g, 2 eq) was added. The mixture was stirred at 0 °C for 6 h. LCMS monitoring showed that no raw material remained. The reaction was quenched with saturated sodium chloride aqueous solution and adjusted to pH~5 with hydrochloric acid. The mixture was extracted with ethyl acetate, dried over anhydrous sodium sulfate, and concentrated. The residue was purified by normal column to obtain 1.4 g of product 1-5, MS: 532.8.

Synthesis of 1-6

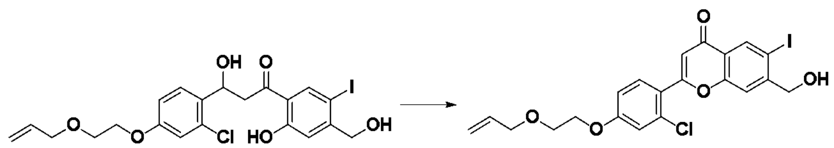

1-5 1-6

1-5 (1.5 g, 1.0 eq) was added to 15 mL DMSO, and a catalytic amount of iodine was added. The mixture was stirred at 110 °C for 6 h. LCMS monitoring showed that no raw material remained. The reaction was quenched with saturated sodium chloride aqueous solution and then sodium thiosulfate was added. The mixture was extracted with ethyl acetate, dried over anhydrous sodium sulfate, and concentrated. The residue was purified by normal column to obtain 900 mg of product 1-6, MS: 532.7.

Synthesis of 1-7

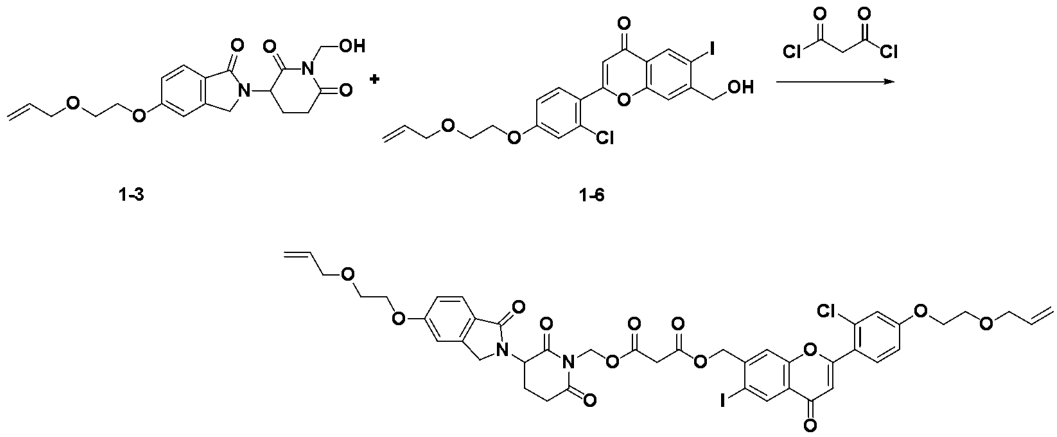

1-3 + 1-6

1-7

The starting compounds 1-3 (400 mg, 1.0 eq) and 1-6 (548 mg, 1.0 eq) were added to 20 mL DCM. Triethylamine (324 mg, 3 eq) was then added under ice bath conditions. The reaction was stirred for 5 minutes, and then malonyl chloride (226 mg, 1.5 eq) was slowly added to the above system. The reaction was stirred for another 0.5 h under ice bath conditions. LC-MS monitoring showed that no raw material remained. The residue was purified directly by prep-TLC (DCM:EA = 25:75) to obtain 200 mg of product 1-7 (yield: 20%), MS: 955.

Synthesis of Example 1

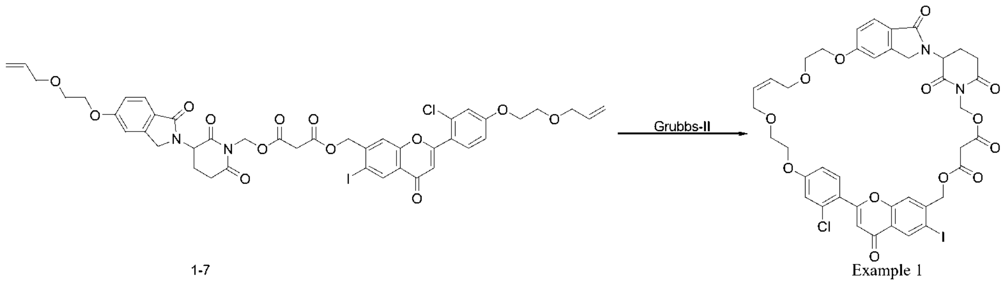


1-7

Example 1

Compound 1-1 (400 mg, 1.0 eq) was added to 50 mL DCE, and then Grubbs (II) (107 mg, 0.3 eq) was added at room temperature. The mixture was heated to 80 °C, and then the reaction was stirred for 1 h. LC-MS monitoring showed that no raw material remained. The residue was purified directly by prep-TLC ($H_2O$: $CH_3CN$ = 20: 80) to obtain 114 mg of Example 1 (yield: 29%).

$^1H$ NMR (400 MHz, DMSO-$d_6$) $\delta$ 8.44 (s, 3H), 7.77 (s, 2H), 7.74 (s, 1H), 7.26 (s, 1H), 6.71 - 6.60 (m, 4H), 5.82 (m, 1H), 5.32 (s, 2H), 5.21 (d, 1H), 4.35 (s, 1H), 4.02 (s, 1H), 3.78 (d, 5H), 2.06 - 1.95 (m, 13H).

MS: 927.1.

Example 2

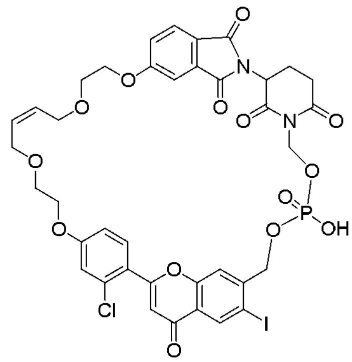

Synthetic route

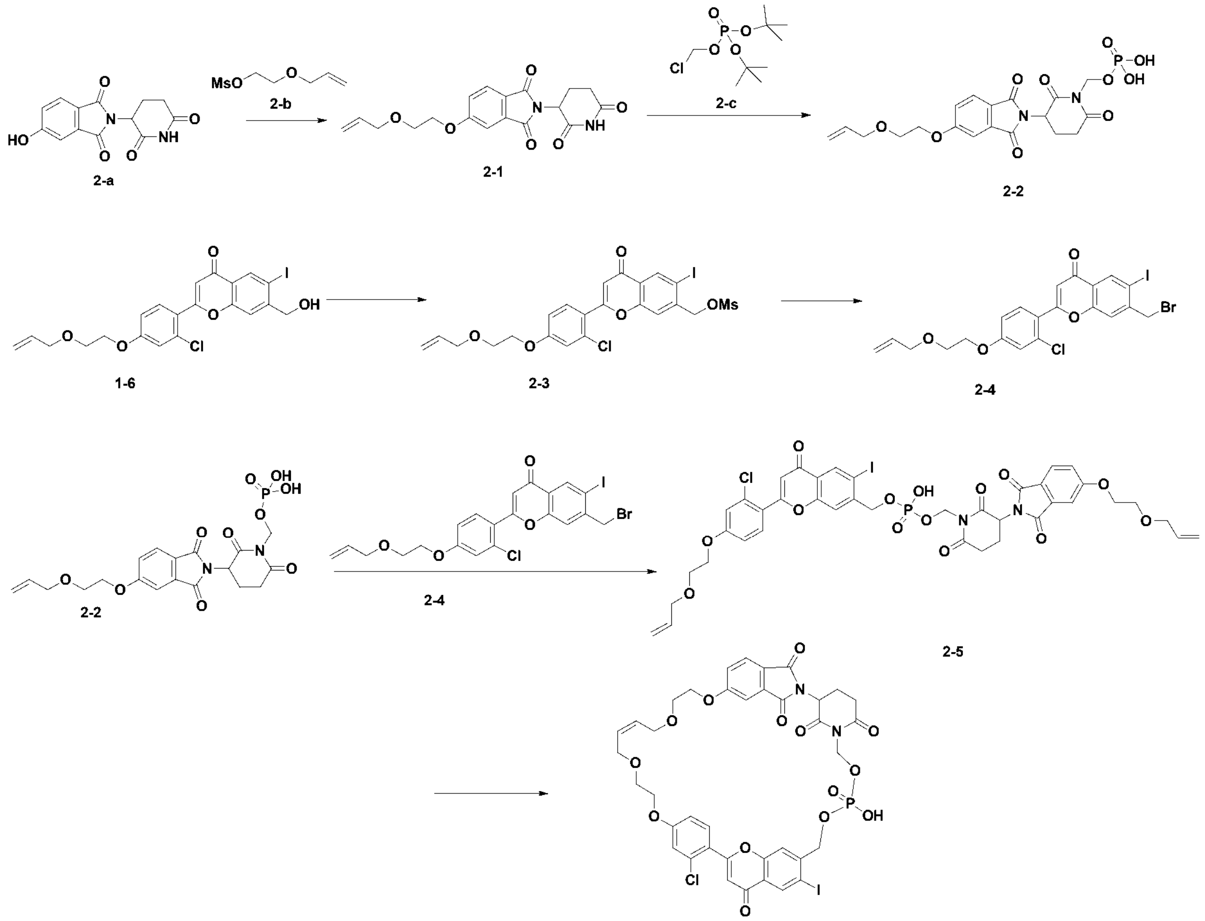


2-a 2-1 2-2 1-6 2-3 2-4 2-2 2-5

Example 2

Synthesis of 2-1

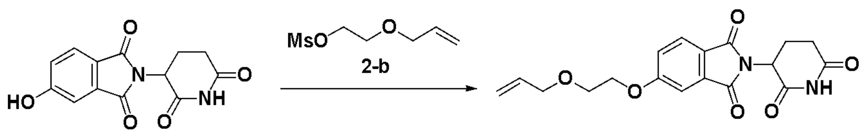


2-a 2-1

2-a (10 g, 1.2 eq) was added to 100 mL of DMF. 2-b (15 g, 1 eq), KI (14 g, 1.2 eq) and $NaHCO_3$ (9.4 g, 2 eq) were added sequentially under nitrogen protection. The reaction was stirred at room temperature for 30 min, heated to 90 °C, and stirred overnight. LCMS monitoring showed that no raw material remained. The reaction was quenched with saturated sodium chloride aqueous solution. The mixture was washed with saturated brine, extracted with ethyl acetate, dried over anhydrous sodium sulfate, and concentrated. The residue was purified by reverse column (0.1% FA in water/ACN =50 %) to obtain 10 g of product 2-1 (yield: 51.2%).

MS:359.

Synthesis of 2-2

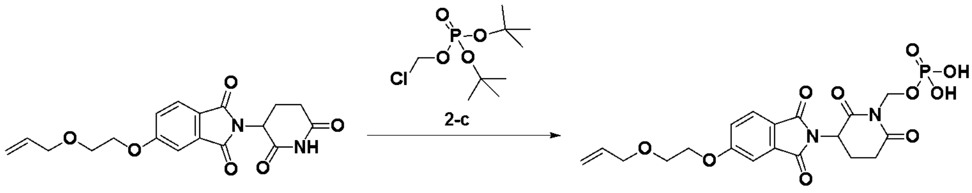


2-1

2-2

2-1 (9.0 g, 1.0 eq) was added to 100 mL DMF, and then 2-c (7.78 g, 1.1 eq) was added. DBU (4.59 g, 1.2 eq) was added at 0 °C. The reaction was stirred at room temperature for 16 hours. LCMS monitoring showed that no raw material remained. The reaction was quenched with saturated sodium chloride aqueous solution. The mixture was extracted with ethyl acetate, dried over anhydrous sodium sulfate, and concentrated. The residue was purified by reverse column (0.1% FA in water/ACN =50 %) to obtain 4 g of product 2-2 (yield: 23.7%), MS: 469.

Synthesis of 2-3

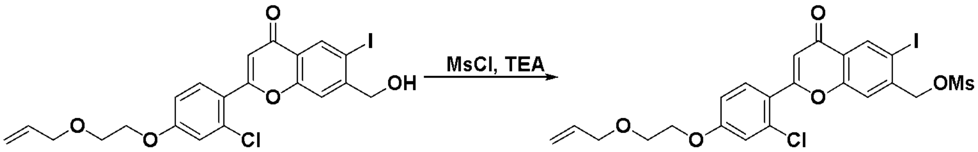


1-6

2-3

1-6 (2 g, 1.1 eq) was added to 250 ml THF. TEA (0.592 g, 1.5 eq) was added at 0 °C under nitrogen protection, and then MsCl (816 mg, 1.2eq) was added dropwise at 0 °C. The mixture was naturally warmed to room temperature, and then the reaction was stirred overnight. LCMS monitoring showed that no raw material remained. The reaction solution was slowly added dropwise to a saturated $NaHCO_3$ aqueous solution for quenching. After quenching, the system pH was between 6 and 7. The mixture was extracted with ethyl acetate, dried over anhydrous sodium sulfate, and concentrated to dryness with rotation. The obtained product was used directly in the next step without further treatment. MS: 590.8.

Synthesis of 2-4

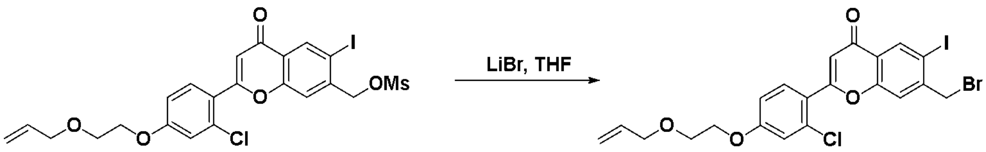


2-3

2-4

2-3 (2 g, 1.0 eq) was added to 200 mL of THF, and LiBr (10 g, 0.eq) was added under nitrogen protection at 110 °C. The reaction was stirred at room temperature overnight. LCMS monitoring showed that no raw material remained. The reaction was quenched with saturated sodium chloride aqueous solution. The mixture was extracted with ethyl acetate, dried over anhydrous sodium sulfate, and concentrated. The residue was purified by normal column (DCM:EA=4:1) to obtain 1.7 g of product 2-4 (yield: 87.6%), MS: 575.6.

Synthesis of 2-5

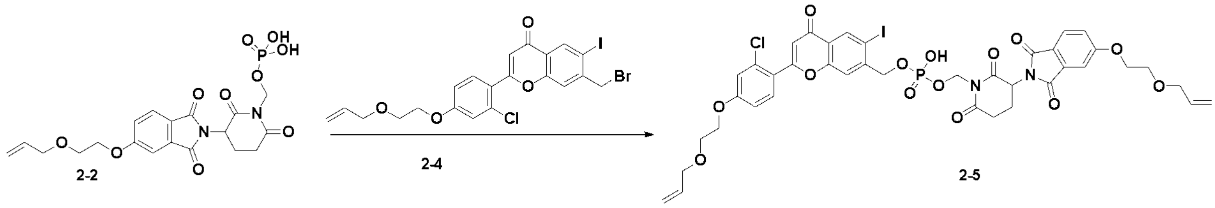

2-2 (0.4 g, 1.0 eq) was added to 20 mL of DMF. $NaHCO_3$ (150 mg, 2.2 eq) was added under nitrogen protection. The mixture was stirred at 30 °C for 20 minutes, and then 2-4 (587 mg, 1.2 eq) was added. The reaction was stirred at 80 °C overnight. LCMS monitoring showed that no raw material remained. The reaction was quenched with saturated aqueous ammonium chloride, extracted with ethyl acetate, washed with saturated brine, dried over anhydrous sodium sulfate, and concentrated. The residue was purified by reverse column (0.1% FA in water/ACN = 40 %) to obtain 200 mg of product 2-5 (yield: 24.3%), MS: 963.1.

Synthesis of Example 2

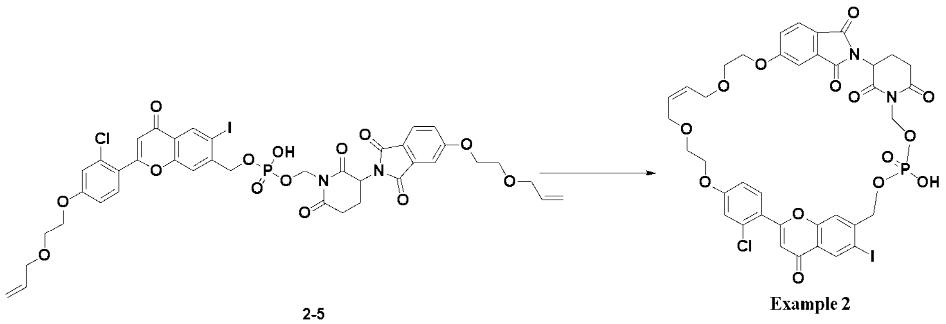

2-5

Example 2

2-5 (1.15 g, 1.0 eq) was added to 140 mL DCE, and Grubbs (II) (304 mg, 0.3 eq) was then added at room temperature. The reaction was monitored by TLC (DCM:MeOH=3:1, $R_f$ = 0.4), and the reaction was stopped at the time point that exhibited the most prominent product formation. The mixture was directly purified by normal column (DCM:MeOH=4:1) to obtain 300 mg of crude product, which was further purified by High-Pressure Liquid Chromatography to give 60 mg of Example 2 (yield: 5.4%).

$^1H$ NMR (400 MHz, DMSO-$d_6$) $\delta$ 8.37 (s, 1H), 7.65 (s, 2H), 7.57 (s, 1H), 7.22 (s, 2H), 7.14 (s, 1H), 7.04 (s, 1H), 6.82 (s, 1H), 6.57 (s, 1H), 5.80 (s, 2H), 5.32 (s, 2H), 4.98 (s, 1H), 4.66 (s, 2H), 4.24 (s, 4H), 4.03 (s, 4H), 3.75 (s, 4H), 2.88 (s, 1H), 2.59 (s, 3H), 1.99 (s, 2H), 1.46 (s, 1H), 1.24 (s, 8H), 0.85 (s, 2H).

MS: 935.

Example 3

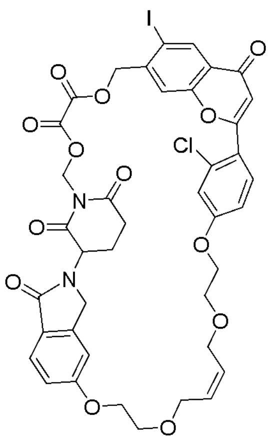

Synthetic route

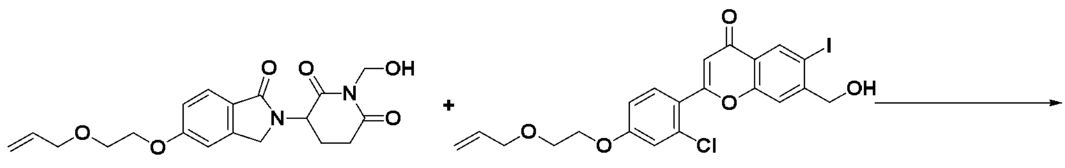

1-3 1-6

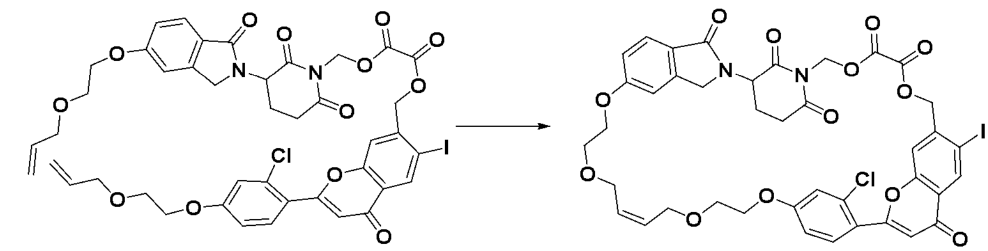

3-1 Example 3

Synthesis of 3-1

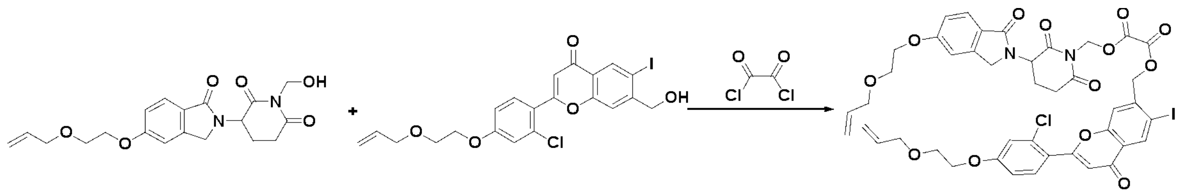

1-3 1-6 3-1

1-3 (314 mg, 1.0 eq) and 1-6 (430 mg, 1.0 eq) were added to 17 mL DCM, and triethylamine (254 mg, 3 eq) was then added under ice bath conditions. The reaction was stirred for 5 minutes, and then oxalyl chloride (128 mg, 1.2 eq) was slowly added to the above system. The reaction was stirred for another 0.5 h under ice bath conditions. LC-MS monitoring showed that no raw material remained. The mixture was directly purified by prep-TLC (DCM:EA = 95:5) to obtain 210 mg of product 3-1 (yield: 27%); MS: 940.1.

Synthesis of Example 3

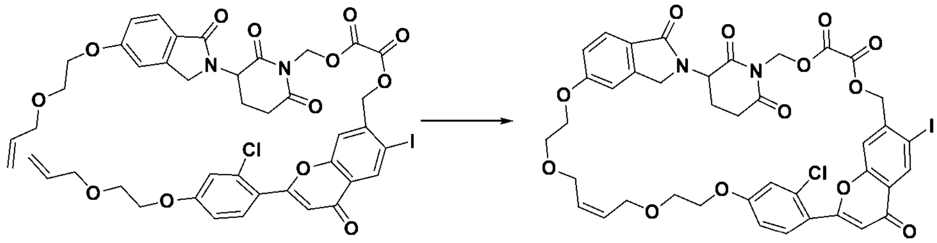

3-1 Example 3

3-1 (150 mg, 1.0 eq) was added to 15 mL DCE. Grubbs (II) (40.5 mg, 0.3 eq) was then added at room temperature. The mixture was heated to 80 °C, and then the reaction was stirred for 1 h. LC-MS monitoring showed that no raw material remained. The mixture was directly purified by prep-TLC ($H_2O$:$CH_3CN$ = 97:3) to obtain 30 mg of product Example 3 (yield:

21%).

$^1$H NMR (400 MHz, DMSO-$d_6$) $\delta$ 8.45 (s, 1H), 7.79 - 7.66 (m, 1H), 7.65 - 7.54 (m, 2H), 6.67 (d, 2H), 5.85 (s, 1H), 5.36 (s, 3H), 4.35 (s, 1H), 3.83 - 3.64 (m, 1H), 3.49 - 3.34 (m, 3H), 2.16 - 1.95 (m, 2H), 1.35 (s, 1H), 1.23 (s, 15H).

MS: 913.1.

Example 4

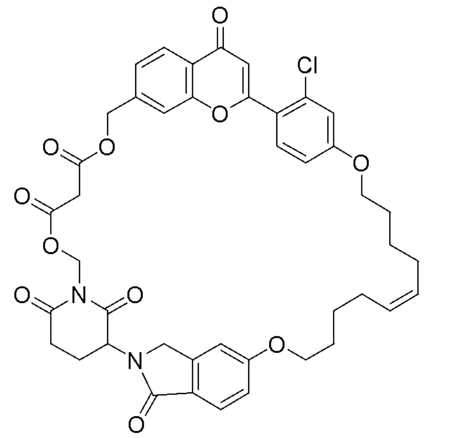

Synthetic route

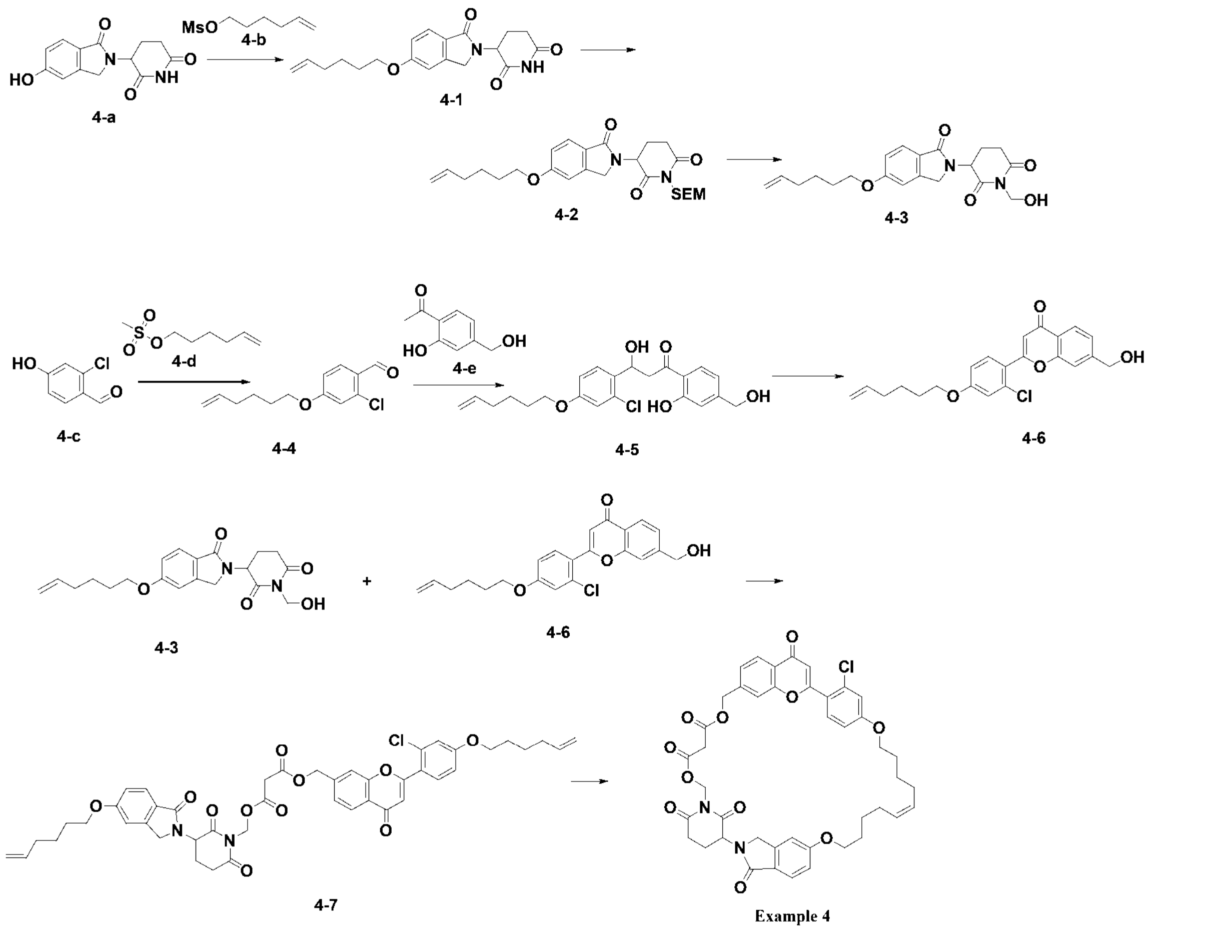

Synthesis of 4-1

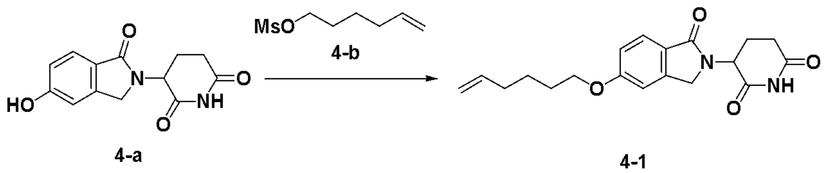


4-a 4-1

4-a (10 g, 1.2 eq) was added to 100 mL of DMF. 4-b (6.84 g, 1 eq), KI (7.5 g, 1.2 eq) and $NaHCO_3$ (6.5 g, 2 eq) were then added sequentially under nitrogen protection. The reaction was stirred at room temperature for 30 min, and then heated to 70 °C and stirred overnight. LCMS monitoring showed that no raw material remained. The reaction was quenched with saturated sodium chloride aqueous solution. The mixture was washed with saturated brine, extracted with ethyl acetate, dried over anhydrous sodium sulfate, and concentrated. The residue was purified by reverse column (0.1% FA in water/ACN =50 %) to obtain 3.2 g of product 4-1, MS: 342.4.

Synthesis of 4-2

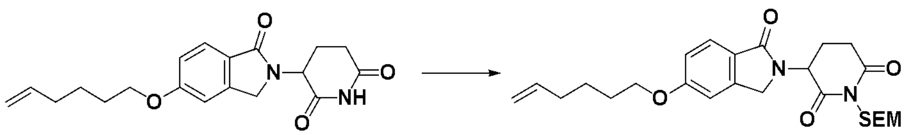

4-1 4-2

4-1 (3.0 g, 1.0 eq) was added to 30 mL DCM, followed by SEMCl (2.1 g, 1.1 eq). DBU (2.2 g, 1.2 eq) was then added at 0 °C. The reaction was stirred at room temperature for 16 h. LCMS monitoring showed that no raw material remained. The reaction was quenched with saturated sodium chloride aqueous solution. The mixture was extracted with ethyl acetate, dried over anhydrous sodium sulfate, and concentrated. The residue was purified by reverse column (0.1% FA in water/ACN =50 %) to obtain 3.4 g of product 4-2, MS: 472.7.

Synthesis of 4-3

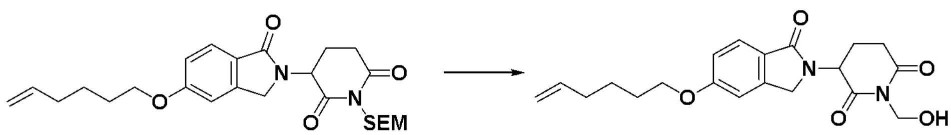

4-2 4-3

4-2 (3.0 g, 1.0 eq) was added to 30 mL DCM, followed by TFA (6 mL). The reaction was stirred at room temperature for 3 h. LCMS monitoring showed that no raw material remained. The reaction was concentrated directly. The residue was purified by reverse column (0.1% FA in water/ACN =50 %) to obtain 2 g of product 4-3, MS: 372.4.

Synthesis of 4-4

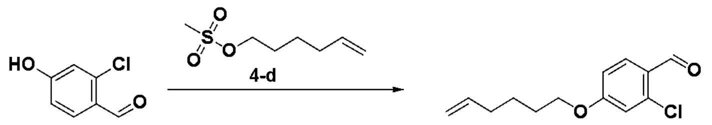


4-c 4-4

4-c (20 g, 1.0 eq) was added to 30 mL CAN. 4-d (27 g, 1.2 eq) and $K_2CO_3$ (21.2 g, 1.2 eq) were then added, and the reaction was stirred at 70 °C for 6 h. LCMS monitoring showed that no raw material remained. The reaction was quenched with saturated sodium chloride aqueous solution. The mixture was extracted with ethyl acetate, dried over anhydrous sodium sulfate, and concentrated. The residue was purified by normal column to obtain 20 g of product 4-4, MS: 238.7.

Synthesis of 4-5

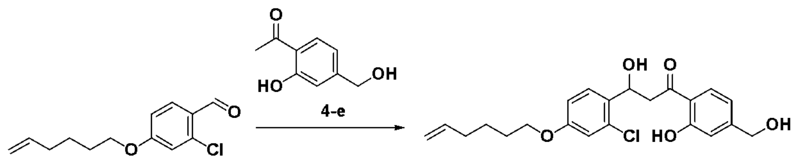


4-4 4-5

4-4 (3 g, 2.0 eq) and 4-e (1 g, 1 eq) were added to 30 mL THF. Potassium tert-butoxide (1.35 g, 2 eq) was then added, and the reaction was stirred at 0 °C for 6 h. LCMS monitoring showed that no raw material remained. The reaction was quenched with saturated sodium chloride aqueous solution and adjusted to pH~5 with hydrochloric acid. The mixture was extracted with ethyl acetate, dried over anhydrous sodium sulfate, and concentrated. The residue was purified by normal column to obtain 1.4 g of product 4-5, MS: 404.9.

Synthesis of 4-6

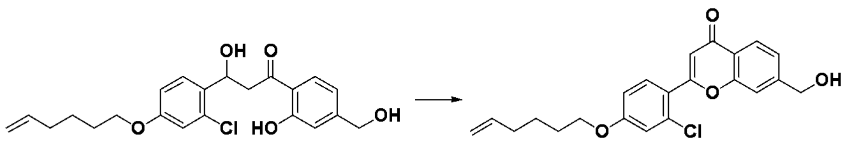

4-5 4-6

4-5 (1.5 g, 1.0 eq) was added to 15 mL DMSO. A catalytic amount of iodine was then added, and the reaction was stirred at 110 °C for 6 h. LCMS monitoring showed that no raw material remained. The reaction was quenched with saturated sodium chloride aqueous solution and then sodium thiosulfate was added. The mixture was extracted with ethyl acetate, dried over anhydrous sodium sulfate, and concentrated. The residue was purified by normal column to obtain 900 mg of product 4-6, MS: 384.9.

Synthesis of 4-7

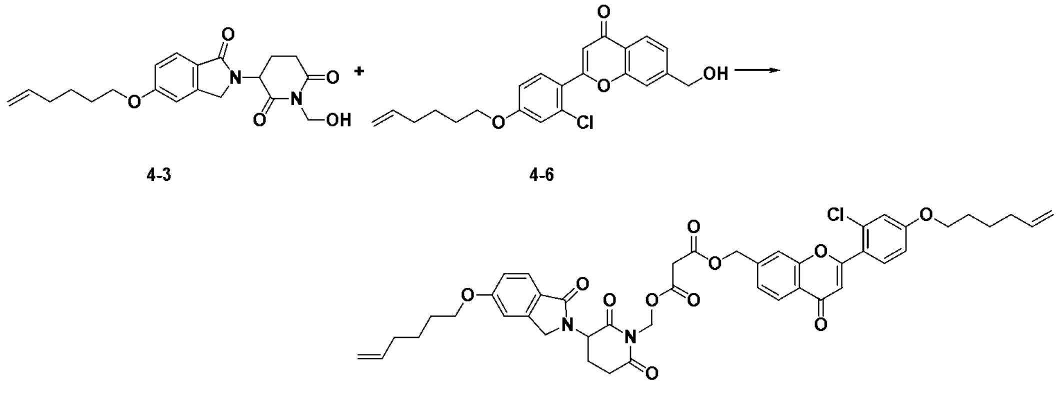

4-3

4-6

4-7

4-6 (300 mg, 1.0 eq) and 4-3 (290 mg, 1.0 eq) were added to 15 mL DCM. Malonyl chloride (100 mg, 1 eq) was then added, and the reaction was stirred at 0 °C for 2 h. LCMS monitoring showed that no raw material remained. The reaction was concentrated directly. The residue was purified by normal column to obtain 150 mg of product 4-7, MS: 825.3.

Synthesis of Example 4

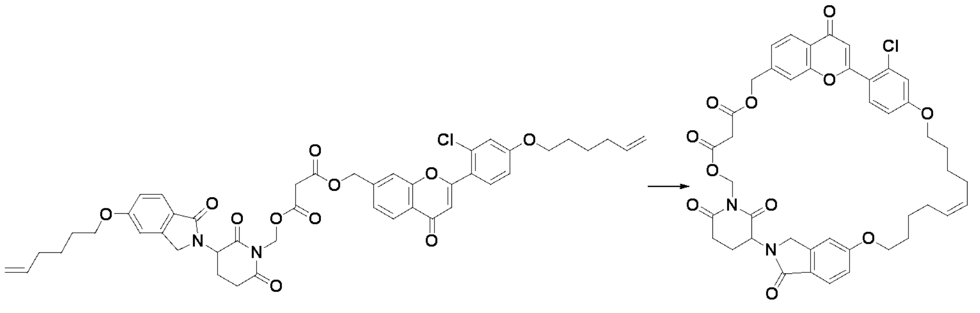

4-7

Example 4

4-7 (166 mg, 1.0 eq) was added to 17 mL DCE. Grubbs (II) (51.3 mg, 0.3 eq) was then added at room temperature. The mixture was heated to 70 °C, and then the reaction was stirred for 30 min. The reaction was monitored by TLC (DCM:MeOH=30:1, $R_f$ =0.4). The residue was purified by prep-TLC to obtain 37 mg of Example 4 (yield: 23.1%).

$^1$H NMR (400 MHz, DMSO-$d_6$) $\delta$ 8.03 (d, J = 8.1 Hz, 1H), 7.72 (d, J = 8.6 Hz, 1H), 7.66 (s, 1H), 7.56 (d, J = 8.3 Hz, 1H), 7.47 (d, J = 8.3 Hz, 1H), 7.21 (d, J = 2.5 Hz, 1H), 7.09 (s, 1H), 7.05 (s, 1H), 6.96 (d, J = 8.2 Hz, 1H), 6.60 (s, 1H), 5.80 (d, J = 9.7 Hz, 1H), 5.60 (d, J = 9.6 Hz, 1H), 5.42 (s, 2H), 5.33 (d, J = 6.9 Hz, 2H), 5.23 (d, J = 5.2 Hz, 1H), 4.29 (d, J = 17.0 Hz, 1H), 4.17 - 4.06 (m, 3H), 3.98 (d, J = 6.2 Hz, 2H), 3.70 (d, J = 11.5 Hz, 2H), 3.05 (s, 1H), 2.81 (s, 1H), 2.07 - 1.97 (m, 5H), 1.70 (s, 4H), 1.47 (s, 5H), 1.23 (s, 7H), 0.85 (d, J = 6.9 Hz, 1H).

MS:797.2.

Example 5

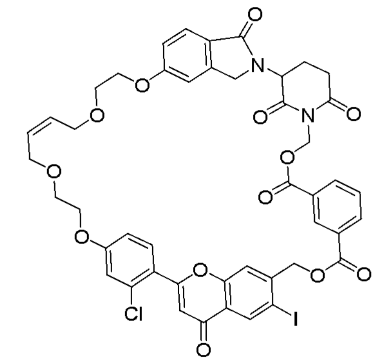

Synthetic route

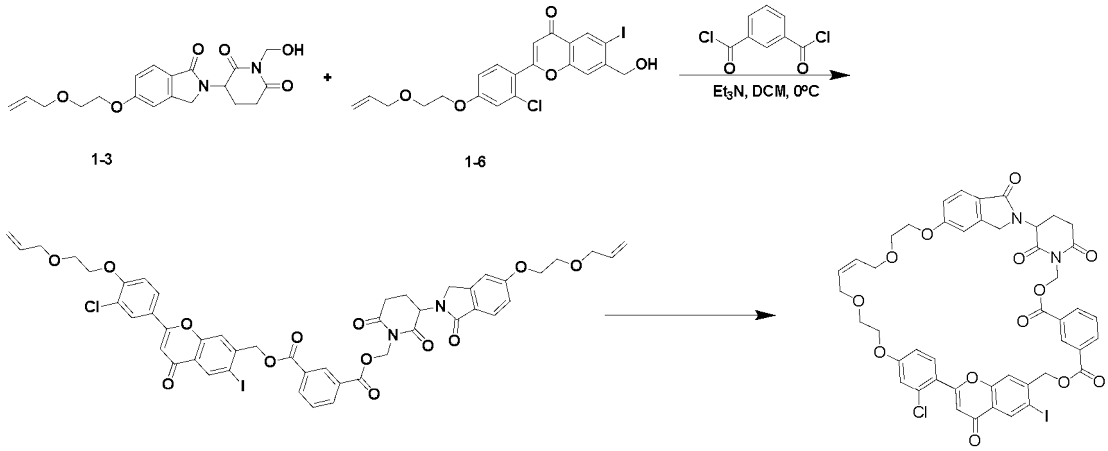


1-3

1-6

5-1

Example 5

Synthesis of 5-1

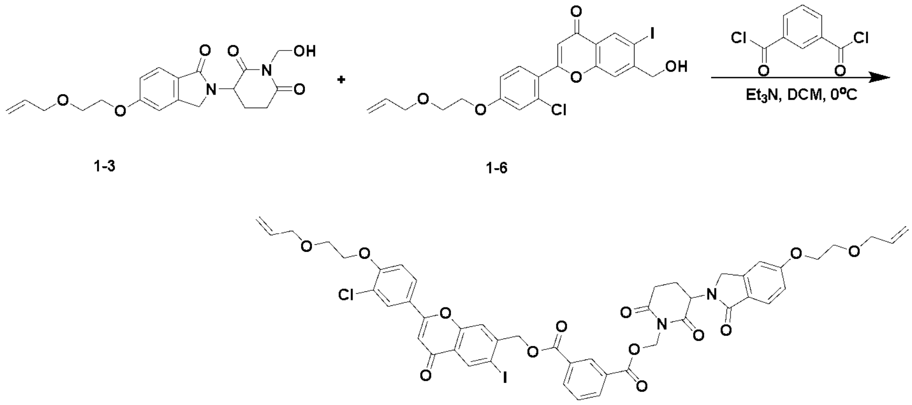


1-3

1-6

5-1

1-3 (374 mg, 1.0 eq) and 1-6 (512 mg, 1.0 eq) were added to 20 mL DCM. Triethylamine (303 mg, 3 eq) was then added under ice bath conditions. The reaction was stirred for 5 minutes, and then isophthaloyl chloride (244 mg, 1.2 eq) was slowly added to the above system. The reaction was stirred for another 0.5 h under ice bath conditions. LC-MS monitoring showed that no raw material remained. The mixture was directly purified by prep-TLC (DCM:EA = 95:5) to give 217 mg of product 5-1 (yield: 21%), MS: 1017.1.

Synthesis of Example 5

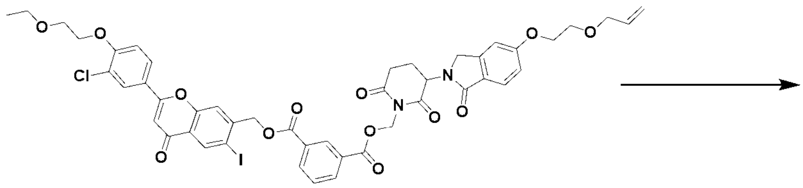

5-1

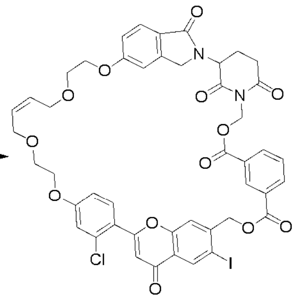

Example 5

5-1 (217 mg, 1.0 eq) was added to 23 mL DCE. Grubbs (II) (54.3 mg, 0.3 eq) was then added at room temperature. The mixture was heated to 80 °C, and then the reaction was stirred for 1 h. LC-MS monitoring showed that no raw material remained. The mixture was directly purified by prep-TLC to obtain 42 mg of product Example 5 (yield: 20%).

$^1$H NMR (400 MHz, DMSO-$d_6$) $\delta$ 8.24 (m, 1H), 8.12 (m, 1H), 7.82 - 7.66 (m, 2H), 7.69 - 7.52 (m, 1H), 7.21 (d, $J$ = 2.5 Hz, 1H), 7.11 - 6.95 (m, 2H), 6.62 (d, 1H), 6.03 - 5.84 (m, 1H), 5.59 (d, 1H), 5.43 (s, 1H), 5.38 - 5.21 (m, 1H), 4.35 - 4.12 (m, 1H), 4.09 (m, 2H), 4.06 - 3.88 (m, 1H), 2.08 - 1.97 (m, 6H), 1.71 (d, 1H), 1.47 (d, 1H), 1.34 (d, 2H), 1.28 (s, 3H), 1.25 (s, 10H), 1.01 - 0.77 (m, 3H).

MS: 989.3.

Example 6

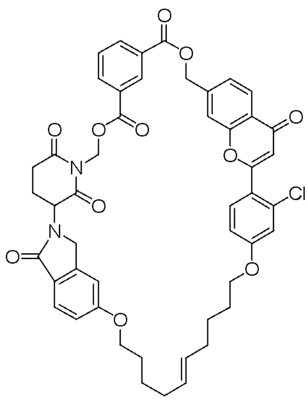

Synthetic route

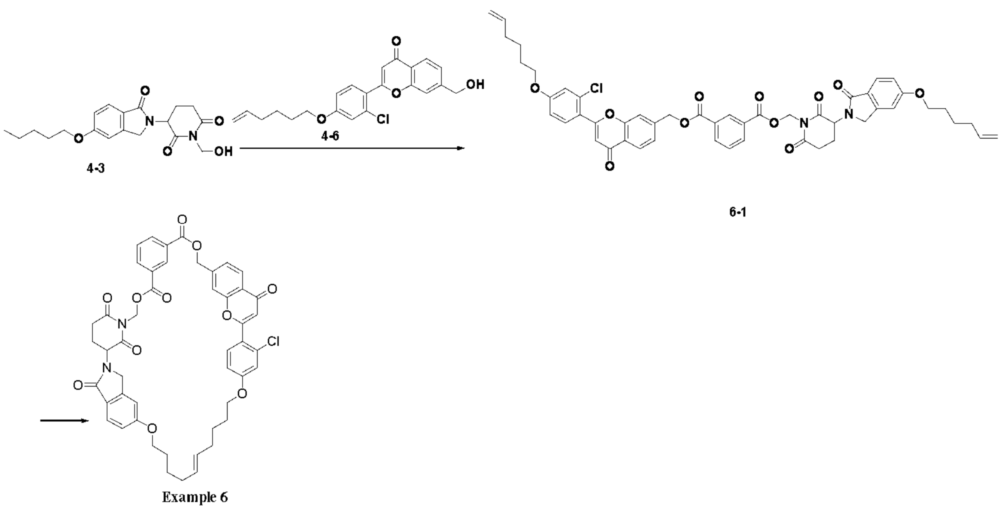

4-3 4-6 6-1 Example 6

Synthesis of 6-1

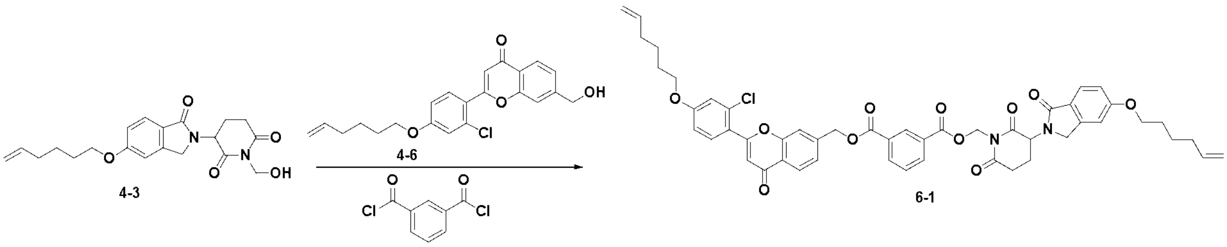

4-3 4-6 6-a 6-1

4-6 (300 mg, 1.0 eq) and 4-3 (290 mg, 1.0 eq) were added to 15 mL DCM. 6-a (157.8 mg, 1 eq) was then added, and the reaction was stirred at 0 °C for 2 h. LCMS monitoring showed that no raw material remained. The reaction was concentrated directly to obtain product 6-1 of 70 mg, MS: 887.4.

Synthesis of Example 6

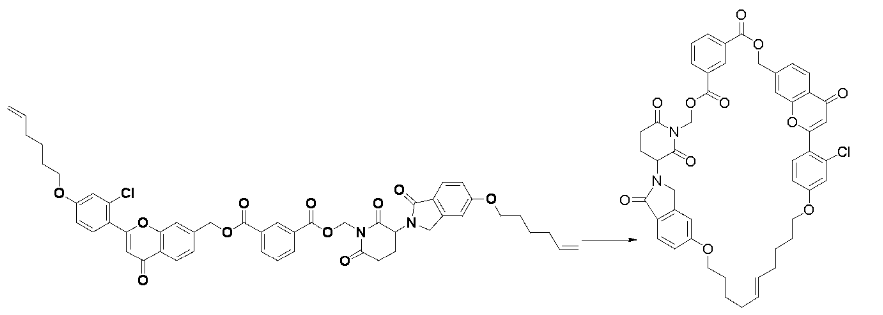

6-1 Example 6

6-1 (70 mg, 1.0 eq) was added to 17 mL DCE. Grubbs (II) (30 mg, 0.3 eq) was then added at room temperature. The mixture was heated to 70 °C, and then the reaction was stirred for 30 min. The reaction was monitored by TLC. The mixture was directly purified by Prep-TLC to obtain 11 mg of product Example 6 (yield: 23.1%).

$^1$H NMR (400 MHz, DMSO-$d_6$) $\delta$ 8.51 (s, 1H), 8.35 (d, J = 7.8 Hz, 1H), 8.24 (d, J = 7.8 Hz, 1H), 8.12 (dd, J = 8.2, 4.8 Hz, 1H), 7.81 - 7.74 (m, 2H), 7.73 (s, 1H), 7.65 (d, J = 8.2 Hz, 1H), 7.58 (d, J = 8.3 Hz, 1H), 7.21 (d, J = 2.5 Hz, 1H), 7.06 (dd, J = 8.8, 2.5 Hz, 2H), 7.00 (d, J = 8.5 Hz, 1H), 6.62 (d, J = 5.0 Hz, 1H), 5.98 (d, J = 9.6 Hz, 1H), 5.92-5.86 (m, 1H).5.42 (s, 2H), 5.33 (d, J = 6.9 Hz, 2H), 5.23 (d, J = 5.2 Hz, 1H), 4.29 (d, J = 17.0 Hz, 1H), 4.17 - 4.06 (m, 3H), 3.98 (d, J = 6.2 Hz, 2H), 3.70 (d,

J = 11.5 Hz, 2H), 3.05 (s, 1H), 2.81 (s, 1H), 2.07 - 1.97 (m, 5H), 1.70 (s, 4H), 1.47 (s, 5H), 1.23 (s, 7H), 0.85 (d, J = 6.9 Hz, 1H)

MS: 859.3.

Example 7

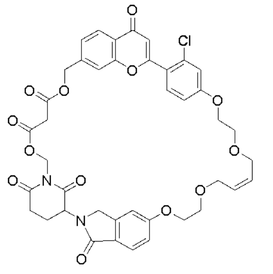

Synthetic route

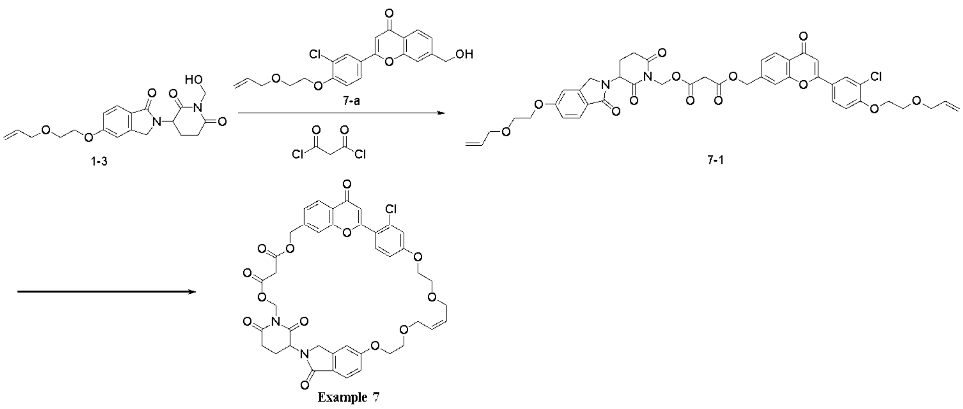


Example 7

Synthesis of 7-1

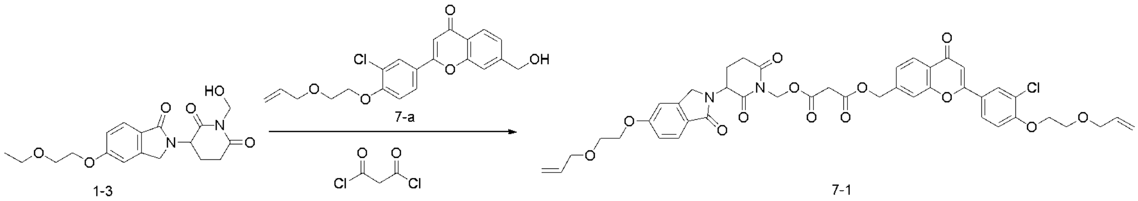


1-3 (1.0 g, 1.0 eq) and 7-a (1.03 g, 1.0 eq) were added to 50 mL DCM. Triethylamine (810.1 mg, 3 eq) was then added under ice bath conditions. The reaction was stirred for 5 minutes, and then malonyl chloride (561.3 mg, 1.5 eq.) was slowly added dropwise to the above system. The reaction was stirred for another 0.5 h under ice bath conditions. LC-MS monitoring showed that no raw material remained. The mixture was directly purified by prep-TLC to give 300 mg of the product; MS: 829.

Synthesis of Example 7

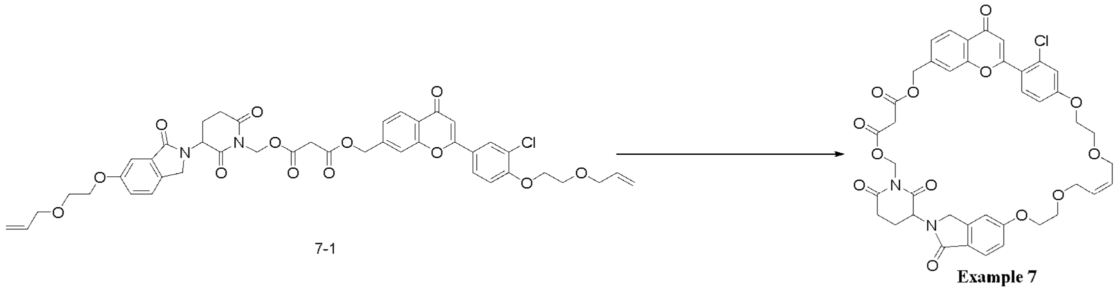

7-1

Example 7

7-1 (300 mg, 1.0 eq) was added to 50 mL DCE. Grubbs (II) (92.8 mg, 0.3 eq) was then added at room temperature. The mixture was heated to 80 °C, and then the reaction was stirred for 60 min. The reaction was monitored by TLC. The mixture was purified by prep-TLC to obtain 85 mg of product Example 7 (yield: 26.1%).

$^{1}H$ NMR (400 MHz, DMSO-$d_6$) $\delta$ 8.06-8.02 (m, 1H), 7.75 (d, J = 13.0 Hz, 4H), 7.3-6.95 (m, 4H), 6.70 - 6.61 (m, 1H), 5.82 (d, J = 8.3 Hz, 2H), 5.37 - 5.10 (m, 5H), 4.52 - 3.89 (m, 8H), 3.78 (d, J = 26.3 Hz, 6H), 3.43 (d, J = 27.5 Hz, 2H), 3.08 (s, 1H), 2.67 (s, 2H), 2.33 (s, 1H)

MS: 801.2.

Example 8

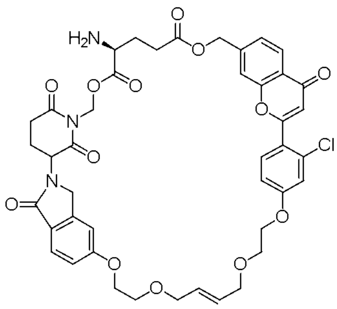

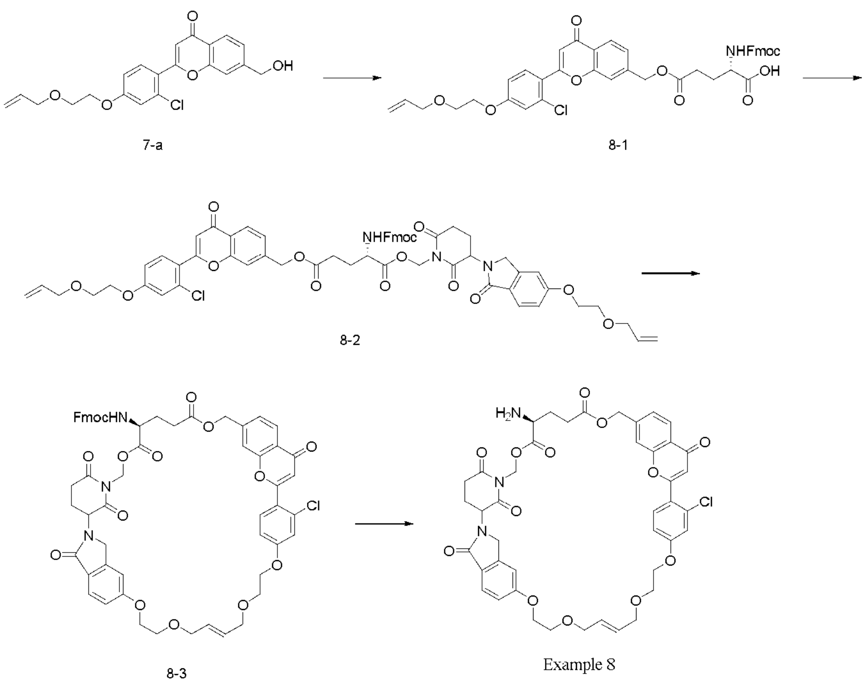

7-a

8-1

8-2

8-3

Example 8

Synthesis of 8-1

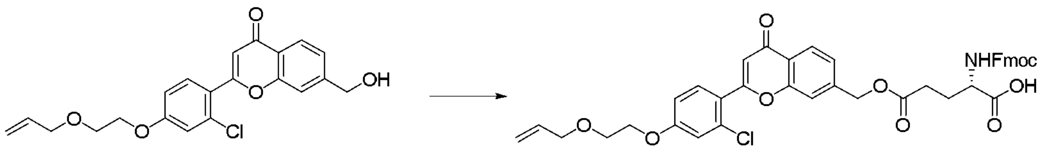

7-a 8-1

5 g of 7-a was added to 100 ml THF. 23.9 g of N-Fmoc-glutamic acid, 5 g of $PPh_3$ and 4.11 g of DEAD were added under stirring. The reaction was stirred at room temperature under $N_2$ protection conditions until 8-1 was completely consumed. The reaction solution was then directly evaporated to dryness with rotation, and the residue was purified by column chromatography to obtain 6 g of product 8-2. MS: [M+H] 738.2.

Synthesis of 8-2

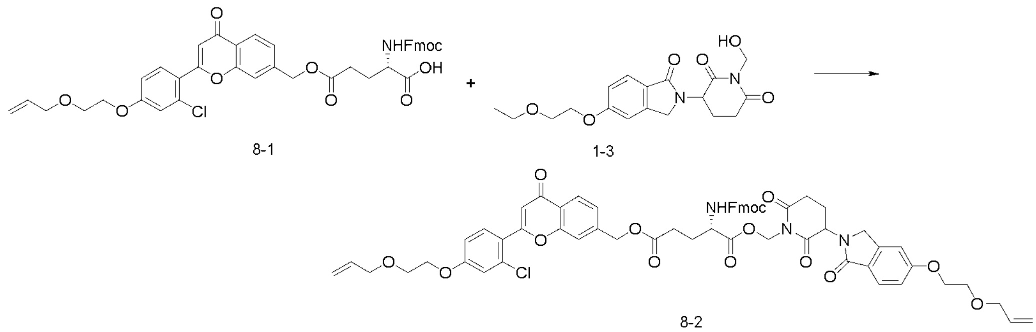

8-1 1-3

8-2

6 g of 8-1 was added to 100 ml THF. 3.65 g of 1-3, 3.2 g of $PPh_3$ and 2.5 g of DEAD were added under stirring at 0 °C under $N_2$ protection conditions. The reaction was stirred at room temperature until 8-1 was completely consumed. The reaction solution was directly evaporated to dryness with rotation, and the residue was purified by column chromatography to obtain 4.5 g of product, which was directly used in the next step reaction. MS: [M+H] 1094.3

Synthesis of 8-3

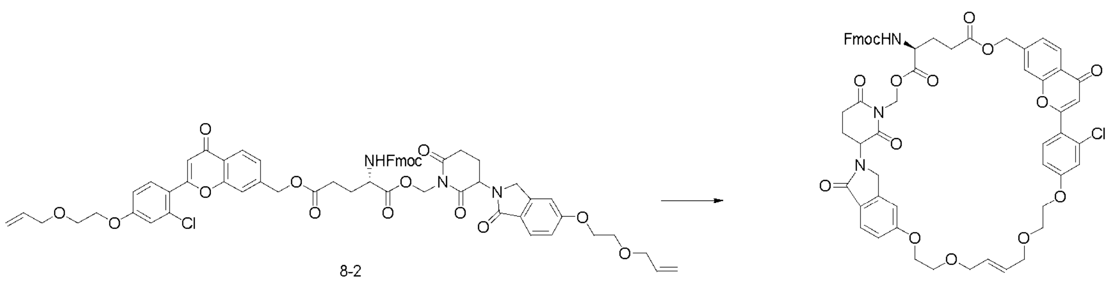

8-2

8-3

600 mg of 8-2 was added to 100 ml of DCE. Grubbs catalyst was then added under stirring, and the mixture was stirred until 8-2 was completely consumed. The reaction solution was evaporated to dryness, and the residue was purified by column chromatography to obtain 300 mg of 8-3. MS: [M+H] 1066.3.

Synthesis of Example 8

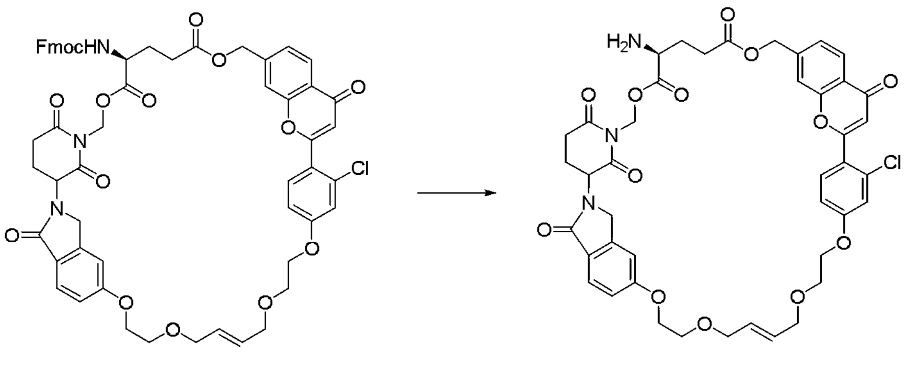

8-3

Example 8

282 mg of 8-3 was added to 90 ml of THF. 11 ml of TEA was then added under stirring. After the addition, the mixture was stirred at room temperature until 8-3 was completely consumed. The mixture was separated by column chromatography to obtain 50 mg product. MS: [M+H] 844.

$^1H$ NMR (400 MHz, DMSO-d6) $\delta$ 8.54 (s, 2H), 8.05 (d, J = 8.0 Hz, 1H), 7.78 - 7.70 (m, 2H), 7.56 (dt, J = 18.6, 9.7 Hz, 3H), 7.27 (s, 1H), 7.11 (d, J = 6.6 Hz, 2H), 7.03 (s, 1H), 6.61 (s, 1H), 5.78 (s, 2H), 5.66 (d, J = 5.7 Hz, 1H), 5.45 - 5.30 (m, 2H), 5.22 (s, 2H), 4.32 (s, 1H), 4.19 (dd, J = 32.2, 14.7 Hz, 8H), 4.02 (s, 4H), 3.72 (s, 4H), 2.57 (s, 2H), 2.08 (s, 4H).

Example 9

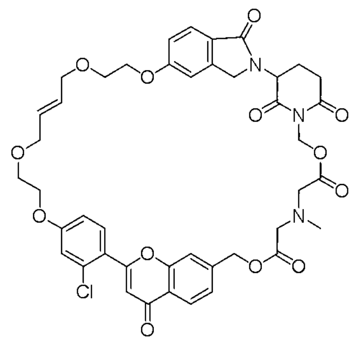

Synthetic route

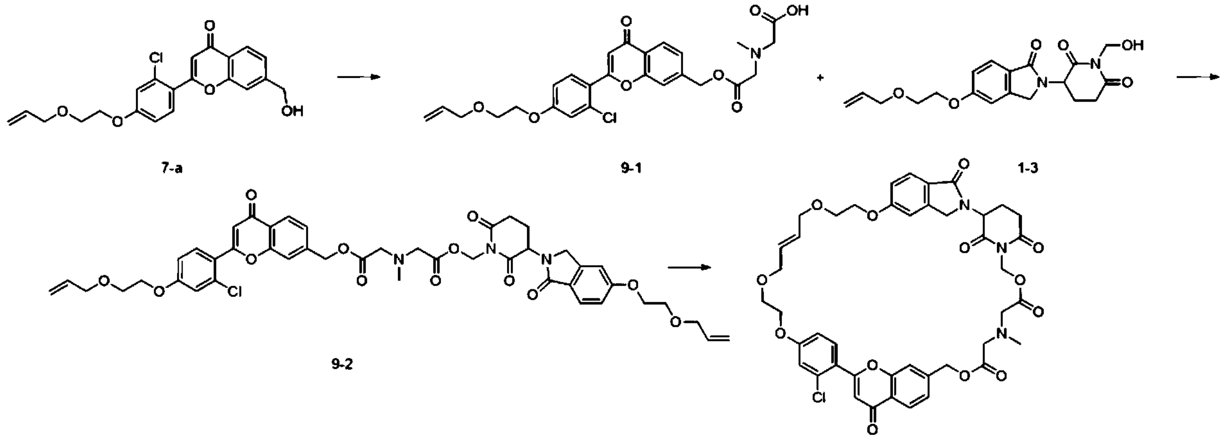

7-a 9-1 1-3

9-2

Example 9

Synthesis of 9-1

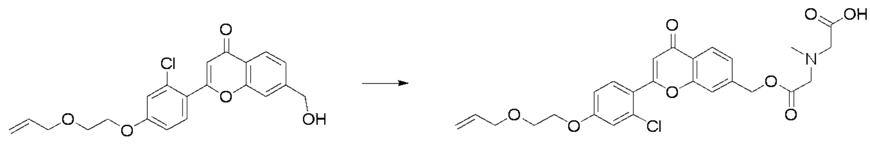

7-a 9-1

200 mg of 7-a was added to 10 ml DMF and then cooled to 0 °C in an ice-water bath. 31 mg of NaH was then added at 0 °C. The mixture was stirred after the addition was completed, and then the mixture was stirred for 0.5 h under $N_2$ protection at a controlled temperature. 4-Methylmorpholine-2,6-dione dissolved in DMF was added dropwise to the reaction solution. The mixture was stirred overnight until the raw material disappeared and the product was generated. The mixture was quenched with saturated $NH_4Cl$, and the reaction solution was concentrated by evaporation. The residue was separated by column chromatography to obtain 210 mg of target product 9-1: [M+H] 516.

Synthesis of 9-2

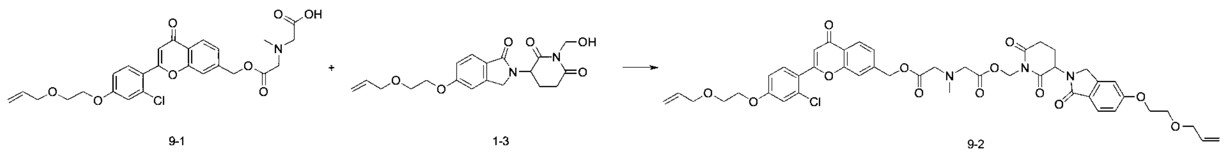

9-1 1-3 9-2

100 mg of 9-1, 80 mg of 1-3 and 76.2 mg of triphenylphosphine were dissolved in 10 ml of THF, and 64.3 mg of DIAD was then added in an ice-water bath. The mixture was stirred after the addition was completed, and then the mixture was stirred for 16 h at room temperature under $N_2$ protection. The system was cooled to 0 °C, and quenched with pure water. The reaction solution was concentrated by evaporation, and the residue was separated by column chromatography to obtain 110 mg of target product 9-2: [M+H] 872.

Synthesis of Example 9

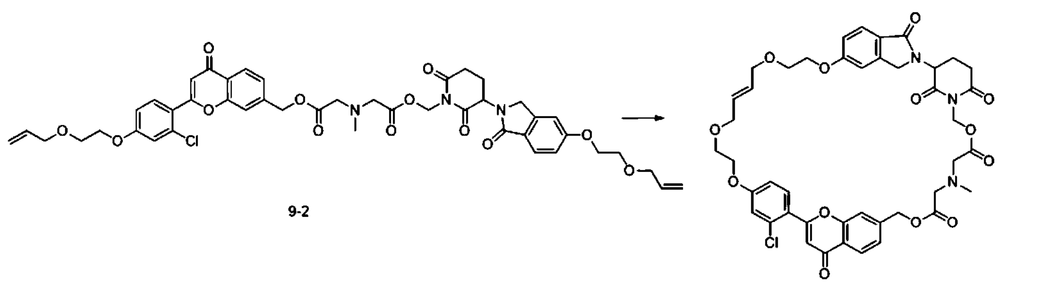

9-2

Example 9

100 mg of 9-2 was dissolved in 100 ml of DCE, and Grubbs (II) was then added at room temperature. The mixture was heated to 90 °C and stirred for 0.5 h under $N_2$ protection. The reaction solution was concentrated by evaporation. The residue was dissolved and then purified by column chromatography to obtain 6.4 mg of target product **Example** 9: [M+H] 844.

$^1$H NMR (400 MHz, DMSO-$d_6$) δ 8.03 (d, *J* = 8.1 Hz, 1H), 7.74 (d, *J* = 8.7 Hz, 1H), 7.65 -7.56 (m, 2H), 7.48 (d, J= 7.8 Hz, 1H), 7.27 (d, J= 2.4 Hz, 1H), 7.15 -7.08 (m, 2H), 7.03 (dd, J= 8.4, 2.2 Hz, 1H), 6.61 (s, 1H), 5.77 (s, 2H), 5.69 (d, *J* = 9.6 Hz, 1H), 5.60 (d, *J* = 9.6 Hz, 1H), 5.28 (s, 2H), 5.23 (dd, *J* = 13.6, 5.1 Hz, 1H), 4.34 (d, *J* = 17.2 Hz, 1H), 4.26-4.10 (m, 5H), 4.01 (d, *J* = 3.0 Hz, 3H), 3.72 (d, *J* = 4.1 Hz, 4H), 3.58 (s, 2H), 3.47 (s, 2H), 3.29 (s, 1H), 3.05 (d, *J* = 13.6 Hz, 1H), 2.81 (d, *J* = 17.1 Hz, 1H), 2.40 (s, 3H), 2.35-2.28 (m, 1H), 2.15-1.95 (m, 1H).

Example 10

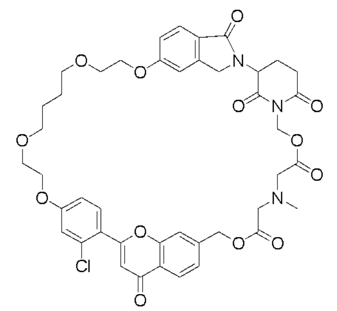

Synthetic route

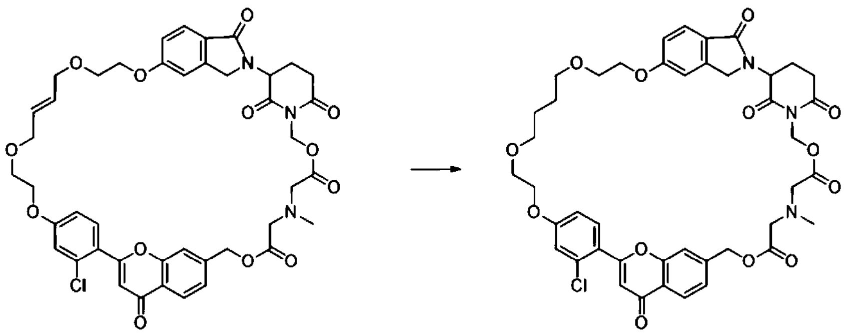

Example 9 Example 10

Synthesis of Example 10

350 mg Example 9 was added to 30 ml THF. Lindlar reagent (10 mg) and quinoline (10 mg, 0.2 eq.) were then added, and the reaction was stirred at room temperature for 0.5 h. Then the reaction was stirred at room temperature under $H_2$ atmosphere for 6 h. The reaction solution was concentrated by evaporation, and the residue was separated by column

chromatography to obtain 85 mg of the target product **Example 10:** [M+H] 846.

$^1$H NMR (400 MHz, DMSO-$d_6$) $\delta$ 8.03 (d, *J* = 8.0 Hz, 1H), 7.74 (d, *J* = 8.8 Hz, 1H), 7.63 (s, 1H), 7.58 (d, *J* = 8.4 Hz, 1H), 7.48 (dd, *J* = 8.2, 1.6 Hz, 1H), 7.27 (d, *J* = 2.5 Hz, 1H), 7.11 (dt, *J* = 5.8, 2.8 Hz, 2H), 7.02 (dd, *J* = 8.5, 2.2 Hz, 1H), 6.61 (s, 1H), 5.69 (d, *J* = 9.6 Hz, 1H), 5.60 (d, *J* = 9.6 Hz, 1H), 5.28 (s, 2H), 5.22 (dd, *J* = 13.4, 5.1 Hz, 1H), 4.34 (d, *J* = 17.0 Hz, 1H), 4.26-4.11 (m, 5H), 3.73-3.64 (m, 4H), 3.57 (s, 2H), 3.46 (d, *J* = 4.2 Hz, 6H), 3.29 (s, 1H), 3.13-2.99 (m, 1H), 2.81 (d, *J* = 17.8 Hz, 1H), 2.40 (s, 3H), 2.05-1.97 (m, 1H), 1.55 (d, *J* = 6.1 Hz, 4H).

Activity Experiment

1. The biological activity of compounds was determined by Western Blot method using the following method:

Reagents: Complete 1640 medium was purchased from Gibco. Fetal bovine serum was purchased from ThermoFisher. Trypsin was produced by ThermoFisher. SHP-2 antibody was produced by CST.

Cell line: SUM159 triple-negative breast cancer cell line was established by Rovera.

Method for assay: Cells growth in the logarithmic phase were collected and suspended in a 1640 medium containing 10% fetal bovine serum, which was then gently pipetted into a single-cell suspension. Viable cells were counted under microscope. 9 mL of the cell suspension was seeded and cultured in a 10 cm culture dish at a final concentration of 2 x $10^5$ cells/mL. After cells were pre-cultured for 24 hours at 37 °C and 5% $CO_2$, different concentrations of solutions of the compounds of the present disclosure in DMSO were added (the final concentration of DMSO was no more than 0.5%), and the negative control group was added with the same volume of DMSO alone. After cultured for another 24 hours, all cells were collected and washed with PBS. The cells were lysed with RIPA lysis buffer, and the lysates were collected into a 1.7 mL centrifuge tube. The proteins were fully denatured after heating in the metal bath at 95 °C for 10 minutes. After loading the samples onto the gel for Western blotting, electrophoresis was performed at a constant voltage of 100V until the band of bromophenol blue reached the terminal point. The proteins were transferred onto a cellulose acetate membrane using the wet transfer method at a constant current of 300-500 mA for 45 minutes, and the proteins were blocked with 5% BSA at room temperature for 1-2 hours. The proteins were incubated with primary antibody diluted 1:500 to 1:8000 at room temperature for 2 hours, and then washed three times. Subsequently, the proteins were incubated with secondary antibody at room temperature for 1 hour. The detection of proteins was performed using ultrasensitive ECL luminescent liquid such as Enlight, and signals were captured using an automatic X-ray film developing machine. For the test results, see the in vitro Western Blot experiment of Example 1 and the in vitro Western Blot experiment of Example 3.

The above result shows that the $DC_{50}$ of Example 1 and Example 3 for degradation of SHP2 in vitro are 210 nM and 37 nM, respectively. The compounds of the present disclosure have good in vitro anti-solid tumor and anti-blood tumor activities and have low toxicity to normal cells.

2. The biological activity of compounds was determined by CCK8 method using the following method:

Reagents: Complete RPMI 1640 medium was purchased from Gibco. Fetal bovine serum was purchased from ThermoFisher. Trypsin was produced by ThermoFisher. SHP-2 antibody was produced by CST. CCK8 was produced by abcam.

Cell line: SUM159 triple-negative breast cancer cell line was established by Rovera.

Method for assay: Cells growth in the logarithmic phase were collected and suspended in a RPMI 1640 medium containing 10% fetal bovine serum, which was then gently pipetted into a single-cell suspension. Viable cells were counted under microscope. The cells were seeded into a 96-well plate at a density of 3-6* $10^3$ cells per well and cultured in an incubator for 24 hours. Test compounds were diluted in multiples, and then the cell culture supernatant was added. The cells were cultured for another 72 hours, and 10 μL of CCK8 detection solution was added to each well. After incubating for 1-4 hours, the absorbance was measured using a microplate reader.

3. PAMPA Experiment

Reagents: lecithin, n-dodecane, HEPES, dimethyl sulfoxide (DMSO), acetonitrile

Instrument and equipment: Multiscreen 96-well plate (up Merck Millipore), Multiscreen 96-well plate (down Merck Millipore), electronic analytical balance, constant temperature incubator, high performance liquid chromatography-mass spectrometry (LC-MS)

Method for operation: Preparation of PAMPA artificial membrane: about 18 mg of lecithin was weighed accurately, and placed into a 1.5 mL EP tube. 1 mL of n-dodecane was added. The mixture was dissolved by ultrasonication, and then thoroughly mixed to obtain a 1.8% lecithin membrane.

The upper layer of the PAMPA model was the plate lid, the middle layer was the 96-well filter plate (donor plate), and the lower layer was the accepter plate. 5 μL of the above 1.8% lecithin membrane was added to the filter membrane of

the donor plate, and then 150 μL of the drug test solution (10 μM) was added. 300 μL of HEPES solution was added to each well of the accepter plate (completed within 10 minutes). The donor plate was slowly placed onto the accepter plate and incubated at 37 °C for 16 hours.

LC-MS detection: 100 μL of the positive control and the test compound was weighed respectively, to which 100 μL of acetonitrile-DMSO solution [acetonitrile-DMSO (4:1)] was added. The mixture was mixed well, and detected by LC-MS at 0H and 16H. The corresponding molecular ion peak was extracted and the peak area was obtained; After the PAMPA model was incubated at 37 °C for 16h, 100 μL of the test solution in the donor plate and the accepter plate was weighed respectively, to which 100 μL of acetonitrile-DMSO solution [acetonitrile-DMSO (4:1)] was added. The mixture was mixed well, and detected by LC-MS. The corresponding molecular ion peak was extracted, the peak area was obtained, and the concentration was calculated.

Calculation formula:

$$Log\ P_e = Log\left\{C \times \left[-Ln\left(1 - \frac{[drug]_{acceptor}}{[drug]_{equilibrium}}\right)\right]\right\}$$

The activities of representative compounds of the present disclosure are shown in Table 1.

Table 1

| Compound | $DC_{50}$ (nM) | $IC_{50}$ (nM) | PAMPA ($-Log\ P_e$) |
|---|---|---|---|
| Example 1 | 210 | 28.83 | 6.71 |
| Example 2 | - | 1000 | 6.0 |
| Example 3 | 37 | 7.427 | 5.94 |
| Example 4 | - | 717.8 | 6.11 |
| Example 5 | 500 | 200.2 | 5.78 |
| Example 6 | 10 | >5000 | 8.55 |
| Example 7 | 188 | 23.11 | - |
| Example 8 | - | 5.326 | 4.68 |
| Example 9 | 4.21 | 2.39 | 5.52 |
| Example 10 | 5.94 | 3.5 | 5.56 |

## Claims

**1.** A compound represented by formula (I), or a pharmaceutically acceptable salt, a stereoisomer, a tautomer, a cis-trans isomer, a solvate, a hydrate, a polymorph, a prodrug or an isotope variant thereof, and a mixture thereof:

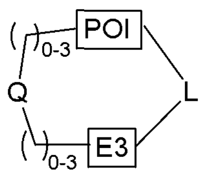

(I)

wherein,

the POI is

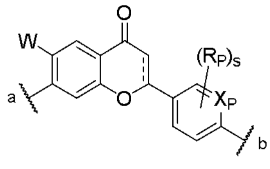;

- - - represents a single bond or a double bond, alternatively a double bond;
each $R_P$ is independently selected from H, D, halogen, $C_{1-6}$ alkyl, $C_{1-6}$ haloalkyl, $C_{2-6}$ alkenyl and $C_{2-6}$ alkynyl;
s is selected from 1, 2 and 3;
$X_P$ is selected from N and $CR_P$;
W is selected from H, D, halogen, $C_{1-6}$ alkyl, $C_{1-6}$ haloalkyl, $C_{2-6}$ alkenyl, $C_{2-6}$ alkynyl, $C_{6-10}$ aryl and 5- to 10-membered heteroaryl;
said W is optionally substituted with 1, 2 or 3 $R_W$;
Rw is selected from H, D, $-C(O)R_a$, $-C(O)NR_bR_c$, $-C(O)OR_a$, $-OC(O)R_a$, $-NR_bC(O)R_a$, $C_{1-6}$ alkyl, $C_{1-6}$ haloalkyl, $C_{2-6}$ alkenyl and $C_{2-6}$ alkynyl;
the a-terminal of the POI is connected to Q via $C_{0-3}$ alkylene, and the b-terminal is connected to L;
L is

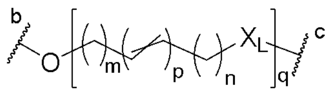;

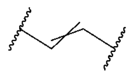

represents a single bond, a cis- or trans-double bond, alternatively a cis-double bond;
each $X_L$ is independently selected from a chemical bond, -O-, $C_{1-6}$ alkylene, $C_{2-6}$ alkenylene, $C_{2-6}$ alkynylene, $C_{3-10}$ cycloalkylene, 5- to 10-membered heterocyclylene, $C_{6-10}$ arylene and 5- to 10-membered heteroarylene;
the $X_L$ is optionally deuterated, up to completely deuterated;
each m is independently selected from 0, 1, 2, 3, 4, 5, 6, 7, 8, 9 and 10;
each n is independently selected from 0, 1, 2, 3, 4, 5, 6, 7, 8, 9 and 10;
each p is independently selected from 0, 1, 2, 3, 4 and 5;
each q is independently selected from 1, 2, 3, 4 and 5;
the b-terminal of L is connected to POI, and the c-terminal is connected to E3, or the c-terminal of L is connected to POI, and the b-terminal is connected to E3;
E3 is

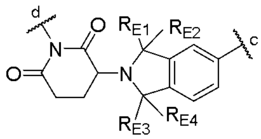;

$R_{E1}$, $R_{E2}$, $R_{E3}$ or $R_{E4}$ are independently selected from H and D, and at least one pair of $R_{E1}$ and $R_{E2}$, $R_{E3}$ and $R_{E4}$ represents =O, =S or =N-OH;
the c-terminal of E3 is connected to L, and the d-terminal is connected to Q via $C_{0-3}$ alkylene;
Q is

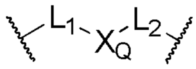;

$L_1$ and $L_2$ are independently selected from NH and O;
$X_Q$ is selected from $-P(O)(Y_Q-R_Q)-$, -S(O)- and $-C(O)-X_{Q1}-C(O)-$;
$Y_Q$ is selected from NH and O;
$X_{Q1}$ is selected from a chemical bond, $C_{1-6}$ alkylene, $C_{2-6}$ alkenylene, $C_{2-6}$ alkynylene, $-C_{1-6}$ alkylene-O-$C_{1-6}$ alkylene, $-C_{1-6}$ alkylene-$NR_b$-$C_{1-6}$ alkylene, $C_{3-10}$ cycloalkylene, 5- to 10-membered heterocyclylene, $C_{6-10}$

arylene and 5- to 10-membered heteroarylene, and the $X_{Q1}$ is optionally substituted with 1, 2, 3, 4 or 5 independently selected R#;
$R_Q$ is selected from H, $C_{1-6}$ alkyl, $C_{1-6}$ haloalkyl, $C_{2-6}$ alkenyl, $C_{2-6}$ alkynyl, $C_{3-10}$ cycloalkyl, 5- to 10-membered heterocyclyl, $C_{6-10}$ aryl and 5- to 10-membered heteroaryl;
$R_a$, $R_b$ and $R_c$ are independently selected from H, $C_{1-6}$ alkyl and $C_{1-6}$ haloalkyl;
R# is selected from H, halogen, OH, $NH_2$, $C_{1-6}$ alkyl, $C_{1-6}$ haloalkyl, $C_{2-6}$ alkenyl and $C_{2-6}$ alkynyl.

**2.** The compound of claim 1, or a pharmaceutically acceptable salt, a stereoisomer, a tautomer, a cis-trans isomer, a solvate, a hydrate, a polymorph, a prodrug or an isotope variant thereof, and a mixture thereof, wherein:

the POI is

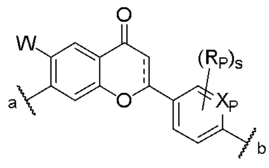
;

- - - represents a single bond or a double bond, alternatively a double bond;
each $R_P$ is independently selected from H, D, halogen, $C_{1-4}$ alkyl and $C_{1-4}$ haloalkyl;
s is selected from 1, 2 and 3;
$X_P$ is selected from N and $CR_P$;
W is selected from H, D, halogen, $C_{1-4}$ alkyl, $C_{1-4}$ haloalkyl and

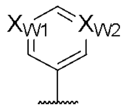
;

$X_{W1}$ and $X_{W2}$ are independently selected from N and $CR_W$;
Rw is selected from H, D, -C(O)$R_a$, -C(O)$NR_bR_c$, -C(O)$OR_a$, -OC(O)$R_a$ and -$NR_b$C(O)$R_a$;
$R_a$, $R_b$ and $R_c$ are independently selected from H, $C_{1-4}$ alkyl and $C_{1-4}$ haloalkyl;
the a-terminal of the POI is connected to Q via $C_{0-3}$ alkylene, and the b-terminal is connected to L; alternatively, the POI is

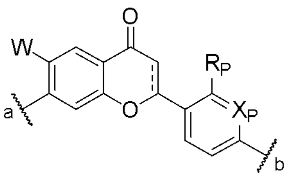
;

- - - represents a single bond or a double bond, alternatively a double bond;
$R_P$ is halogen;
$X_P$ is selected from N and CH;
W is selected from H, halogen and

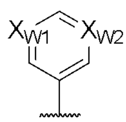
;

$X_{W1}$ and $X_{W2}$ are independently selected from N and $CR_W$;
Rw is selected from H, -C(O)$R_a$ and -C(O)$NR_bR_c$;
$R_a$, $R_b$ and $R_c$ are independently selected from H, $C_{1-4}$ alkyl and $C_{1-4}$ haloalkyl;
the a-terminal of the POI is connected to Q via $C_{0-3}$ alkylene, and the b-terminal is connected to L; still alternatively, the POI is selected from

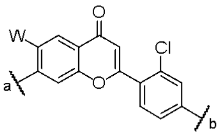, 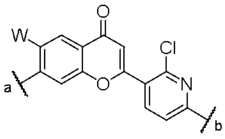, 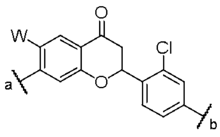

and

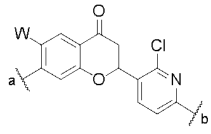;

W is selected from H, halogen,

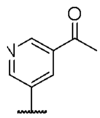, 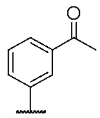 and 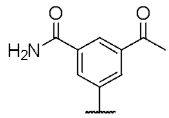,

alternatively W is selected from H, I and

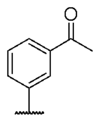;

the a-terminal of the POI is connected to Q via $C_{0-3}$ alkylene, and the b-terminal is connected to L.

**3.** The compound of claim 1 or 2, or a pharmaceutically acceptable salt, a stereoisomer, a tautomer, a cis-trans isomer, a solvate, a hydrate, a polymorph, a prodrug or an isotope variant thereof, and a mixture thereof, wherein:

L is

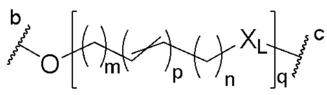;

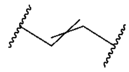

represents a single bond, a cis- or trans-double bond, alternatively a cis-double bond;
each $X_L$ is independently selected from a chemical bond, -O-, $C_{1-4}$ alkylene, phenylene and 5- to 6-membered heteroarylene; the $X_L$ is optionally deuterated, up to completely deuterated;
each m is independently selected from 0, 1, 2, 3, 4, 5, 6, 7, 8, 9 and 10;
each n is independently selected from 0, 1, 2, 3, 4, 5, 6, 7, 8, 9 and 10;
each p is independently selected from 0, 1, 2, 3, 4 and 5;
each q is independently selected from 1, 2, 3, 4 and 5;
the b-terminal of L is connected to POI, and the c-terminal is connected to E3, or the c-terminal of L is connected to POI, and the b-terminal is connected to E3;
alternatively,
L is

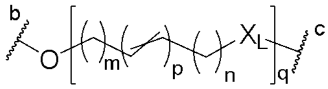;

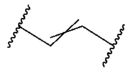

represents a single bond, a cis- or trans-double bond, alternatively a cis-double bond;
each $X_L$ is independently selected from a chemical bond, -O-, $-CH_2-$,

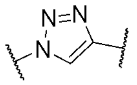 and 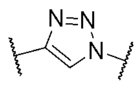,

alternatively each $X_L$ is independently selected from -O- and $-CH_2-$;
each m is independently selected from 0, 1, 2, 3, 4, 5, 6, 7 and 8, alternatively independently selected from 0, 1, 2, 3, and 4, and still alternatively each m is 1;
each n is independently selected from 0, 1, 2, 3, 4, 5, 6, 7 and 8, alternatively independently selected from 0, 1, 2, 3, and 4, and still alternatively is 1 or 2;
each p is independently selected from 0, 1, 2 and 3, alternatively independently or 1;
each q is independently selected from 1, 2, 3, 4 and 5, alternatively 3;
the b-terminal of L is connected to POI, and the c-terminal is connected to E3, or the c-terminal of L is connected to POI, and the b-terminal is connected to E3;
still alternatively,
L is selected from:

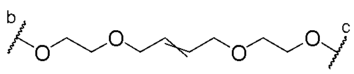 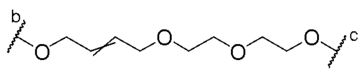 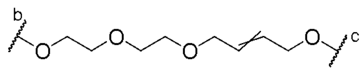

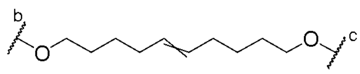 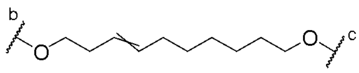 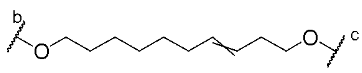

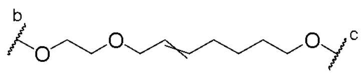 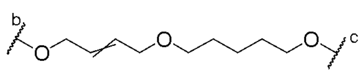 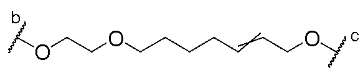

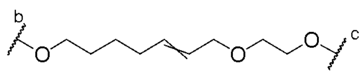 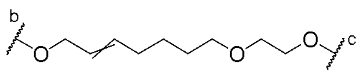 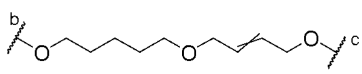

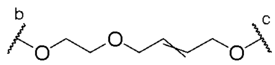 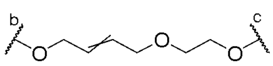 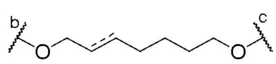 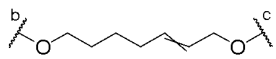

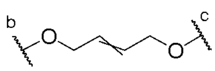 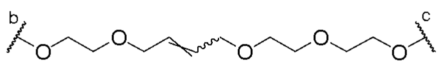 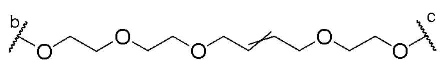

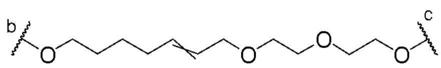 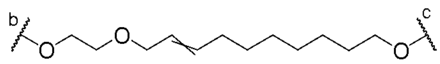

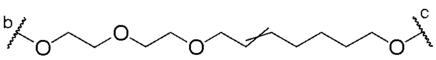 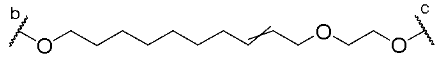

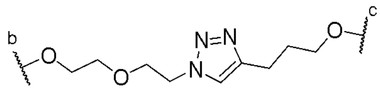 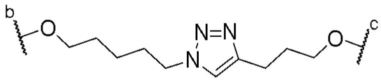 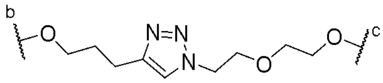

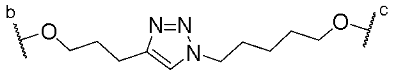 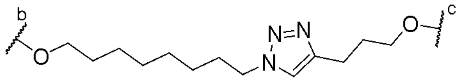

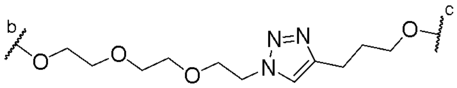 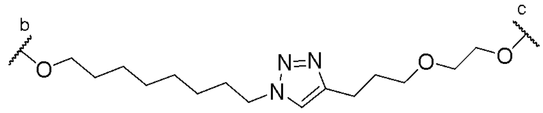

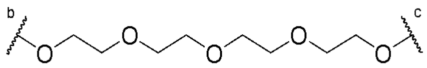;

alternatively, L is

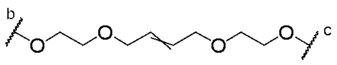, 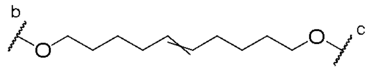,

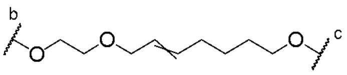, 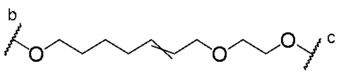 or 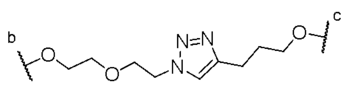;

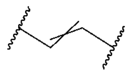

represents a single bond, a cis- or trans-double bond, alternatively a cis-double bond; the b-terminal of the L is connected to the POI, and the c-terminal is connected to E3, or the c-terminal of the L is connected to the POI, and the b-terminal is connected to E3.

**4.** The compound of any one of claims 1 to 3, or a pharmaceutically acceptable salt, a stereoisomer, a tautomer, a cis-trans isomer, a solvate, a hydrate, a polymorph, a prodrug or an isotope variant thereof, and a mixture thereof, wherein:

E3 is

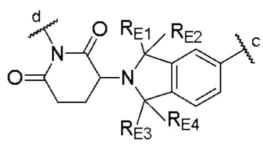;

$R_{E1}$, $R_{E2}$, $R_{E3}$ or $R_{E4}$ are independently selected from H and D, and at least one pair of $R_{E1}$ and $R_{E2}$, $R_{E3}$ and $R_{E4}$ represents =O;
the c-terminal of E3 is connected to L, and the d-terminal is connected to Q via $C_{0-3}$ alkylene;
alternatively,
E3 is selected from

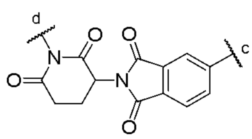, 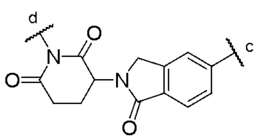 and 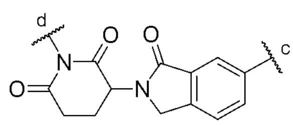;

the c-terminal of E3 is connected to L, and the d-terminal is connected to Q via $C_{0-3}$ alkylene.

**5.** The compound of any one of claims 1 to 4, or a pharmaceutically acceptable salt, a stereoisomer, a tautomer, a cis-trans isomer, a solvate, a hydrate, a polymorph, a prodrug or an isotope variant thereof, and a mixture thereof, wherein:

Q is

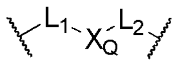;

$L_1$ and $L_2$ are independently selected from NH and O;
$X_Q$ is selected from -P(O)($Y_Q$-$R_Q$)-, -S(O)- and -C(O)-$X_{Q1}$-C(O)-;
$Y_Q$ is selected from NH and O;
$X_{Q1}$ is selected from a chemical bond, $C_{1-6}$ alkylene, $C_{2-6}$ alkenylene, $C_{2-6}$ alkynylene, -$C_{1-6}$ alkylene-$NR_b$-$C_{1-6}$ alkylene, 5- to 10-membered heterocyclylene and $C_{6-10}$ arylene, and the $X_{Q1}$ is optionally substituted with 1, 2, 3, 4 or 5 independently selected R#;
$R_b$ is selected from H, $C_{1-4}$ alkyl and $C_{1-4}$ haloalkyl;
$R_Q$ is selected from H, $C_{1-6}$ alkyl, $C_{2-6}$ alkenyl, $C_{2-6}$ alkynyl and $C_{6-10}$ aryl;
R# is selected from H, halogen, OH, $NH_2$, $C_{1-4}$ alkyl, $C_{2-4}$ alkenyl and $C_{2-4}$ alkynyl;
alternatively,
Q is

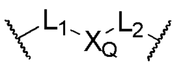;

$L_1$ and $L_2$ are independently selected from NH and O;
$X_Q$ is selected from -P(O)($Y_Q$-$R_Q$)-, -S(O)- and -C(O)-$X_{Q1}$-C(O)-;
$Y_Q$ is selected from NH and O;
$X_{Q1}$ is selected from a chemical bond, $C_{1-4}$ alkylene, -$C_{1-4}$ alkylene-$NR_b$-$C_{1-4}$ alkylene, 5- to 6-membered heterocyclylene (e.g.,

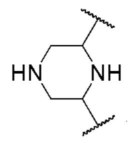

) and phenylene, wherein the $C_{1-4}$ alkylene is optionally substituted with 1, 2 or 3 independently selected R#;
$R_b$ is selected from H, $C_{1-4}$ alkyl and $C_{1-4}$ haloalkyl;
$R_Q$ is selected from H, $C_{1-4}$ alkyl and phenyl;
R# is selected from H and $NH_2$;
still alternatively,
Q is selected from

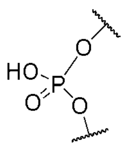, 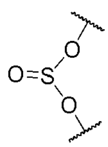, 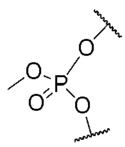, 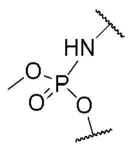, 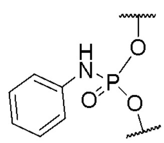, 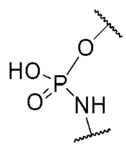,

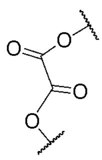, 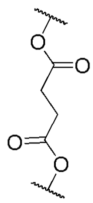, 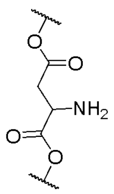, 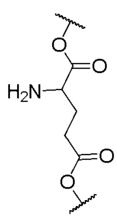 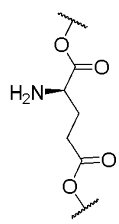, 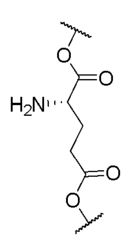, 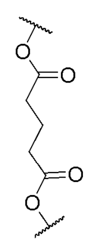, 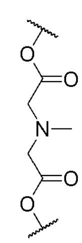, 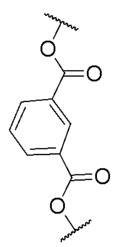,

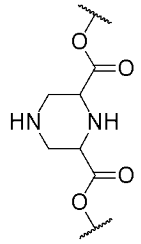 and 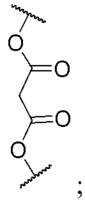 ;

alternatively, Q is selected from

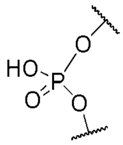 , 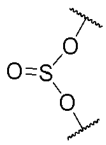 , 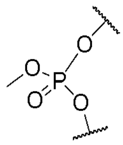 , 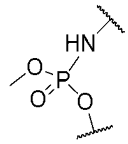 ,

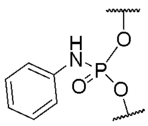 , 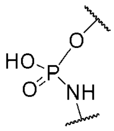 , 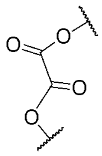 , 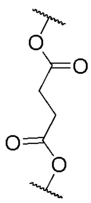 , 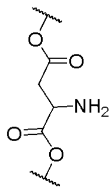 , 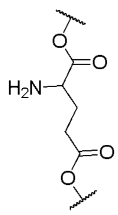 , 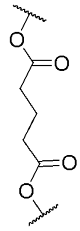 , 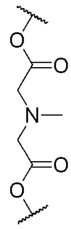 , 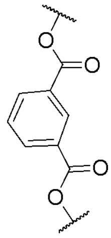 ,

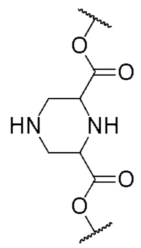 and 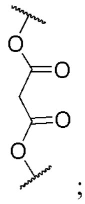 ;

and still alternatively, Q is

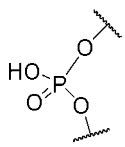 , 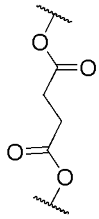 , 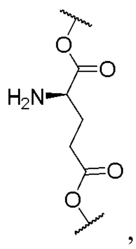 , 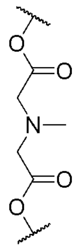 , 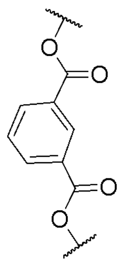

or

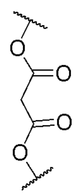 .

**6.** The compound of any one of claims 1 to 5, or a pharmaceutically acceptable salt, a stereoisomer, a tautomer, a cis-trans isomer, a solvate, a hydrate, a polymorph, a prodrug or an isotope variant thereof, and a mixture thereof, wherein:

the POI is

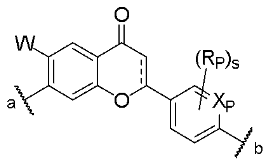;

- - - represents a single bond or a double bond, alternatively a double bond;
each $R_P$ is independently selected from H, D, halogen, $C_{1-4}$ alkyl and $C_{1-4}$ haloalkyl;
s is selected from 1, 2 and 3;
$X_P$ is selected from N and $CR_P$;
W is selected from H, D, halogen, $C_{1-4}$ alkyl, $C_{1-4}$ haloalkyl and

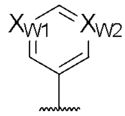;

$X_{W1}$ and $X_{W2}$ are independently selected from N and $CR_W$;
Rw is selected from H, D, $-C(O)R_a$, $-C(O)NR_bR_c$, $-C(O)OR_a$, $-OC(O)R_a$ and $-NR_bC(O)R_a$;
the a-terminal of the POI is connected to Q via $C_{0-3}$ alkylene, and the b-terminal is connected to L;
L is

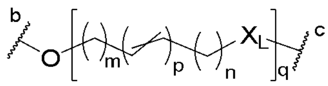;

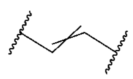

represents a single bond, a cis- or trans-double bond, alternatively a cis-double bond;
each $X_L$ is independently selected from a chemical bond, -O-, $C_{1-4}$ alkylene, phenylene and 5- to 6-membered heteroarylene; the $X_L$ is optionally deuterated, up to completely deuterated;
each m is independently selected from 0, 1, 2, 3, 4, 5, 6, 7, 8, 9 and 10;
each n is independently selected from 0, 1, 2, 3, 4, 5, 6, 7, 8, 9 and 10;
each p is independently selected from 0, 1, 2, 3, 4 and 5;
each q is independently selected from 1, 2, 3, 4 and 5;
the b-terminal of L is connected to POI, and the c-terminal is connected to E3, or the c-terminal of L is connected to POI, and the b-terminal is connected to E3;
E3 is

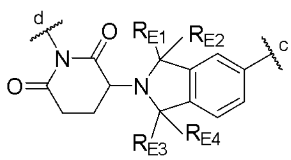;

$R_{E1}$, $R_{E2}$, $R_{E3}$ or $R_{E4}$ are independently selected from H and D, and at least one pair of $R_{E1}$ and $R_{E2}$, $R_{E3}$ and $R_{E4}$ represents =O;
the c-terminal of E3 is connected to L, and the d-terminal is connected to Q via $C_{0-3}$ alkylene;
Q is

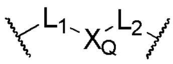;

$L_1$ and $L_2$ are independently selected from NH and O;
$X_Q$ is selected from $-P(O)(Y_Q-R_Q)-$, $-S(O)-$ and $-C(O)-X_{Q1}-C(O)-$;
$Y_Q$ is selected from NH and O;
$X_{Q1}$ is selected from a chemical bond, $C_{1-6}$ alkylene, $C_{2-6}$ alkenylene, $C_{2-6}$ alkynylene, $-C_{1-6}$ alkylene-$NR_b$-$C_{1-6}$ alkylene, 5- to 10-membered heterocyclylene and $C_{6-10}$ arylene, and the $X_{Q1}$ is optionally substituted with 1, 2, 3, 4 or 5 independently selected R#;
$R_Q$ is selected from H, $C_{1-6}$ alkyl, $C_{2-6}$ alkenyl, $C_{2-6}$ alkynyl and $C_{6-10}$ aryl;
$R_a$, $R_b$ and $R_c$ are independently selected from H, $C_{1-4}$ alkyl and $C_{1-4}$ haloalkyl;
R# is selected from H, halogen, OH, $NH_2$, $C_{1-4}$ alkyl, $C_{2-4}$ alkenyl and $C_{2-4}$ alkynyl.

**7.** The compound of any one of claims 1 to 6, or a pharmaceutically acceptable salt, a stereoisomer, a tautomer, a cis-trans isomer, a solvate, a hydrate, a polymorph, a prodrug or an isotope variant thereof, and a mixture thereof, wherein:

the POI is

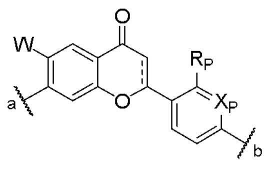;

- - - represents a single bond or a double bond, alternatively a double bond;
$R_P$ is halogen;
$X_P$ is selected from N and CH;
W is selected from H, halogen and

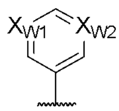;

$X_{W1}$ and $X_{W2}$ are independently selected from N and $CR_W$;
Rw is selected from H, $-C(O)R_a$ and $-C(O)NR_bR_c$;
the a-terminal of the POI is connected to Q via $C_{0-3}$ alkylene, and the b-terminal is connected to L;
L is

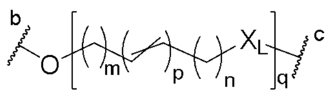;

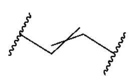

represents a single bond, a cis- or trans-double bond, alternatively a cis-double bond;
each $X_L$ is independently selected from a chemical bond, -O-, $-CH_2-$,

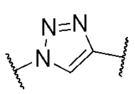 and 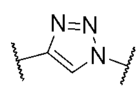,

alternatively each $X_L$ is independently selected from -O- and $-CH_2-$;
each m is independently selected from 0, 1, 2, 3, 4, 5, 6, 7 and 8, alternatively independently selected from 0, 1, 2,

3, and 4, and still alternatively m = 1;

each n is independently selected from 0, 1, 2, 3, 4, 5, 6, 7 and 8, alternatively independently selected from 0, 1, 2, 3, and 4, and still alternatively n=1 or 2;

each p is independently selected from 0, 1, 2 and 3, alternatively independently selected from 0 and 1;

each q is independently selected from 1, 2, 3, 4 and 5, alternatively independently is 3;

the b-terminal of L is connected to POI, and the c-terminal is connected to E3, or the c-terminal of L is connected to **POI,** and the b-terminal is connected to E3;

E3 is

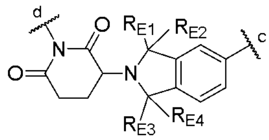
;

$R_{E1}$, $R_{E2}$, $R_{E3}$ or $R_{E4}$ are independently selected from H and D, and at least one pair of $R_{E1}$ and $R_{E2}$, $R_{E3}$ and $R_{E4}$ represents =O;

the c-terminal of E3 is connected to L, and the d-terminal is connected to Q via $C_{0-3}$ alkylene;

Q is

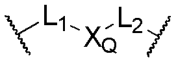
;

$L_1$ and $L_2$ are independently selected from NH and O;

$X_Q$ is selected from -P(O)($Y_Q$-$R_Q$)-, -S(O)- and -C(O)-$X_{Q1}$-C(O)-;

$Y_Q$ is selected from NH and O;

$X_{Q1}$ is selected from a chemical bond, $C_{1-4}$ alkylene, -$C_{1-4}$ alkylene-$NR_b$-$C_{1-4}$ alkylene, 5- to 6-membered heterocyclylene (e.g.,

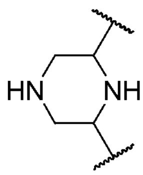

) and phenylene, and the $C_{1-4}$ alkylene is optionally substituted with 1, 2 or 3 independently selected R#;

$R_Q$ is selected from H, $C_{1-4}$ alkyl and phenyl;

$R_a$, $R_b$ and $R_c$ are independently selected from H, $C_{1-4}$ alkyl and $C_{1-4}$ haloalkyl;

R# is selected from H and $NH_2$.

**8.** The compound of any one of claims 1 to 7, or a pharmaceutically acceptable salt, a stereoisomer, a tautomer, a cis-trans isomer, a solvate, a hydrate, a polymorph, a prodrug or an isotope variant thereof, and a mixture thereof, wherein:

the POI is selected from

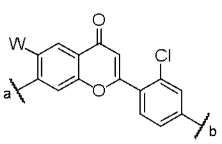
,
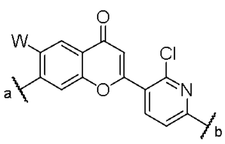
,
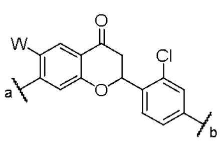

and

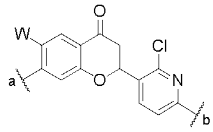,

alternatively the POI is selected from

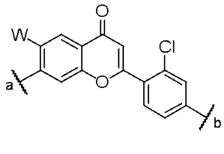 and 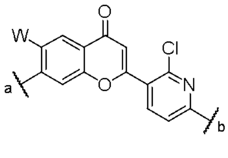;

W is selected from H, halogen,

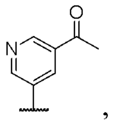, 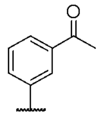 and 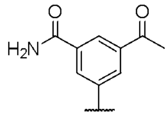,

alternatively W is selected from H, I and

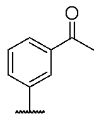;

the a-terminal of the POI is connected to Q via $C_{0-3}$ alkylene, and the b-terminal is connected to L; L is selected from:

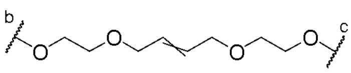 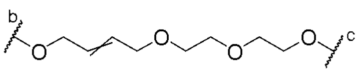 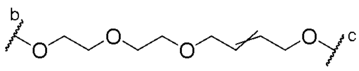

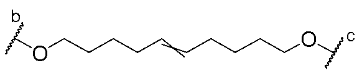 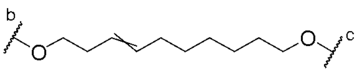 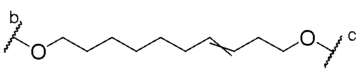

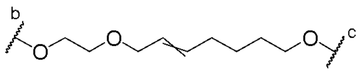 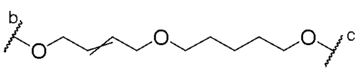 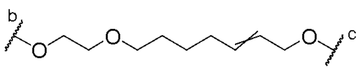

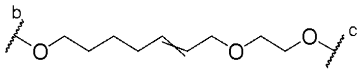 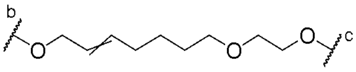 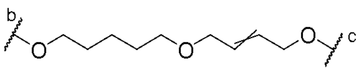

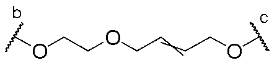 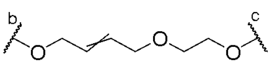 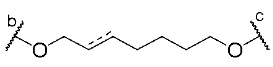 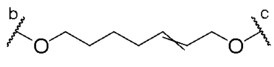

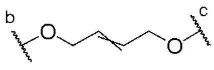 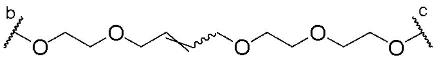 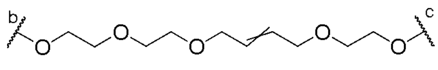

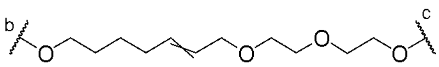 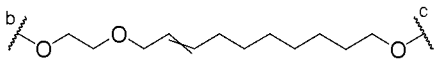

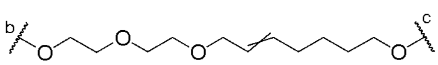 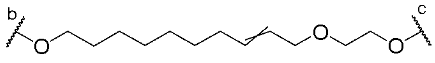

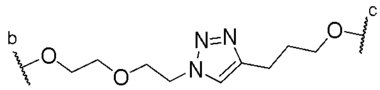 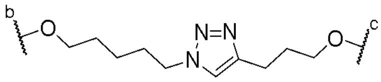 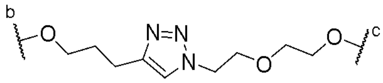

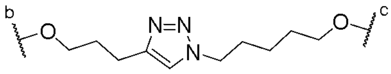 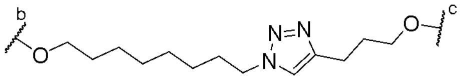

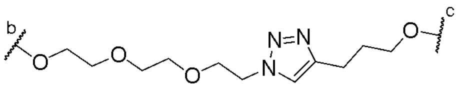 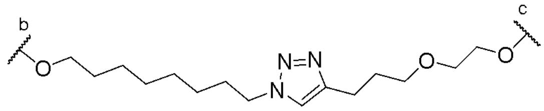

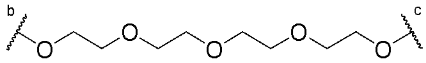;

alternatively, L is

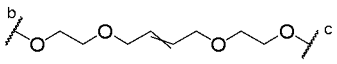, 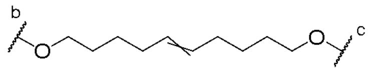,

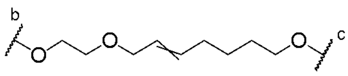, 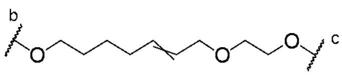 or 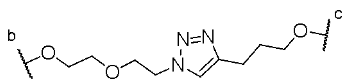;

the b-terminal of L is connected to POI, and the c-terminal is connected to E3, or the c-terminal of L is connected to POI, and the b-terminal is connected to E3;

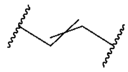

represents a single bond, a cis- or trans-double bond, alternatively a cis-double bond;
E3 is selected from

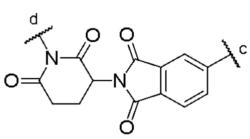, 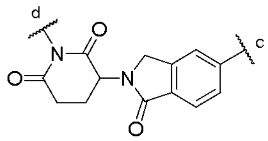 and 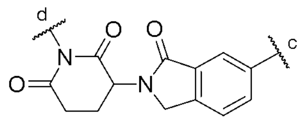;

the c-terminal of E3 is connected to L, and the d-terminal is connected to Q via $C_{0-3}$ alkylene;
Q is selected from

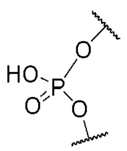, 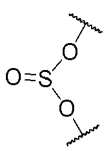, 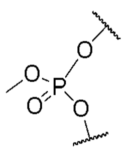, 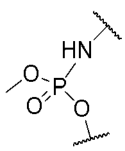, 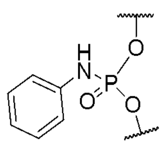, 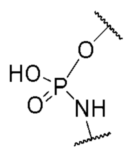,

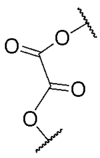, 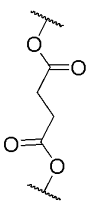, 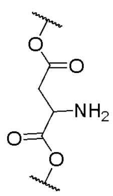, 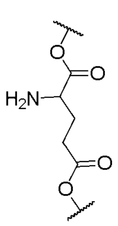, 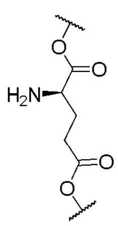, 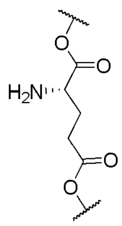, 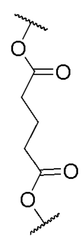, 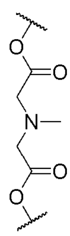, 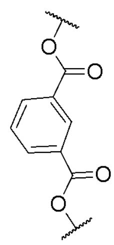,

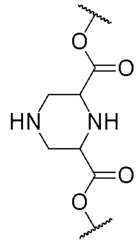 and 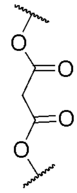 ;

alternatively, Q is selected from

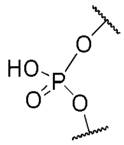 , 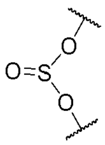 , 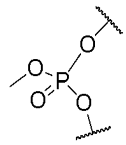 , 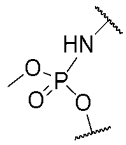 ,

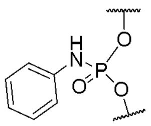 , 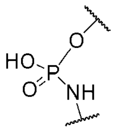 , 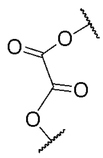 , 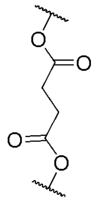 , 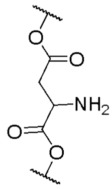 , 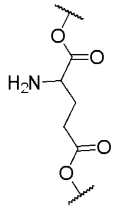 , 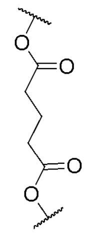 , 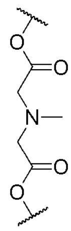 , 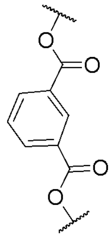 ,

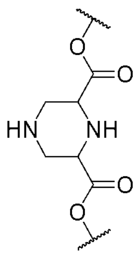 and 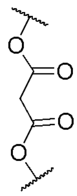 ;

and still alternatively, Q is

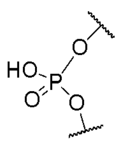 , 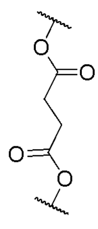 , 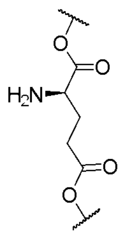 , 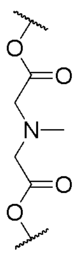 , 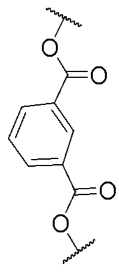

or

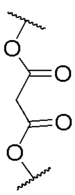 .

**9.** The compound of any one of claims 1 to 8, or a pharmaceutically acceptable salt, a stereoisomer, a tautomer, a cis-trans isomer, a solvate, a hydrate, a polymorph, a prodrug or an isotope variant thereof, and a mixture thereof, wherein the compound is selected from:

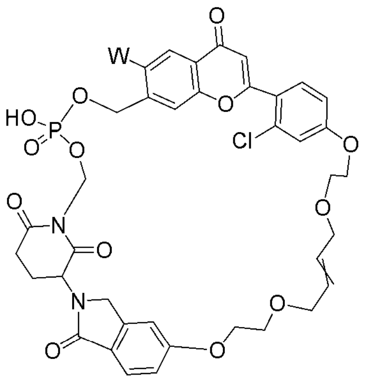 1,

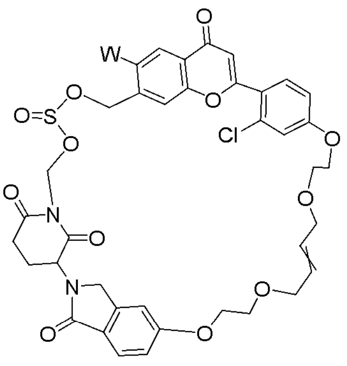 2,

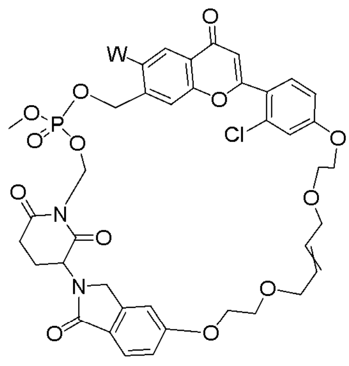 3,

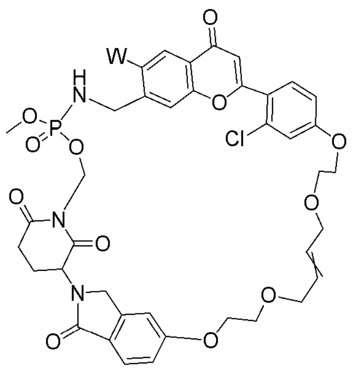 ,

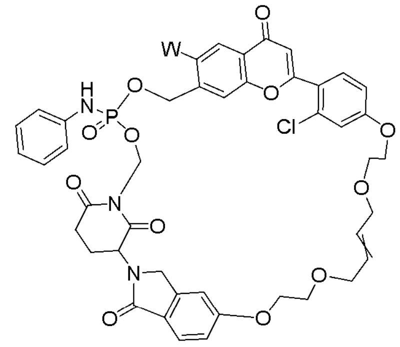 ,

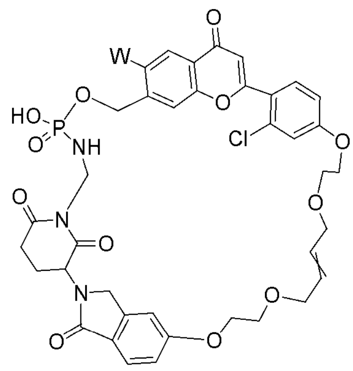 ,

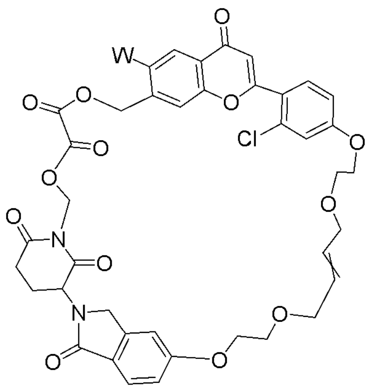 4,

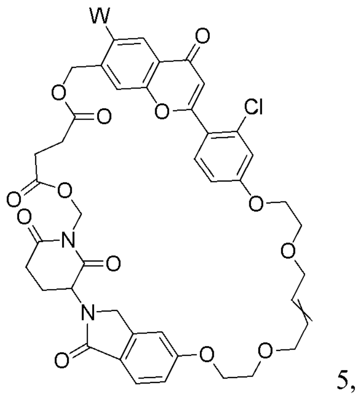 5,

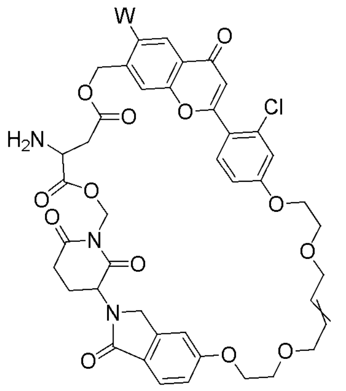 6,

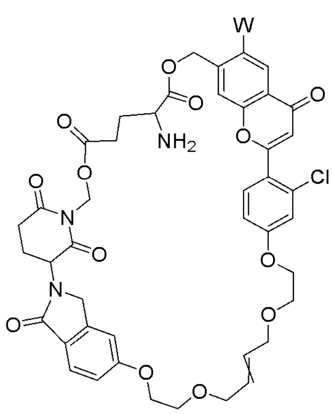 7,

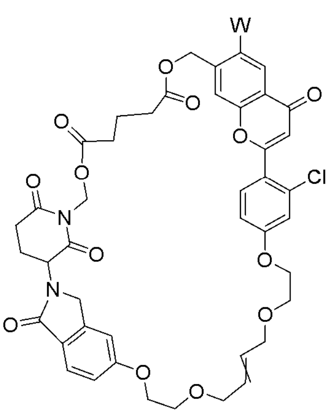 8,

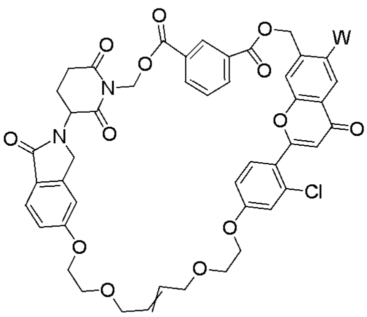 9,

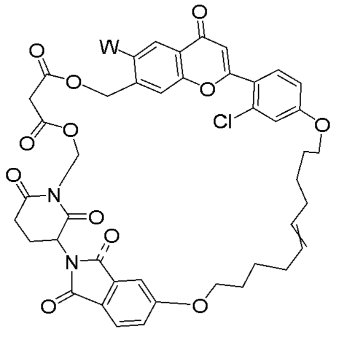 10, 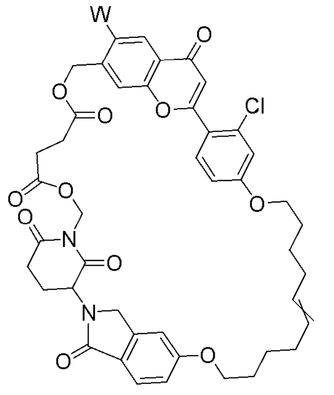 13, 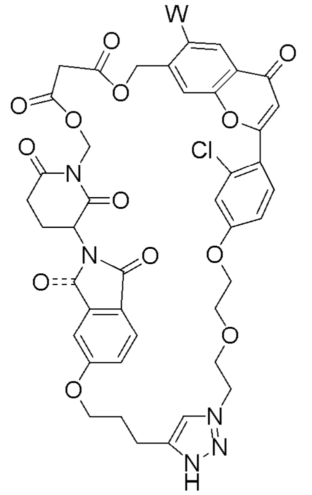 11,

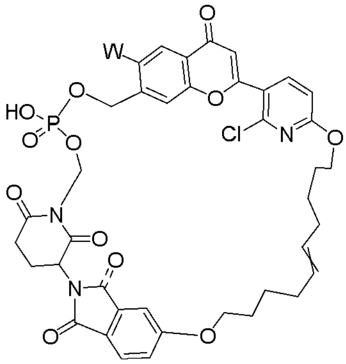 12, 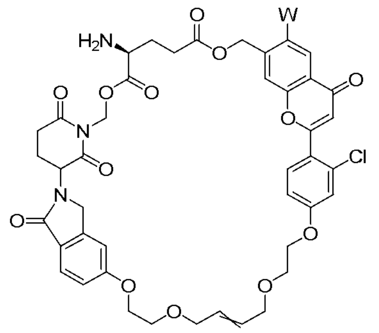 and 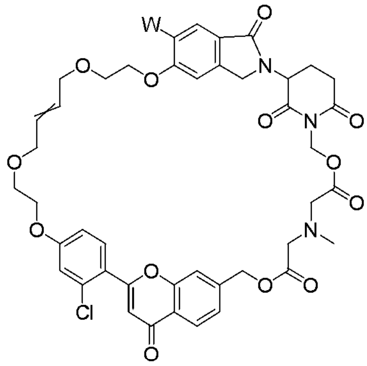 ,

wherein,

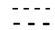 represents a single bond or a double bond;

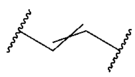

represents a single bond, a cis- or trans-double bond, alternatively a cis-double bond;

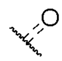

represents absent or

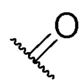 ;

W is selected from H, I,

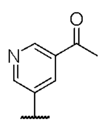 , 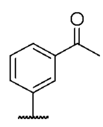 and 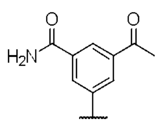 ,

alternatively W is selected from H, I and

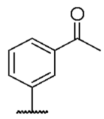 ;

alternatively, the compound is selected from:

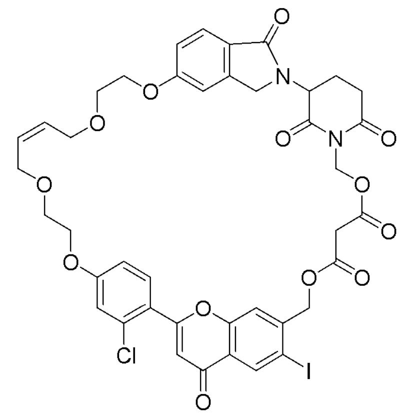 , 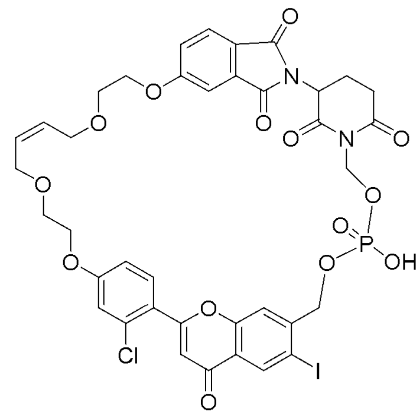 , 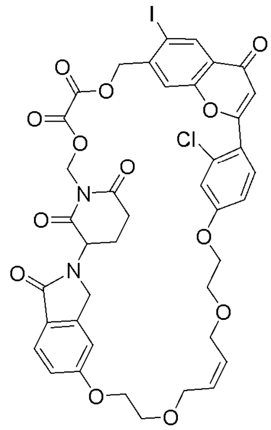 ,

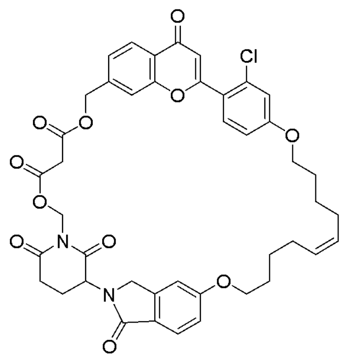 , 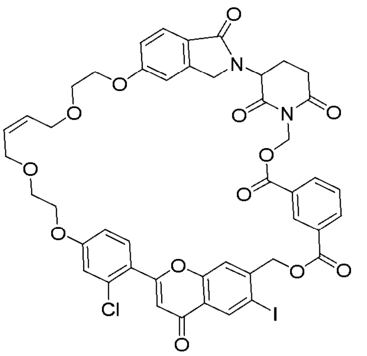 , 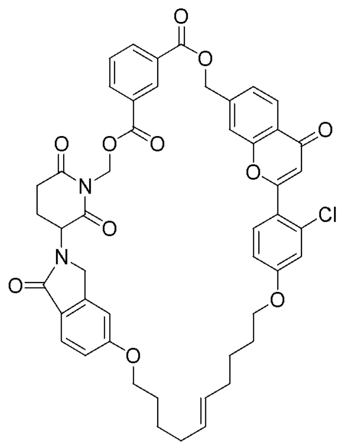 ,

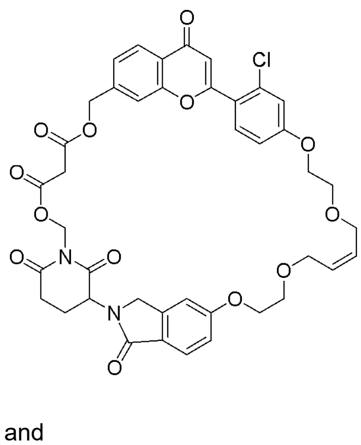 , 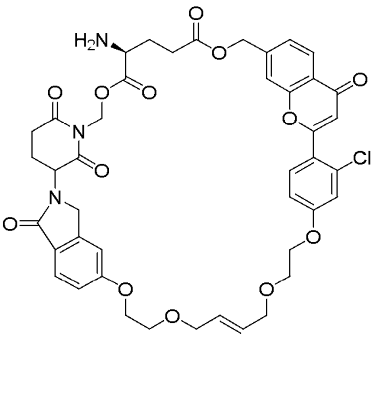 , 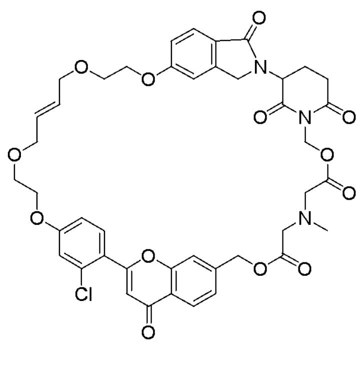

and

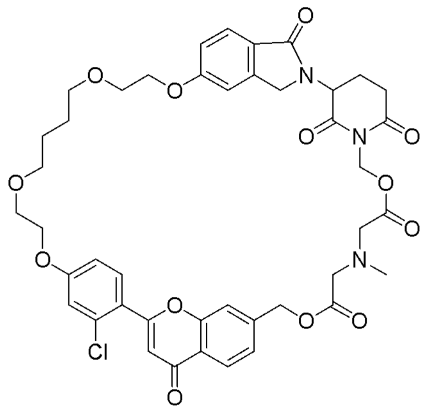

.

**10.** A pharmaceutical composition comprising a compound of any one of claims 1 to 9, or a pharmaceutically acceptable salt, a stereoisomer, a tautomer, a cis-trans isomer, a solvate, a hydrate, a polymorph, a prodrug or an isotope variant thereof, and a mixture thereof, and a pharmaceutically acceptable excipient; optionally, the pharmaceutical composition further comprises an additional therapeutic agent.

**11.** Use of a compound of any one of claims 1 to 9, or a pharmaceutically acceptable salt, a stereoisomer, a tautomer, a cis-trans isomer, a solvate, a hydrate, a polymorph, a prodrug or an isotope variant thereof, and a mixture thereof in the manufacture of a medicament for inhibiting protein tyrosine phosphatase.

**12.** Use of a compound of any one of claims 1 to 9, or a pharmaceutically acceptable salt, a stereoisomer, a tautomer, a cis-trans isomer, a solvate, a hydrate, a polymorph, a prodrug or an isotope variant thereof, and a mixture thereof in the manufacture of a medicament for inhibiting SHP2.

**13.** Use of a compound of any one of claims 1 to 9, or a pharmaceutically acceptable salt, a stereoisomer, a tautomer, a cis-trans isomer, a solvate, a hydrate, a polymorph, a prodrug or an isotope variant thereof, and a mixture thereof in the manufacture of a medicament for treating a tumor disease.

**14.** A method for treating a tumor disease in a subject, the method comprising administering to the subject a compound of any one of claims 1 to 9, or a pharmaceutically acceptable salt, a stereoisomer, a tautomer, a cis-trans isomer, a solvate, a hydrate, a polymorph, a prodrug or an isotope variant thereof, and a mixture thereof, or a pharmaceutical composition of claim 10.

**15.** A compound of any one of claims 1 to 9, or a pharmaceutically acceptable salt, a stereoisomer, a tautomer, a cis-trans isomer, a solvate, a hydrate, a polymorph, a prodrug or an isotope variant thereof, and a mixture thereof, or a pharmaceutical composition of claim 10 for use in treating a tumor disease.

**16.** The use of claim 13 or the method of claim 14 or the use of the compound or composition of claim 15, wherein the tumor disease is selected from a hematological tumor and a solid tumor disease.

**17.** The use or method or use of the compound or composition of claim 16, wherein the hematological tumor or solid tumor disease is selected from solid tumors such as breast cancer, lung cancer, neuroblastoma, oral squamous cell carcinoma, colorectal tumor, ovarian cancer, cervical cancer, prostate cancer and pancreatic cancer, hematological tumor diseases such as myeloid leukemia and lymphoid leukemia, and other relapsed and refractory advanced solid and hematological tumors.

**INTERNATIONAL SEARCH REPORT**

International application No.
**PCT/CN2024/073173**

**A. CLASSIFICATION OF SUBJECT MATTER**

C07D311/22(2006.01)i; C07D311/04(2006.01)i; C07D405/14(2006.01)i; C07D209/02(2006.01)i; A61K31/403(2006.01)i; A61K31/352(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT; CNKI; VEN; WOTXT; EPTXT; USTXT; STN; 万方, WANFANG: 北京泰德制药股份有限公司, 李文明, 李晓博, 鲁鹏, 刘俊荣, 王宁, 康嘉龙, 胡慧娟, 周蓉, 余晓磊, 彭丽娇, 周丽莹, 甘乐凌, 张宇, 蛋白靶向降解, 蛋白降解靶向, 嵌合体, 联合体, 蛋白酪氨酸磷酸酶, Src同源, 黄酮, 泛素, 连接酶, proteolytic targeting chimera, PROTACs, protein tyrosine phosphatase, PTP, SHP2, Src homology, flavone, flavonoid?, E3, ligase, 结构式, structural formula

**C. DOCUMENTS CONSIDERED TO BE RELEVANT**

| Category* | Citation of document, with indication, where appropriate, of the relevant passages | Relevant to claim No. |
|---|---|---|
| A | BIAN, Jinlei et al. "Discovery of Wogonin-based PROTACs Against CDK9 and Capable of Achieving Antitumor Activity" *Bioorganic Chemistry,* Vol. vol. 81, 30 August 2018 (2018-08-30), ISSN: 0045-2068, abstract, Scheme 2, Scheme 3, and Table 1 | 1-17 |
| A | WO 2021236578 A1 (SALK INST FOR BIOLOGICAL STUDI et al.) 25 November 2021 (2021-11-25) entire document | 1-17 |
| A | CN 114890995 A (HUAQIAO UNIVERSITY) 12 August 2022 (2022-08-12) entire document | 1-17 |
| A | CN 112672993 A (PROTECH CO., LTD.) 16 April 2021 (2021-04-16) entire document | 1-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 March 2024** | **31 March 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/073173**

**C. DOCUMENTS CONSIDERED TO BE RELEVANT**

| Category* | Citation of document, with indication, where appropriate, of the relevant passages | Relevant to claim No. |
|---|---|---|
| A | WO 2022165432 A1 (ICAHN SCHOOL MED MOUNT SINAI) 04 August 2022 (2022-08-04)<br>entire document | 1-17 |
| A | VEMULAPALLI Vidyasiri et al. "Targeted Degradation of the Oncogenic Phosphatase SHP2" *Biochemistry*, Vol. 60, No. (34), 19 August 2021 (2021-08-19),<br>ISSN: 0006-2960,<br>pages 2593-2609 | 1-17 |

Form PCT/ISA/210 (second sheet) (July 2022)

**INTERNATIONAL SEARCH REPORT**

International application No.
**PCT/CN2024/073173**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14-17**
because they relate to subject matter not required to be searched by this Authority, namely:

Claims 14 and 15 set forth a method for treating tumor diseases, and a use in treating tumor diseases, which fall within methods for treating a living human or animal body, that is, fall within the cases set forth in PCT Rule 39.1(iv) for which an international search is not required.

The technical solutions of dependent claims 16 and 17, which refer to claims 14 and 15, also fall within the cases set out in PCT Rule 39.1(iv) for which an international search is not required.

Nevertheless, a search is still performed on the basis of the designation of the subject matter of claims 14-17 being a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/073173**

| Patent document cited in search report | Publication date (day/month/year) | Patent family member(s) | Publication date (day/month/year) |
|---|---|---|---|
| WO 2021236578 A1 | 25 November 2021 | JP 2023527281 A | 28 June 2023 |
| | | CA 3183957 A1 | 25 November 2021 |
| | | EP 4153578 A1 | 29 March 2023 |
| CN 114890995 A | 12 August 2022 | CN 114890995 B | 20 June 2023 |
| CN 112672993 A | 16 April 2021 | WO 2020022785 A1 | 30 January 2020 |
| | | IN 202147004416 A | 12 February 2021 |
| | | AU 2019312065 A1 | 18 February 2021 |
| | | IL 280358 A | 25 March 2021 |
| | | EP 3828164 A1 | 02 June 2021 |
| | | US 2021299253 A1 | 30 September 2021 |
| | | JP 2021532132 A | 25 November 2021 |
| | | EP 3828164 A4 | 18 May 2022 |
| | | AU 2022279386 A1 | 19 January 2023 |
| WO 2022165432 A1 | 04 August 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310095725
- CN 202410052474
- US 5376645 A


### Non-patent literature cited in the description


- **K. S. GROSSMAN et al.** *Adv. Cancer Res*, 2010, vol. 106, 53-89
- **BERGE S. M. et al.** Pharmaceutical Salts. *J. Pharm. Sci.*, 1977, vol. 66, 1-19
- Bioreversible Carriers in Drug Design. **T. HIGUCHI** ; **V. STELLA**. Prodrugs as Novel Delivery Systems. American Pharmaceutical Association and Pergamon Press, 1987, vol. 14
- **D. FLEISHER** ; **S. RAMON** ; **H. BARBRA**. Improved oral drug delivery: solubility limitations overcome by the use of prodrugs. *Advanced Drug Delivery Reviews*, 1996, vol. 19 (2), 115-130
- Remington's Pharmaceutical Sciences. Mack Publishing Company, 1985